(19) Europäisches Patentamt
European Patent Office
Office européen des brevets

(11) EP 2 053 409 A1

(12) EUROPEAN PATENT APPLICATION

(43) Date of publication:
**29.04.2009 Bulletin 2009/18**

(21) Application number: **09151175.8**

(22) Date of filing: **12.11.2004**

(51) Int Cl.:
***G01N 33/68*** (2006.01) ***A61K 31/16*** (2006.01)
***A61K 39/395*** (2006.01)

(84) Designated Contracting States:
**CH DE ES FR GB IT LI**
Designated Extension States:
**LT LV**

(30) Priority: **20.11.2003 US 523845 P**

(62) Document number(s) of the earlier application(s) in accordance with Art. 76 EPC:
**04026956.5 / 1 533 619**

(71) Applicant: **F. Hoffmann-La Roche AG**
**4070 Basel (CH)**

(72) Inventors:
- **Rosinski, James Andrew**
**Nutley, NJ 07110 (US)**
- **Kochan, Jarema Peter**
**Towaco, NJ 07082 (US)**

(74) Representative: **Küng, Peter**
**F. Hoffmann-La Roche AG**
**Patent Department**
**Grenzacherstrasse 124**
**4070 Basel (CH)**

Remarks:
This application was filed on 23-01-2009 as a divisional application to the application mentioned under INID code 62.

# (54) Specific markers for metabolic syndrome

(57) The present invention provides polypeptides which are predominately expressed in visceral adipose tissue which can be used as markers for the measurement of the levels of visceral adipose tissue in a subject. The invention also provides methods for the measurement of the levels of visceral adipose tissue by obtaining a biological sample and detecting and/or measuring the increase of one or more polypeptides as disclosed herein. Screening methods relating to agonists and antagonists of the specific polypeptides disclosed herein are provided. Antibodies may also be raised against these polypeptide markers for the detection and/or treatment of metabolic syndrome related comorbidities.

Figure 6

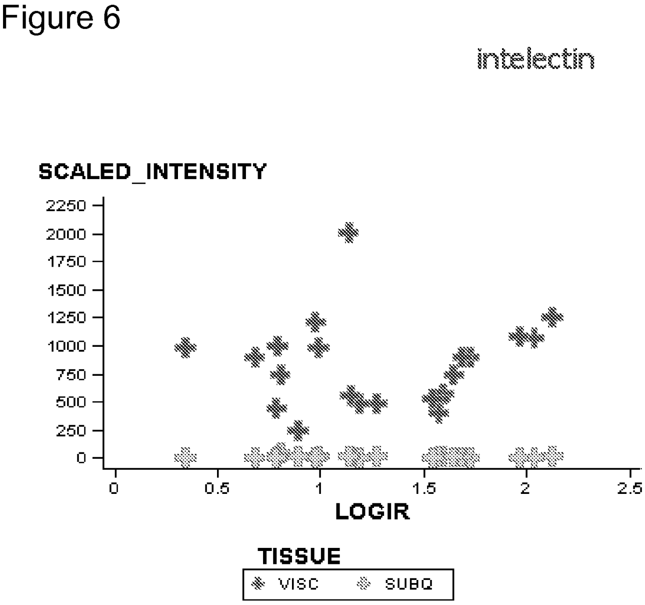


EP 2 053 409 A1

**Description**

In both men and women, visceral adipose tissue accumulation is associated with an increased risk of the development of non-insulin dependent diabetes, myocardial infarction, stroke and other arteriosclerotic diseases and their associated risk factors, including insulin resistance, elevated blood lipids, glucose and hypertension. The clustering of these risk factors has been designated 'Metabolic Syndrome', also called 'Syndrome X', the 'Insulin Resistance Syndrome' or the 'Deadly Quartet'. This syndrome is also characterized by one or more endocrine disturbances and is therefore also called 'Neuro-endocrine Syndrome' (Marin, P. Neuroendocrine News, 21(3) 1996, 2). These disturbances include low serum levels of sex steroids (testosterone in men, and estrogens in women), signs of a decreased action of growth hormone, and an excessive secretion of cortisol. The latter has been shown clinically as a major causative process for the development of Metabolic Syndrome as demonstrated by successful treatment with the cortisol synthesis inhibitor ketoconazole (WO 96/04912).

Conditions related to Metabolic Syndrome include diabetes mellitus type II (IDDM), non-insulin dependent diabetes (NIDDM), myocardial infarction, stroke and other arteriosclerotic diseases as well as the risk factors for these diseases, insulin resistance in general, abdominal obesity caused by accumulation of visceral adipose tissue, elevated serum lipids, and raised diastolic and/or systolic blood pressure.

Visceral adipose tissue is known as the intra-abdominal fat, the adipose depot associated with central obesity. This adipose depot is to be distinguished from the subcutaneous adipose depot, which is located throughout the body. It is the visceral adipose tissue, which has been associated with an increased risk for disorders, as well as mortality. Visceral adipose tissue plays a key role in this process by modulating whole body metabolism, in as yet undefined ways. In obesity, the relative amounts of visceral adipose tissue can vary from individual to individual, and the only means of precisely defining the levels of visceral adipose tissue is via the use of magnetic resonance imaging and by computed tomography. These complex techniques can provide a detailed determination of the levels of visceral adipose tissue, but are not available to the routine access to measure the community at large for healthcare purposes. In addition, the costs associated with MRI and CT scans are quite large, and thus not applicable to routine screening.

As can be seen, there is a need for a relatively simple and cost-efficient technique for measuring, monitoring and tracking levels of visceral adipose tissue as a method for diagnosing and possibly treating metabolic syndrome as well as a method for finding potential compounds for the treatment of metabolic syndrome.

According to one aspect of the present invention, a method for the measurement of levels of visceral adipose tissue comprises obtaining a biological sample; and detecting or measuring the level of a polypeptide marker, the polypeptide marker comprising at least one polypeptide selected from the group consisting of the polypeptides having SEQ ID Nos. 2, 4, 6, 8, 10, 12, 14, 16, 18, 20, 22, 24, 26, 28, 30, 32, 34, 36 and 38.

According to one aspect of the present invention, a method for the measurement of levels of visceral adipose tissue comprises detecting or measuring the level of a polypeptide marker in a biological sample, the polypeptide marker comprising at least one polypeptide selected from the group consisting of the polypeptides having SEQ ID Nos. 2, 4, 6, 8, 10, 12, 14, 16, 18, 20, 22, 24, 26, 28, 30, 32, 34, 36 and 38.

According to another aspect of the present invention, a method for measuring the level of visceral adipose tissue in a subject comprises obtaining a biological sample; and detecting or measuring the level of a marker, the nucleic acid marker comprising at least one nucleic acid molecule selected from the group consisting of the nucleic acid molecules of SEQ ID Nos. 1, 3, 5, 7, 9, 11, 13, 15, 17, 19, 21, 23, 25. 27, 29, 31, 33, 35 and 37.

According to another aspect of the present invention, a method for measuring the level of visceral adipose tissue in a subject comprises detecting or measuring the level of a marker in a biological sample, the nucleic acid marker comprising at least one nucleic acid molecule selected from the group consisting of the nucleic acid molecules of SEQ ID Nos. 1, 3, 5, 7, 9, 11, 13, 15, 17, 19, 21, 23, 25. 27, 29, 31, 33, 35 and 37.

According to a further aspect of the present invention, there is provided a screening method for identifying a compound which interacts with a polypeptide that is predominately expressed in visceral adipose tissue, the polypeptide being selected from the group consisting of the polypeptides having SEQ ID Nos. 2, 4, 6, 8, 10, 12, 14, 16, 18, 20, 22, 24, 26, 28, 30, 32, 34, 36 and 38, comprising contacting said polypeptide with a compound or a plurality of compounds under conditions which allow interaction of the compound with the polypeptide; and detecting the interaction between the compound or plurality of compounds with the polypeptide.

According to yet another aspect of the present invention, there is provided a screening method for identifying a compound which is an agonist or an antagonist of a polypeptide that is predominately expressed in visceral adipose tissue, the polypeptide being selected from the group consisting of the polypeptides having SEQ Nos. 2, 4, 6, 8, 10, 12, 14, 16, 18, 20, 22, 24, 26, 28, 30, 32, 34, 36 and 38, comprising contacting said polypeptide with a compound under conditions which allow interaction of the compound with said polypeptide; determining a first level of activity of the polypeptide; determining a second level of activity of the polypeptide expressed in a host which has not been contacted with the compound; and quantitatively relating the first level of activity with the second level of activity, wherein when the first level of activity is less than the second level of activity, the compound is identified as an antagonist of the polypeptide.

According to still a further aspect of the present invention, there is provided a screening method for identifying a compound which is an inhibitor of the expression of a polypeptide that is predominately expressed in visceral adipose tissue, the polypeptide being selected from the group consisting of the polypeptides having SEQ ID Nos. 2, 4, 6, 8, 10, 12, 14, 16, 18, 20, 22, 24, 26, 28, 30, 32, 34, 36 and 38, comprising contacting a host which expresses the polypeptide with a compound; determining a first expression level or activity of the polypeptide; determining a second expression level or activity of the polypeptide in a host which has not been contacted with the compound; and quantitatively relating the first expression level or activity with the second expression level or activity, wherein when the first expression level or activity is less than the second expression level or activity, the compound is identified as an inhibitor of the expression of the polypeptide.

According to another aspect of the present invention, there are provided a method of correlating protein levels in a mammal with a diagnosis of the level of visceral adipose tissue, comprising selecting one or more proteins selected from the group consisting of the proteins having SEQ Nos. 2, 4, 6, 8, 10, 12, 14, 16, 18, 20, 22, 24, 26, 28, 30, 32, 34, 36 and 38; determining the level of the one or more proteins in the mammal; and generating an index number, Y, which indicates a base level of visceral adipose tissue.

According to still another aspect of the present invention, there is provided a kit for screening of compounds that activate or inhibit a polypeptides or stimulate or inhibit the expression of any of said polypeptides, the polypeptides being selected from the group consisting of the polypeptides having SEQ Nos. 2, 4, 6, 8, 10, 12, 14, 16, 18, 20, 22, 24, 26, 28, 30, 32, 34, 36 and 38.

According to a further aspect of the present invention, there is provided a method for monitoring serum levels of one or more proteins to measure levels of visceral adipose tissue in a subject, the method comprising raising antibodies of said one or more proteins; detecting the serum level of the proteins; and comparing the serum level to those subjects known to have a specific level of visceral adipose tissue.

According to yet a further aspect of the present invention, there is provided a method for treating metabolic syndrome comprising administering, to a patient in need thereof, a therapeutically effective amount of at least one antibody against at least one protein, or antigen-binding fragment thereof, selected from the group consisting of the proteins having SEQ ID Nos. 2, 4, 6, 8, 10, 12, 14, 16, 18, 20, 22, 24, 26, 28, 30, 32, 34, 36 and 38.

According to another aspect of the present invention, there is provided a method for treating metabolic syndrome comprising administering, to a patient in need thereof, a therapeutically effective amount of at least one protein, protein fragment or peptide selected from the group consisting of the proteins having SEQ ID Nos. 2, 4, 6, 8, 10, 12, 14, 16, 18, 20, 22, 24, 26, 28, 30, 32, 34, 36 and 38.

The above, and other objects, features and advantages of the present invention will become apparent from the following description read in conjunction with the accompanying drawings and claims.

Figures 1 through 16 are graphs of the scaled intensity vs. log of the insulin resistance for various adipose levels of RNA measured by Affymetrix analysis in visceral and subcutaneous adipose tissues. Figures 17 and 18 are graphs of the scaled intensity vs. log of the insulin resistance for various adipose levels of RNA measured by STEP analysis in visceral and subcutaneous adipose tissues.

The problem of identifying gene and polypeptides suitable as markers of metabolic syndrome for early diagnosis of the disease, and the long felt need for such markers, was overcome by the present invention. It was surprisingly found that a specific set of genes are more selectively secreted in visceral adipose tissue. The differentially expressed genes, and the polypeptides they encode, along with their accession numbers, are listed in Table 1.

Table 1: Visceral Adipose Secreted Proteins

| Name | Abbreviation | Alias | GenBank | Locus Link | MRNA | Protein |
|---|---|---|---|---|---|---|
| Annexin A8 | ANX8 | Annexin VII, annexin VIII | X16662 SEQ ID No. 1 | 244 | NM001630 | NP001621 SEQ ID No. 2 |
| Complement component 4A | C4A, C4S, CO4 | Acidic C4, C4A anaphylatoxin, Rodgers form of C4 | AH002623 | 720 | NM007293 SEQ ID No. 3 | NP009224 SEQ ID No. 4 |
| Complement component 7 | C7 | | J03507 SEQ ID No. 5 | 730 | NM000587 | NP000578 SEQ ID No. 6 |
| Fibroblast growth factor 9 | FGF9 | GAF, HBFG-9, glia-activating factor | D14838 SEQ ID No. 7 | 2254 | NM002010 | NP002001 SEQ ID No. 8 |

(continued)

| Name | Abbreviation | Alias | GenBank | Locus Link | MRNA | Protein |
|---|---|---|---|---|---|---|
| Gremlin | | DRM, IHG-2, CKTSF1B1, cysteine knot superfamily 1, BMPantagonist 1 | AF110137 SEQ ID No. 9 | 26585 | NM013372 | NP037504 SEQ ID No. 10 |
| Intelectin | ITLN | LFR, FLJ20022, endothelial lectin HL-1, intestinal lactoferrin receptor | AK000029 SEQ ID No. 11 | 55600 | NM017625 | NP060095 SEQ ID No. 12 |
| Kallikrein 11 | KLK11 | TLSP, PRSS20, MGC33060, hippostasin | BC022068 SEQ ID No. 13 | 11012 | NM006853 | NP006844 SEQ ID No. 14 |
| Mesothelin | MSLN | MPF, SMR, CAK1 | U40434 SEQ ID No. 15 | 10232 | NM005823 | NP037536 SEQ ID No. 16 |
| Pleiotrophin | PTN | HARP, HBNF, HBGF8, NEGF1 | AB004306 SEQ ID No. 17 | 5764 | NM002825 | NP002816 SEQ ID No. 18 |
| Small inducible cytokine subfamily A member 21 | SCYA21 | CKb9, TCA4, MGC34555, 6CKine, CCL21 chemokine (C-C motif) ligand 21 | AB002409 BI833188 SEQ ID No. 19 | 6366 | NM002989 | NP002980 SEQ ID No. 20 |
| Trefoil Factor 3 | TFF3 | ITF, HITF, human intestinal trefoil factor | L08044 | 7033 | NM003226 SEQ ID No. 21 | NP003217 SEQ ID No. 22 |
| Tissue factor pathway inhibitor 2 | TFPI-2 | PPS, placental protein 5 | D29992 SEQ ID No. 23 | 7980 | NM006528 | NP006519 SEQ ID No. 24 |
| Sulfatase 1 | | | | 23213 | NM015170 SEQ ID No. 25 | NP055985 SEQ ID No. 26 |
| IGFBP2 | | | | 3485 | NM000597 SEQ ID No. 27 | NP000588 SEQ ID No. 28 |
| Cystatin E/M | | | | 1474 | NM001323 SEQ ID No. 29 | NP001314 SEQ ID No. 30 |
| Pregnancy-assoc plasma protein A | | | | 5069 | NM002581 SEQ ID No. 31 | NP002572 SEQ ID No. 32 |

(continued)

| Name | Abbreviation | Alias | GenBank | Locus Link | MRNA | Protein |
|---|---|---|---|---|---|---|
| Butyrlcholinesterase | BCHE-1 | | | 590 | NM000055 SEQ ID No. 33 | NP000046 SEQ ID No. 34 |
| Endothelial lectin HL-2 | Intelectin 2, HLS2-II | | | 142683 | NM080878 SEQ ID No. 35 | NP543154 SEQ ID No. 36 |
| Mesothelin (variant 1), Megakaryocyte potentiating factor precursor | MSLN | MPF, SMR, CAK1 | | 10232 | NM005823 SEQ ID No. 37 | NP005814 SEQ ID No. 38 |

Based on the polypeptides listed in table 1, the present invention provides a marker for measuring the relative amount of visceral adipose tissue present in a subject. This measurement may then be correlated to the diagnosis of metabolic syndrome or an early stage of metabolic syndrome. These markers comprise at least one polypeptide selected from the group consisting of the polypeptides listed in table 1 (SEQ NOs: 2, 4, 6, 8, 10, 12, 14, 16, 18, 20, 22, 24, 26, 28, 30, 32, 34, 36 and 38). Thus, the term "marker" as used herein refers to one or more polypeptides that are predominately expressed in visceral adipose tissue and that can be used to measure the amount of visceral adipose tissue, and therefore, can be used to diagnose metabolic syndrome, a pre-metabolic syndromatic state or a susceptibility to develop metabolic syndrome. The markers may be used either alone or as combinations of multiple polypeptides that are known to be expressed in visceral adipose tissues.

The term "polypeptide" as used herein, refers to a polymer of amino acids, and not to a specific length. Thus, peptides, oligopeptides and proteins are included within the definition of polypeptide.

Preferably, the marker of this invention is a marker comprising at least one polypeptide selected from the group consisting of the polypeptides listed in table 1.

With the identification of polypeptides predominately expressed in visceral adipose tissue, the present invention provides an in vitro method for the measurement of the levels of visceral derived secreted proteins in an individual. The amounts of the measured proteins can be extrapolated to the total amount of visceral adipose tissue in the individual, thereby determining the levels of visceral adipose tissue in an individual. Moreover, it will also be possible to determine whether the visceral adipose tissue differs between individuals, by measuring the levels of visceral derived secreted proteins. Furthermore, differences in secreted proteins can be correlated with different comorbidities found in different individuals.

The term "differentially expressed" or "predominately expressed" in accordance with this invention relates to marker genes which express proteins that are secreted mainly by tissues and/or cells derived from visceral adipose tissue.

In accordance with the present invention, the term "biological sample" as employed herein means a sample which comprises material wherein the differential expression of marker genes may be measured and may be obtained from an individual. Particular preferred samples comprise body fluids, like blood, serum, plasma, urine, synovial fluid, spinal fluid, cerebrospinal fluid, semen or lymph, as well as body tissues, such as visceral adipose tissue.

The detection and/or measurement of the differentially expressed marker genes may comprise the detection of an increase, decrease and/or the absence of a specific nucleic acid molecule, for example RNA or cDNA, the measurement/detection of a expressed polypeptide/protein as well as the measurement/detection of a (biological) activity (or lack thereof) of the expressed protein/polypeptide. The (biological) activity may comprise enzymatic activities, activities relating to signaling pathway-events e.g. antigen-recognition as well as effector-events.

Methods for the detection/measurement of RNA and/or cDNA levels are well known in the art and comprise methods as described in the appended examples. Such methods include, but are not limited to PCR-technology, northern blots, affymetrix chips, and the like.

The term "detection" as used herein refers to the qualitative determination of the absence or presence of polypeptides. The term "measured" as used herein refers to the quantitative determination of the differences in expression of polypeptides in biological samples from patients. Additionally, the term "measured" may also refer to the quantitative determination of the differences in expression of polypeptides in biological samples from visceral adipose tissues.

Methods for detection and/or measurement of polypeptides in biological samples are well known in the art and include, but are not limited to, Western-blotting, ELISAs or RIAs, or various proteomics techniques. Monoclonal or polyclonal antibodies recognizing the polypeptides listed in Table 1, or peptide fragments thereof, can either be generated for the purpose of detecting the polypeptides or peptide fragments, eg. by immunizing rabbits with purified proteins, or known antibodies recognizing the polypeptides or peptide fragments can be used. For example, an antibody capable of binding to the denatured proteins, such as a polyclonal antibody, can be used to detect the peptides of this invention in a Western Blot. An example for a method to measure a marker is an ELISA. This type of protein quantitation is based on an antibody capable of capturing a specific antigen, and a second antibody capable of detecting the captured antigen. A further method for the detection of a diagnostic marker for the measurement of levels of visceral adipose tissue is by analyzing biopsy specimens for the presence or absence of the markers of this invention. Methods for the detection of these markers are well known in the art and include, but are not limited to, immunohistochemistry or immunofluorescent detection of the presence or absence of the polypeptides of the marker of this invention. Methods for preparation and use of antibodies, and the assays mentioned hereinbefore are described in Harlow, E. and Lane, D. Antibodies: A Laboratory Manual, (1988), Cold Spring Harbor Laboratory Press.

While the analysis of one of the polypeptides listed in Table 1 may accurately diagnose levels of visceral adipose tissue, the accuracy of the diagnosis may be increased by analyzing combinations of multiple polypeptides listed in Table 1. Thus, the in vitro method herein before described, comprises a marker which comprises at least two of the polypeptides listed in Table 1.

For diagnosis of visceral adipose tissue levels, suitable biological samples need to be analyzed for the presence or absence of a marker. The biological samples can be serum, plasma, or various tissues including cells of adipose tissue. Cells from adipose tissue can be obtained by any known method, such as ERCP, secretin stimulation, fine-needle aspiration, cytologic brushings and large-bore needle biopsy.

It is also possible to diagnose visceral adipose tissue levels by detecting and/or measuring nucleic acid molecules coding for the marker hereinbefore described. Preferably, the nucleic acid molecule is RNA or DNA.

In one embodiment of the present invention, the in vitro method herein before described comprises comparing the expression levels of at least one of the nucleic acids encoding the polypeptide marker in an individual known to have elevated levels of visceral adipose tissue, to the expression levels of the same nucleic acids in an individual known to have low or normal levels of visceral adipose tissue.

In another embodiment of the present invention the in vitro method herein before described comprises comparing the expression level of the marker in an individual known to have elevated levels of visceral adipose tissue, to the expression levels of the same nucleic acids in an individual known to have low or normal levels of visceral adipose tissue. In a more preferred embodiment of the in vitro method, an increase of the expression levels of the marker is indicative of the susceptibility to develop metabolic syndrome.

Yet, in another embodiment of the present invention, the inventive in vitro method comprises a method, wherein the detection and/or measuring step is carried out by detecting and/or measuring protein(s)/polypeptide(s) or a fragment thereof encoded by the gene(s) as listed in Table 1. Again, these detection/measuring steps comprise methods known in the art, like inter alia, proteomics, immuno-chemical methods like Western-blots, ELISAs and the like.

Preferably, in the in vitro method of the present invention the expression levels of at least two marker genes as listed in Table 1 are compared.

The present invention also provides a screening method for identifying and/or obtaining a compound which interacts with a polypeptide listed in table 1, that is predominantly expressed in visceral adipose tissue, comprising the steps of contacting the polypeptide with a compound or a plurality of compounds under conditions which allow interaction of the compound with the polypeptide; and detecting the interaction between the compound or plurality of compounds with the polypeptide.

For polypeptides that are associated with the cell membrane on the cell surface, or which are expressed as transmembrane or integral membrane polypeptides, the interaction of a compound with the polypeptides can be detected with different methods which include, but are not limited to, methods using cells that either normally express the polypeptide or in which the polypeptide is overexpressed, eg. by detecting displacement of a known ligand which is labeled by the compound to be screened. Alternatively, membrane preparations may be used to test for interaction of a compound with such a polypeptide.

Interaction assays to be employed in the method disclosed herein may comprise FRET-assays (fluorescence resonance energy transfer; as described, inter alia, in Ng, Science 283 (1999), 2085-2089 or Ubarretxena-Belandia, Biochem. 38 (1999), 7398-7405), TR-FRETs and biochemical assays as disclosed herein. Furthermore, commercial assays like "Amplified Luminescent Proximity Homogenous Assay™" (BioSignal Packard) may be employed. Further methods are well known in the art and, inter alia, described in Fernandez, Curr. Opin. Chem. Biol. 2 (1998), 547-603.

The "test for interaction" may also be carried out by specific immunological and/or biochemical assays which are well known in the art and which comprise, e.g., homogenous and heterogenous assays as described herein below. The interaction assays employing read-out systems are well known in the art and comprise, inter alia, two-hybrid screenings (as, described, inter alia, in EP-0 963 376, WO 98/25947, WO 00/02911; and as exemplified in the appended examples), GST-pull-down columns, coprecipitation assays from cell extracts as described, inter alia, in Kasus-Jacobi, Oncogene 19 (2000), 2052-2059, "interaction-trap" systems (as described, inter alia, in US 6,004,746) expression cloning (e.g. lamda gt11), phage display (as described, inter alia, in US 5,541,109), in vitro binding assays and the like. Further interaction assay methods and corresponding read out systems are, inter alia, described in US 5,525,490, WO 99/51741, WO 00/17221, WO 00/14271 or WO 00/05410. Vidal and Legrain (1999) in Nucleic Acids Research 27, 919-929 describe, review and summarize further interaction assays known in the art which may be employed in accordance with the present invention.

Homogeneous (interaction) assays comprise assays wherein the binding partners remain in solution and comprise assays, like agglutination assays. Heterogeneous assays comprise assays like, inter alia, immuno assays, for example, Enzyme Linked Immunosorbent Assays (ELISA), Radioactive Immunoassays (RIA), Immuno Radiometric Assays (IRMA), Flow Injection Analysis (FIA), Flow Activated Cell Sorting (FACS), Chemiluminescent Immuno Assays (CLIA) or Electrogenerated Chemiluminescent (ECL) reporting.

The present invention further provides a screening method for identifying and/or obtaining a compound which is an agonist or an antagonist of a polypeptide listed in Table 1 that is predominantly expressed in visceral adipose tissue, comprising the steps of a) contacting the polypeptide with a compound identified and/or obtained by the screening method described above under conditions which allow interaction of the compound with the polypeptide; b) determining the activity of the polypeptide; c) determining the activity of the polypeptide expressed in the host as defined in (a), which has not been contacted with the compound; and d) quantitatively relating the activity as determined in (b) and (c), wherein a decreased activity determined in (b) in comparison to (c) is indicative for an agonist or antagonist. This screening assay can be performed either as an in vitro assay, or as a host-based assay. The host to be employed in the screening methods of the present invention and comprising and/or expressing a polypeptide listed in Table 1 may comprise prokaryotic as well as eukaryotic cells. The cells may comprise bacterial cells, yeast cells, as well as cultured (tissue) cell lines, inter alia, derived from mammals. Furthermore animals may also be employed as hosts, for example a non-human transgenic animal. Accordingly, the host (cell) may be transfected or transformed with the vector comprising a nucleic acid molecule coding for a polypeptide which is differentially regulated in visceral adipose tissue as disclosed herein. The host cell or host may therefore be genetically modified with a nucleic acid molecule encoding such a polypeptide or with a vector comprising such a nucleic acid molecule. The term "genetically modified" means that the host cell or host comprises in addition to its natural genome a nucleic acid molecule or vector coding for a polypeptide listed in Table 1 or at least a fragment thereof. The additional genetic material may be introduced into the host (cell) or into one of its predecessors/parents. The nucleic acid molecule or vector may be present in the genetically modified host cell or host either as an independent molecule outside the genome, preferably as a molecule which is capable of replication, or it may be stably integrated into the genome of the host cell or host.

Preferably, the present invention further provides a screening method for identifying and/or obtaining a compound which is an antagonist of a polypeptide listed in Table 1 that is predominantly expressed in visceral adipose tissue.

As mentioned herein above, the host cell of the present invention may be any prokaryotic or eukaryotic cell. Suitable prokaryotic cells are those generally used for cloning like E. coli or Bacillus subtilis. Yet, these prokaryotic host cells are also envisaged in the screening methods disclosed herein. Furthermore, eukaryotic cells comprise, for example, fungal or animal cells. Examples for suitable fungal cells are yeast cells, preferably those of the genus Saccharomyces and most preferably those of the species Saccharomyces cerevisiae. Suitable animal cells are, for instance, insect cells, vertebrate cells, preferably mammalian cells, such as e.g. CHO, HeLa, NIH3T3 or MOLT-4. Further suitable cell lines known in the art are obtainable from cell line depositories, like the American Type Culture Collection (ATCC).

A compound which interacts with a polypeptide listed in table 1 and which inhibits or antagonizes the polypeptide is identified by determining the activity of the polypeptide in the presence of the compound.

The term "activity" as used herein relates to the functional property or properties of a specific polypeptide. For the enzymes, the term "activity" relates to the enzymatic activity of a specific polypeptide. For adhesion molecules, the term "activity" relates to the adhesive properties of a polypeptide and may be determined using assays such as, but not limited to, adhesion assays, cell spreading assays, or in vitro interaction of the adhesion molecule with a known ligand. For cytoskeletal proteins, the term "activity" relates to the regulation of the cytoskeleton by such polypeptides, or to their incorporation into the cytoskeleton. As a non-limiting example, the ability of Gelsolin to regulate actin polymerization, or of Filamin A to promote orthogonal branching of actin filaments, may be determined using in vitro actin polymerization assays. Activity in relation to the regulation of cytoskeletal structures may further be determined by, as non-limiting examples, cell spreading assays, cell migration assays, cell proliferation assays or immunofluorescence assays, or by staining actin filaments with fluorescently labeled phalloidin. For ion channels the term "activity" relates to ion flux (Chloride lux) across the membrane. For transcription factors, the term "activity" relates to their ability to regulate gene transcription. The transcriptional activity of a gene can be determined using commonly used assays, such as a reporter gene assay. For growth factors and hormones or their receptors, the term "activity" relates to their ability to bind to their receptors or ligands, respectively, and to induce receptor activation and subsequent signaling cascades, and/or it relates to the factor's or receptor's ability to mediate the cellular function or functions eventually caused by growth factor or hormone mediated receptor activation. Growth factor or hormone binding to receptors can be determined by commonly known ligand binding assays. Receptor activation can be determined by testing for receptor autophosphorylation, or by assaying for modification or recruitment of downstream signaling mediators to the receptors (by immunoprecipitation and Western Blotting of signaling complexes). Cellular functions regulated by growth factors or hormones and their receptors can be cell proliferation (eg determined by using thymidine incorporation or cell counts), cell migration assays (eg determined by using modified Boyden chambers), cell survival or apoptosis assays (eg determined by using DAPI staining), angiogenesis assays (eg in vitro assays to measure endothelial tube formation that are commercially available). In addition to these assays, other assays may be used as well to determine these and other cellular functions.

Inhibitors, antagonists, activators or agonists as identified and/or obtained by the methods of the present invention are particularly useful in the therapeutic management, prevention and or treatment of metabolic syndrome related comorbidities.

Inhibitors or antagonists of a polypeptide listed in Table 1 may be identified by the screening method described above when there is a decreased activity determined in the presence of the compound in comparison to the absence of the compound in the screening method, which is indicative for an inhibitor or antagonist.

Therefore, potential inhibitors or antagonists to be identified, screened for and/or obtained with the method of the present invention include molecules, preferably small molecules which bind to, interfere with and/or occupy relevant sites on the expressed marker genes that are predominately present in visceral adipose tissue.

It is furthermore envisaged that such inhibitors interfere with the synthesis/production of (functional) upregulated marker genes or gene products, like, e.g. anti-sense constructs, ribozymes and the like. The inhibitors and/or antagonist which can be screened for and obtained in accordance with the method of the present invention include, inter alia, peptides, proteins, nucleic acids including DNA, RNA, RNAi, PNA, ribozymes, antibodies, small organic compounds, small molecules, ligands, and the like.

Accordingly, the inhibitor and/or antagonist of differentially expressed marker genes may comprises (an) antibody(ies). The antibody(ies) may comprise monoclonal antibodies as well as polyclonal antibodies. Furthermore, chimeric antibodies, synthetic antibodies as well as antibody fragments (like Fab, F(ab)2, Fv, scFV), or a chemically modified derivative of antibodies are envisaged. It is envisaged that the antibodies bind to the marker gene or its gene product and/or interfere its activity.

In addition, oligonucleotides and/or aptamers which specifically bind to the marker genes as defined herein or which interfere with the activity of the marker genes are envisaged as inhibitors and/or antagonists. The term "oligonucleotide" as used in accordance with the present invention comprises coding and non-coding sequences, it comprises DNA and RNA and/or comprises also any feasible derivative. The term "oligonucleotide" further comprises peptide nucleic acids (PNAs) containing DNA analogs with amide backbone linkages (Nielson, Science 274 (1991), 1497-1500). Oligonucleotides which may inhibit and/or antagonize the marker gene activity and which can be identified and/or obtained by the method of the present invention can be, inter alia, easily chemically synthesized using synthesizers which are well known in the art and are commercially available like, e.g., the AB1 394 DNA-RNA Synthesizers. Additionally, the use of synthetic small interfering dsRNAs of ~22 nt (siRNAs) may be used for suppressing gene expression.

Further to the screening methods disclosed above, this invention provides a screening method for identifying and/or obtaining a compound which is an inhibitor of the expression of a polypeptide listed in table 1 that is predominately expressed in visceral adipose tissue, comprising the steps of a) contacting a host which expresses the polypeptide with a compound; b) determining the expression level and/or activity of the polypeptide; c) determining the expression level and/or activity of the polypeptide in the host as defined in (a), which has not been contacted with the compound; and d) quantitatively relating the expression level of the polypeptide as determined in (b) and (c), wherein a decreased expression level determined in (b) in comparison to (c) is indicative for an inhibitor of the expression of the polypeptide.

An inhibitor of the expression of a polypeptide listed in table 1 is identified by the screening method described hereinbefore when a decreased expression of the protein is determined in the presence of the compound in comparison to the absence of the compound in the screening method, which is indicative for an inhibitor of expression of a polypeptide.

The term "express" as used herein relates to expression levels of a polypeptide listed in table 1 that is predominately expressed in visceral adipose tissue. Preferably, expression levels are at least 2 fold, more preferably at least 3 fold, even more preferably at least 4 fold, most preferably at least 5 fold higher in visceral adipose tissue cells than in, for example, subcutaneous adipose tissue.

Furthermore, the present invention provides a compound identified and/or obtained by any of the screening methods hereinbefore described. The compound is further comprised in a pharmaceutical composition. Any conventional carrier material can be utilized. The carrier material can be an organic or inorganic one suitable for eteral, percutaneous or parenteral administration. Suitable carriers include water, gelatin, gum arabic, lactose, starch, magnesium stearate, talc, vegetable oils, polyalkylene-glycols, petroleum jelly and the like. Furthermore, the pharmaceutical preparations may contain other pharmaceutically active agents. Additional additives such as flavoring agents, stabilizers, emulsifying agents, buffers and the like may be added in accordance with accepted practices of pharmaceutical compounding.

The compound may be used for the preparation of a medicament for the treatment or prevention of metabolic syndrome. In addition, the compound may also be used for the preparation of a diagnostic composition for diagnosing levels of visceral adipose tissue. Preferably, the compound comprises an antibody, an antibody-derivative, an antibody fragment, a peptide or an antisense construct.

Within the scope of the present invention, antibodies against the proteins listed in table 1, or antigen-binding fragments thereof, may be used in an in vitro method for the measurement of levels of visceral adipose tissue.

In order to efficiently perform diagnostic screenings, the present invention provides a kit for the diagnosis of the level of visceral adipose tissue in a patient comprising one or more of the antibodies, or antigen-binding fragments thereof, described above. Another kit provided by this invention is a kit for the diagnosis of the level of visceral adipose tissue in a patient comprising one or more of the nucleic acids coding for the marker hereinbefore described. Yet another kit provided by this invention is a kit for screening of compounds that agonize or antagonize any of the polypeptides listed in table 1, or inhibit the expression of any of the polypeptides.

As mentioned herein above, the inhibitor and/or antagonist may also comprise small molecules. Small molecules, however may also be identified as activators or agonists by the herein disclosed methods. The term "small molecule" relates, but is not limited to small peptides, inorganic and/or organic substances or peptide-like molecules, like peptide-analogs comprising D-amino acids.

Furthermore, peptidomimetics and/or computer aided design of appropriate antagonist, inhibitors, agonists or activators may be employed in order to obtain candidate compounds to be tested in the inventive method. Appropriate computer systems for the computer aided design of, e.g., proteins and peptides are described in the prior art, for example, in Berry, Biochem. Soc. Trans. 22 (1994), 1033-1036; Wodak, Ann. N. Y. Acad. Sci. 501 (1987), 1-13; Pabo, Biochemistry 25 (1986), 5987-5991. The results obtained from the above-described computer analysis can be used in combination with the method of the invention for, e.g., optimizing known compounds, substances or molecules. Appropriate compounds can also be identified by the synthesis of peptidomimetic combinatorial libraries through successive chemical modification and testing the resulting compounds, e.g., according to the methods described herein. Methods for the generation and use of peptidomimetic combinatorial libraries are described in the prior art, for example in Ostresh, Methods in Enzymology 267 (1996), 220-234 and Dorner, Bioorg. Med. Chem. 4 (1996), 709-715. Furthermore, the three-dimensional and/or crystallographic structure of inhibitors activators, agonists or activators of the markers of the present invention or of the nucleic acid molecule encoding the expressed markers can be used for the design of peptidomimetic inhibitors, antagonists, agonists or activators to be tested in the method of the invention (Rose, Biochemistry 35 (1996), 12933-12944; Rutenber, Bioorg. Med. Chem. 4 (1996), 1545-1558).

The compounds to be screened with the method(s) of the present invention do not only comprise single, isolated compounds. It is also envisaged that mixtures of compounds are screened with the method of the present invention. It is also possible to employ extracts, like, inter alia, cellular extracts from prokaryotic or eukaryotic cells or organisms.

In addition, the compound identified or refined by the inventive method can be employed as a lead compound to achieve, modified site of action, spectrum of activity, organ specificity, and/or improved potency, and/or decreased toxicity (improved therapeutic index), and/or decreased side effects, and/or modified onset of therapeutic action, duration of effect, and/or modified pharmakinetic parameters (resorption, distribution, metabolism and excretion), and/or modified physico-chemical parameters (solubility, hygroscopicity, color, taste, odor, stability, state), and/or improved general specificity, organ/tissue specificity, and/or optimized application form and route may be modified by esterification of carboxyl groups, or esterification of hydroxyl groups with carbon acids, or esterification of hydroxyl groups to, e.g. phosphates, pyrophosphates or sulfates or hemi succinates, or formation of pharmaceutically acceptable salts, or formation of pharmaceutically acceptable complexes, or synthesis of pharmacologically active polymers, or introduction of hydrophylic moieties, or introduction/exchange of substituents on aromates or side chains, change of substituent pattern, or modification by introduction of isosteric or bioisosteric moieties, or synthesis of homologous compounds, or introduction of branched side chains, or conversion of alkyl substituents to cyclic analogues, or dramatization of hydroxyl group to ketales, acetales, or N-acetylation to amides, phenylcarbamates, or synthesis of Mannich bases, imines, or transformation of ketones or aldehydes to Schiff's bases, oximes, acetales, ketales, enolesters, oxazolidines, thiozolidines or combinations thereof.

Additionally, the invention provides for the use of a compound or a plurality of compounds which is obtainable by the method disclosed herein for the preparation of a diagnostic composition for diagnosing the level of visceral adipose tissue in a patient. It is, for example envisaged that specific antibodies, fragments thereof or derivatives thereof which specifically detect or recognize differentially expressed marker gene products as disclosed herein be employed in such diagnostic compositions. Yet, specific primers/primer pairs which may detect and/or amplify the marker gene of the present invention may be employed in the diagnostic compositions.

Accordingly, the compound to be used in the pharmaceutical as well as in the diagnostic composition may comprises an antibody, an antibody-derivative, an antibody fragment, a peptide or a nucleic acid, like primers/primer pairs as well as anti-sense constructs, RNAi or ribozymes. Thus, a therapeutically effective amount of at least one antibody against at least one protein, or antigen-binding fragment thereof, or of at least one protein, protein fragment or peptide selected from the group consisting of the proteins having Seq ID Nos. 2, 4, 6, 8, 10, 12, 14, 16, 18, 20, 22, 24, 26, 28, 30, 32, 34, 36 and 38, can be used for the preparation of a medicament for the treatment of metabolic syndrome.

The diagnostic composition may also comprise suitable means for detection known in the art.

The present invention also provides the use of a nucleic acid selected from the group consisting of the nucleic acids of SEQ ID No. 1, 3, 5, 7, 9, 11, 13, 15, 17, 19, 21, 23, 25, 27, 29, 31, 33, 35, and 37 as a marker in the diagnosis of visceral adipose tissue accumulation; and the use of a polypeptide selected from the group consisting of the proteins of SEQ ID No. 2, 4, 6, 8, 10, 12, 14, 16, 18, 20, 22, 24, 26, 28, 30, 32, 34, 36, and 38, as a marker for the diagnosis of visceral adipose tissue accumulation.

The invention is further described by reference to the following biological examples which are merely illustrative and are not to be construed as a limitation of scope.

**Examples:**

Total RNA was extracted using Ultraspec® RNA (Biotecx, Houston, TX) according to the manufacturer's protocol, and purified using the RNeasy Mini kit (Qiagen, Valencia, CA) with DNase treatment. Double-stranded cDNA was synthesized from 10 ug total RNA by SuperScript™ Double-Stranded cDNA Synthesis Kit (Life Technology, Rockville, MD) using the T7-T24 primer. The double-stranded cDNA product was purified by phenol/chloroform/isoamyl extraction using phase lock gels (Eppendorf, Westbury, NY). Double-stranded cDNA was further converted into cRNA using the *in vitro* transcription (IVT) MEGAscript™ T7 kit (Ambion, Austin, TX) and labelled with biotinylated nucleotides[1]. The *in vitro* transcription product was purified using the RNeasy Mini kit (Qiagen, Valencia, CA), and fragmented as described (Wodicka L, Dong H, Mittmann M, Ho MH, Lockhart DJ. Genome-wide expression monitoring in Saccharomyces cerevisiae. Nat Biotechnol 1997;15:1359-67). Hybridization of the fragmented *in vitro* transcription product to the Human Genome U95 (HG-U95) Genechip® array set was performed as suggested by the manufacturer (Affymetrix, Santa Clara, CA).

Statistical Methods

All numeric analyses were conducted on signal intensities as reported by the Affymetrix's MAS algorithms (Affymetrix Technical Note: New Statistical Algorithms for Monitoring Gene Expression on GeneChip® Probe Arrays. (2001)). Chips were each standardized to the overall mean of the all of the chips in the experiment. Genes were not separately standardized.

The analysis of the data was constructed as a linear model (Draper N., Smith H. Applied Regression Analysis, Second Edition John Wiley and Sons. New York, New York. (1966); Searle S. R. Linear Models John Wiley and Sons. New York, New York. (1971)) with factors for BMI, tissue of origin (subcutaneous vs. visceral adipose), insulin resistance (measured by HOMA), fasting glucose, fasting insulin and the interactions between tissue of origin and fasting glucose, fasting insulin, and insulin resistance respectively. Calculations were done using SAS version 8.1. The equation for the model is as follows:

$$\text{Signal Intensity} = \text{BMI} + \text{tissue} + \text{IR} + \text{glucose} + \text{insulin} + \text{tissue}{*}\text{IR} + \text{tissue}{*}\text{gluocose} + \text{tissue}{*}\text{insulin} + \text{error}$$

Nine statistical tests (contrasts) were then performed using this model. 1) Effect in visceral adipose; 2) Effect in subcutaneous adipose; 3) Differential effect between visceral and subcutaneous adipose. Each of those three tests was performed with the three interaction terms resulting in the final 9 tests.

Results of the model calculations and statistical contrasts were then filtered to result in the final genes of interest. Significance was defined as a p-value for the entire model less than 0.001 and a p-value for the specific contrast of less than 0.01. The p-value cutoffs were chosen so as to control for false positives while still finding the majority of true positives (Sokal R. R., Rohlf F. J. Biometry W. H. Freeman and Company. New York, New York. (1969)).

Finally genes were annotated through linking the Genbank accession numbers provided by Affymetrix with the Unigene http://www.ncbi.nlm.nih.gov /entrez/query.fcgi?db=unigene) and LocusLink (http://www.ncbi.nlm.nih.gov /LocusLink/) annotations for those accession numbers.

All references discussed throughout the above specification are herein incorporated in their entirety by reference for the subject matter they contain.

It should be understood, of course, that the foregoing relates to preferred embodiments of the invention and that modifications may be made without departing from the spirit and scope of the invention as set forth in the following claims.

## Claims

1. A method for the measurement of levels of visceral adipose tissue comprising: detecting or measuring the level of a polypeptide marker in a biological sample, wherein said polypeptide marker comprises a polypeptide of SEQ ID No. 12.

2. The method of claim 1, wherein said biological sample is derived from the group consisting of serum, plasma, and cells of visceral adipose tissue.

3. The method of claim 1 or 2, wherein the level of said polypeptide marker in an individual known to have elevated levels of visceral adipose tissue is compared to the expression levels of the same polypeptide marker in an individual known to have low or normal levels of visceral adipose tissue.

4. The methods of any one of claims 1 to 3, wherein an increase of the level of said polypeptide marker over time is indicative of metabolic syndrome or the susceptibility to metabolic syndrome.

5. A method for measuring the level of visceral adipose tissue in a subject comprising:

   detecting or measuring the level of a nucleic acid marker in a biological sample wherein said nucleic acid marker comprises a nucleic acid molecule of SEQ ID No. 11.

6. The method of claim 5, wherein said nucleic acid marker is RNA.

7. The method claim 5 or 6, wherein the expression level of said nucleic acid marker in an individual known to have elevated levels of visceral adipose tissue is compared to the expression levels of the same polypeptide marker in an individual known to have low or normal levels of visceral adipose tissue.

8. The method of any one of claims 5 to 7, wherein an increase of the expression level of said nucleic acid marker over time is indicative of metabolic syndrome or the susceptibility to metabolic syndrome.

9. A screening method for identifying a compound which interacts with a polypeptide that is predominately expressed in visceral adipose tissue, wherein said polypeptide comprises SEQ ID No. 12, said method comprising:

   contacting said polypeptide with a compound or a plurality of compounds under conditions which allow interaction of said compound with said polypeptide; and
   detecting the interaction between said compound or plurality of compounds with said polypeptide.

**10.** A screening method for identifying a compound which is an agonist or an antagonist of a polypeptide that is predominately expressed in visceral adipose tissue, wherein said polypeptide comrprises SEQ ID No. 12, said method comprising:

contacting said polypeptide with a compound under conditions which allow interaction of said compound with said polypeptide;
determining a first level of activity of said polypeptide;
determining a second level of activity of said polypeptide expressed in a host which has not been contacted with said compound; and
quantitatively relating the first level of activity with the second level of activity, wherein when said first level of activity is less than said second level of activity, said compound is identified as an antagonist of said polypeptide.

**11.** A screening method for identifying a compound which is an inhibitor of the expression of a polypeptide that is predominately expressed in visceral adipose tissue, wherein said polypeptide comprises SEQ ID No. 12, said method comprising:

contacting a host which expresses said polypeptide with a compound;
determining a first expression level or activity of said polypeptide;
determining a second expression level or activity of said polypeptide in a host which has not been contacted with said compound; and
quantitatively relating the first expression level or activity with the second expression level or activity, wherein when said first expression level or activity is less than said second expression level or activity, said compound is identified as an inhibitor of the expression of said polypeptide.

**12.** A method of correlating protein levels in a mammal with a diagnosis of the level of visceral adipose tissue, comprising:

determining the level of a protein comprising SEQ. ID. No. 12 in a biological sample of said mammal; and
generating an index number, Y, which indicates a base level of visceral adipose tissue.

**13.** The method according to claim 12, further comprising comparing index number, Y, to index numbers of subjects known to have specified levels of visceral adipose tissue.

**14.** The method of claim 12 or 13, further comprising monitoring said index number, Y, over time, to determine the progression of the level of visceral adipose tissue, thereby predicting a susceptibility to developing metabolic syndrome.

**15.** Use of a nucleic acid comprising SEQ ID No. 11 as a marker in the diagnosis of visceral adipose tissue accumulation.

**16.** Use of a polypeptide comprising SEQ ID No. 12 as a marker for the diagnosis of visceral adipose tissue accumulation.

Figure 1

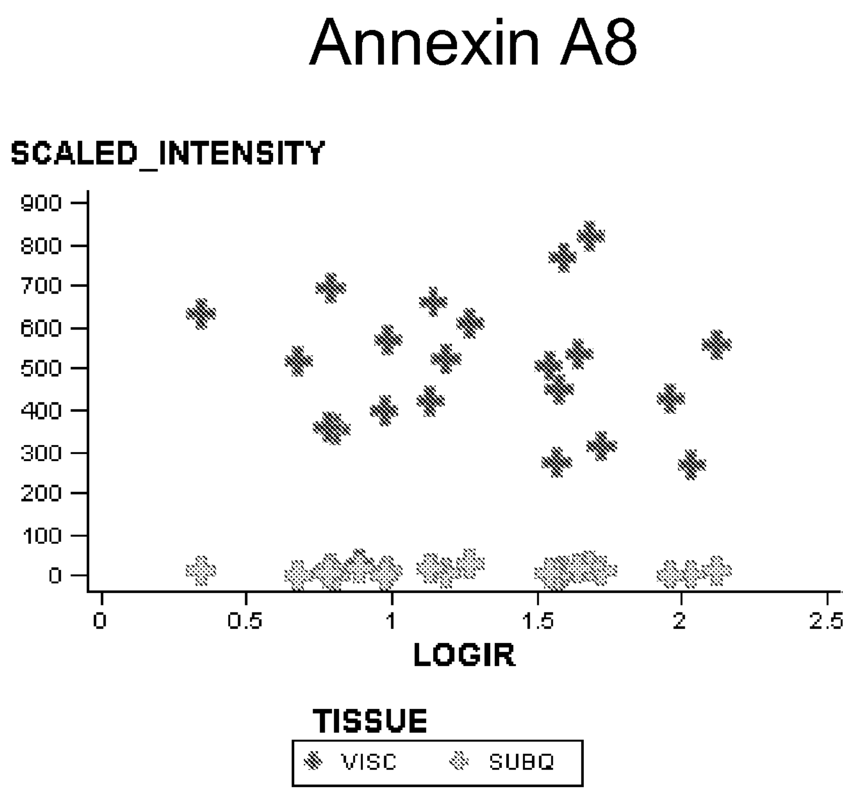
Annexin A8
SCALED_INTENSITY
900
800
700
600
500
400
300
200
100
0
0
0.5
1
1.5
2
2.5
LOGIR
TISSUE
VISC
SUBQ

Figure 2

Complement component 4A

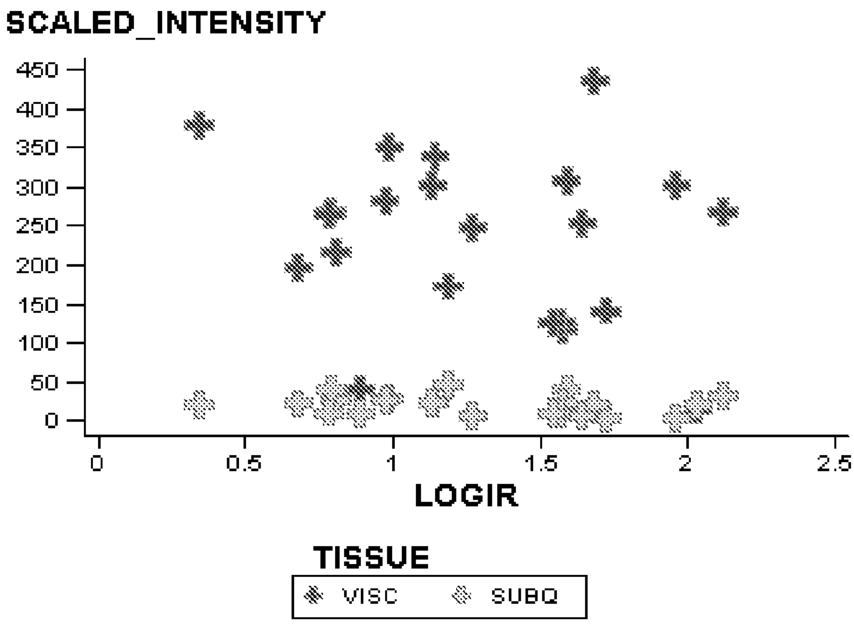

Figure 3

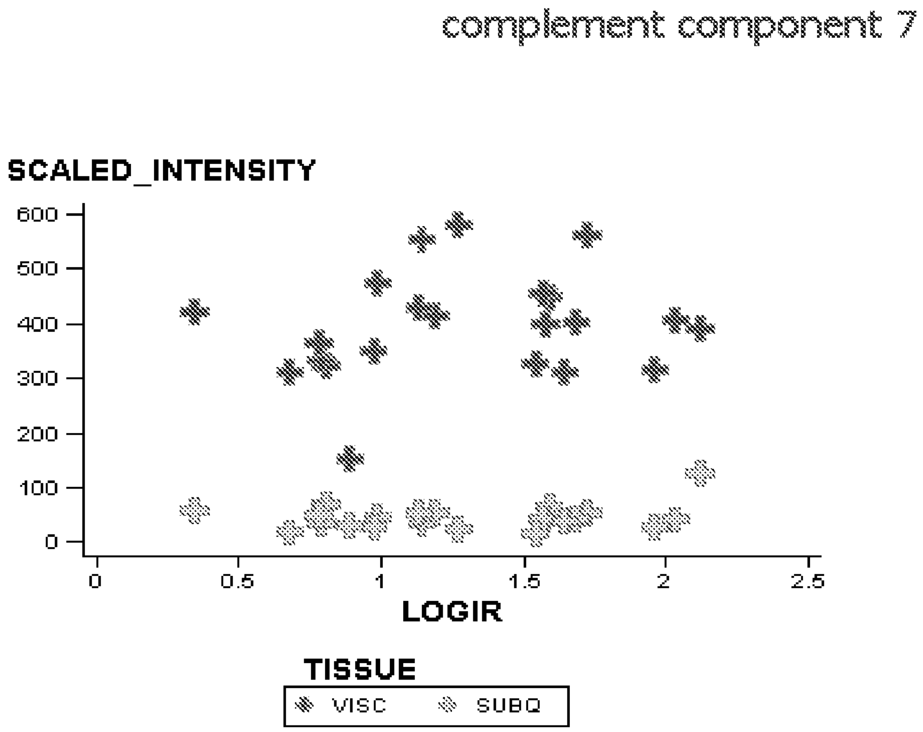
complement component 7
SCALED_INTENSITY
600
500
400
300
200
100
0
0
0.5
1
1.5
2
2.5
LOGIR
TISSUE
VISC
SUBQ

Figure 4

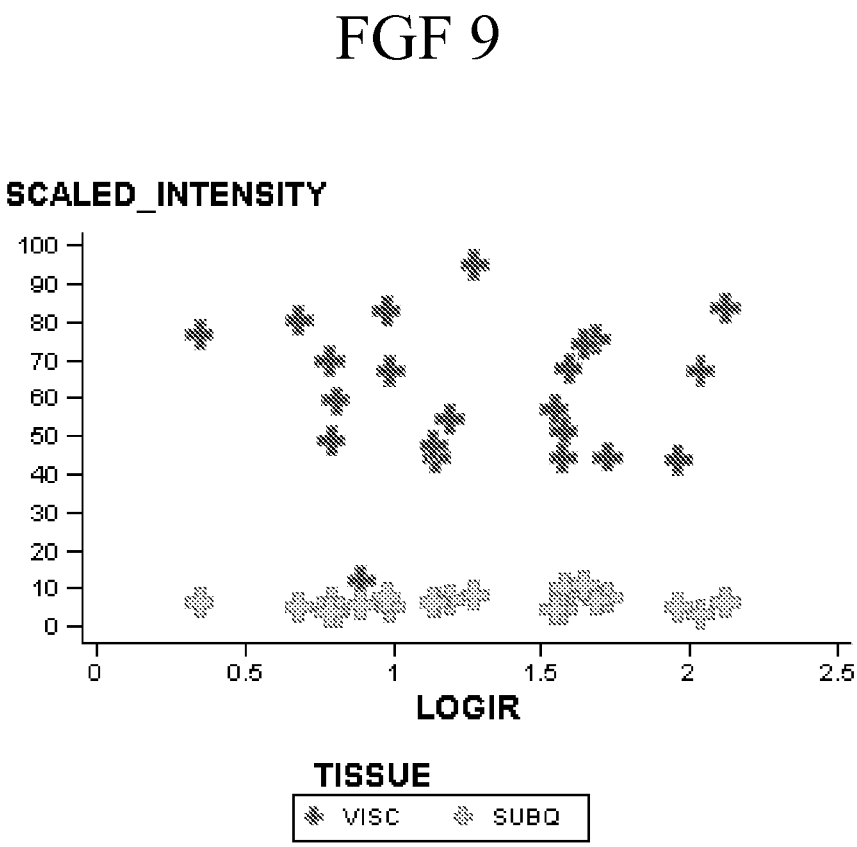
FGF 9
SCALED_INTENSITY
100
90
80
70
60
50
40
30
20
10
0
0
0.5
1
1.5
2
2.5
LOGIR
TISSUE
VISC
SUBQ

Figure 5

cysteine knot superfamily 1,
BMP antagonist 1

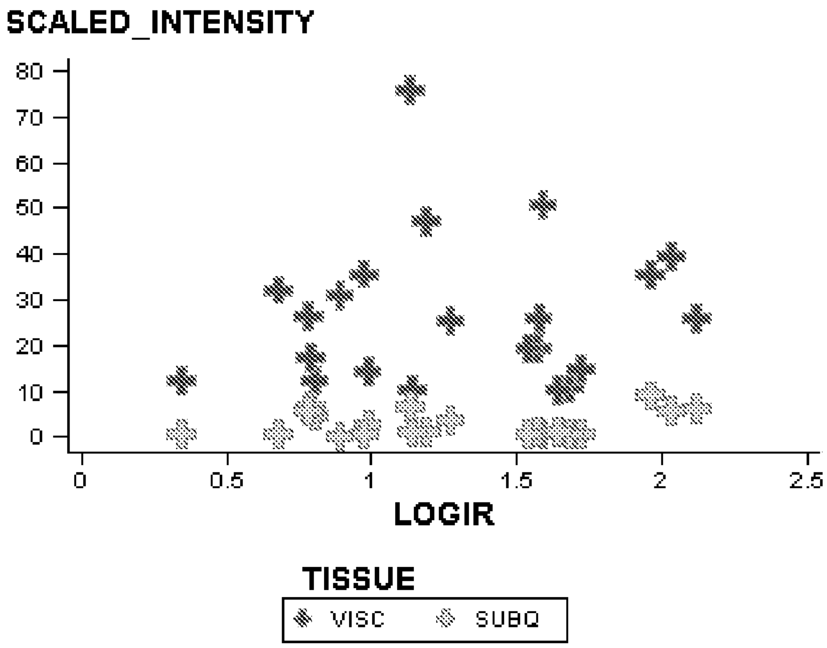

Figure 6

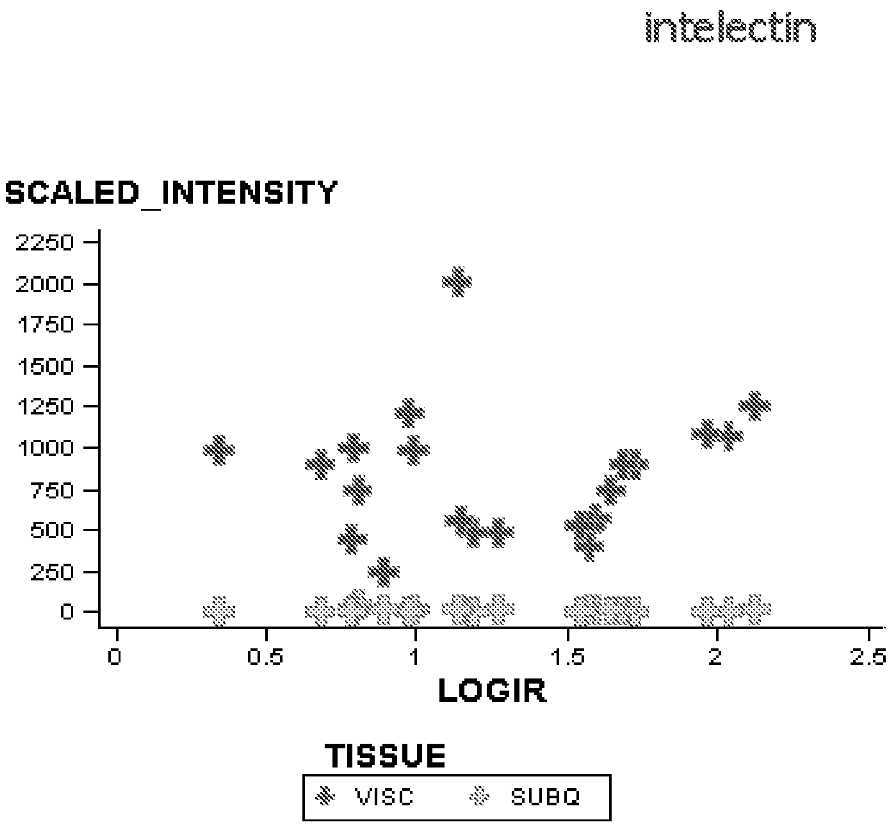
intelectin
SCALED_INTENSITY
2250
2000
1750
1500
1250
1000
750
500
250
0
0
0.5
1
1.5
2
2.5
LOGIR
TISSUE
VISC
SUBQ

Figure 7

Kallikrein 11

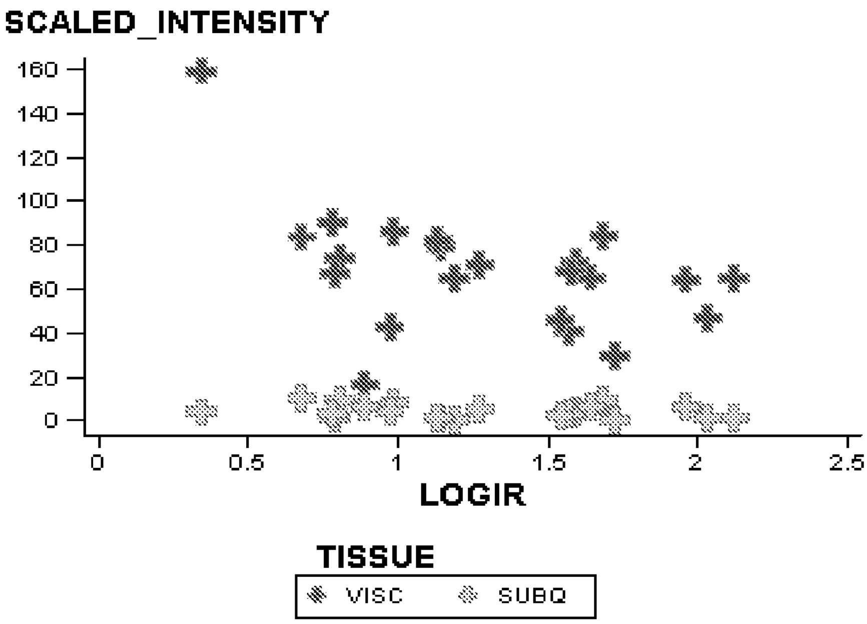

Figure 8

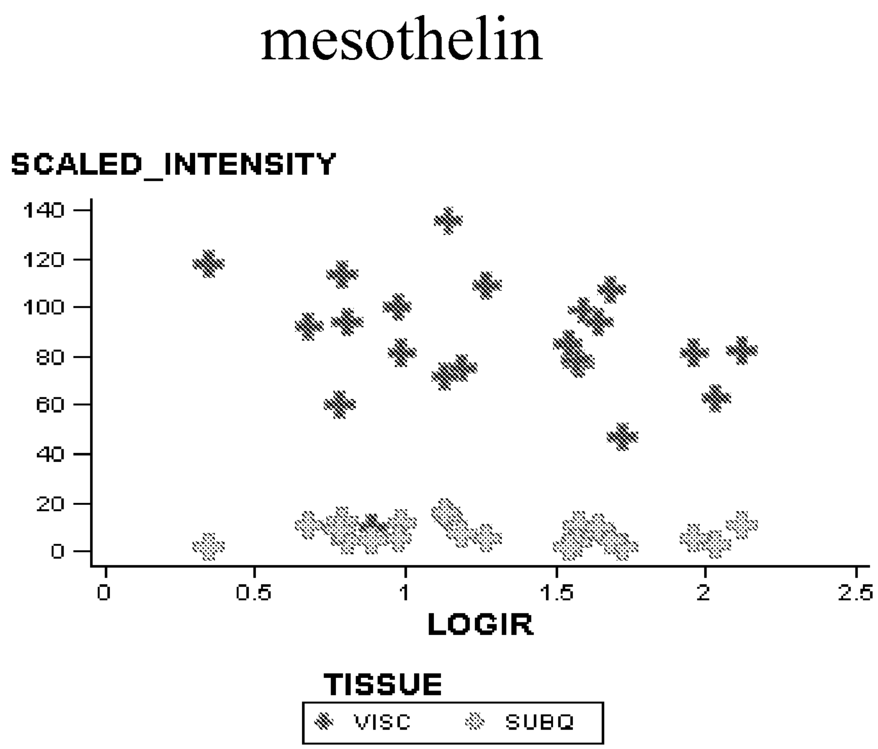
mesothelin
SCALED_INTENSITY
140
120
100
80
60
40
20
0
0
0.5
1
1.5
2
2.5
LOGIR
TISSUE
VISC
SUBQ

Figure 9

pleiotrophin

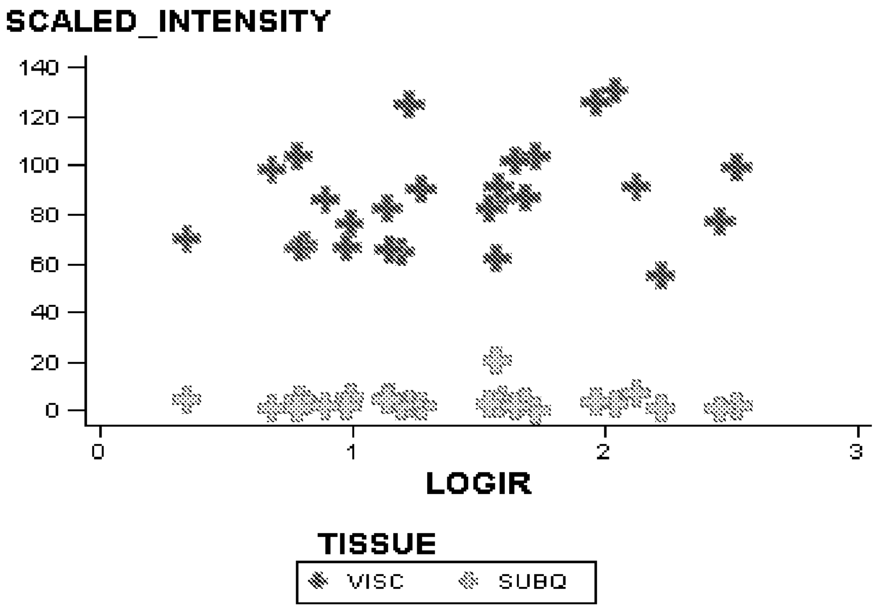

Figure 10

small inducible cytokine subfamily A (Cys-Cys), member 21

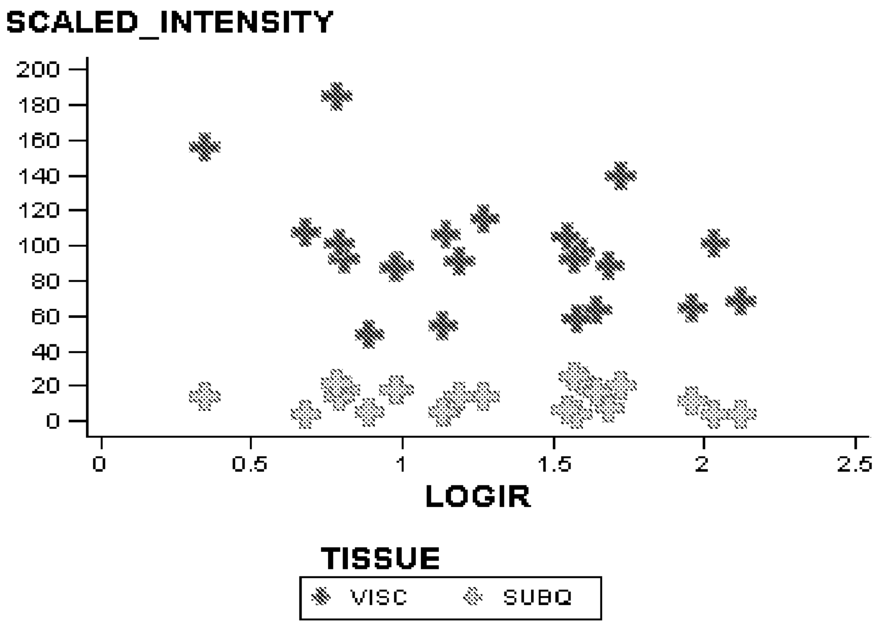

Figure 11

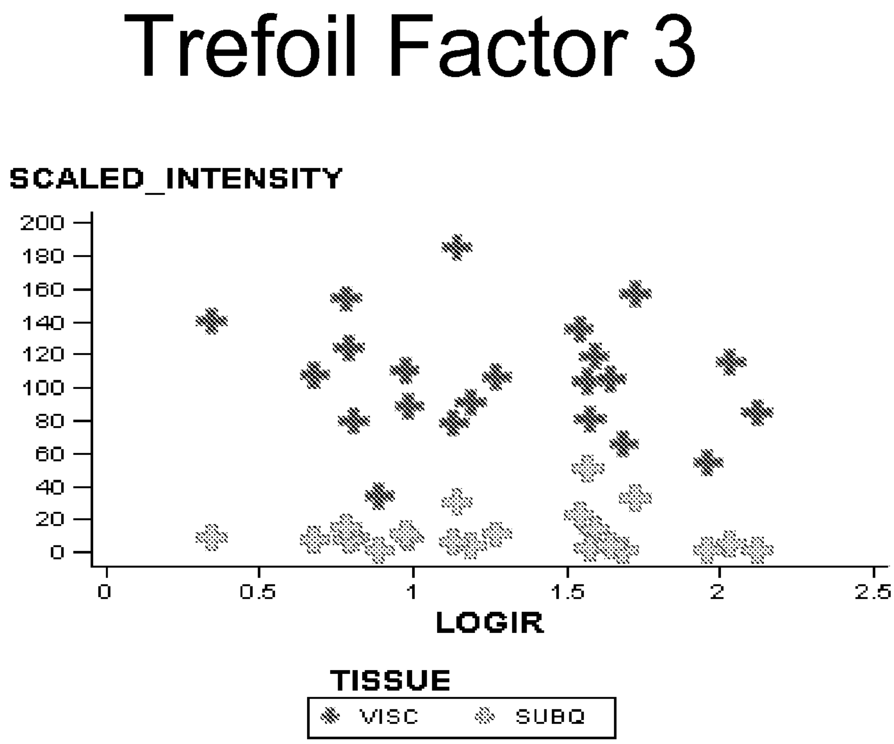
Trefoil Factor 3
SCALED_INTENSITY
200
180
160
140
120
100
80
60
40
20
0
0
0.5
1
1.5
2
2.5
LOGIR
TISSUE
VISC
SUBQ

Figure 12

tissue factor pathway inhibitor 2

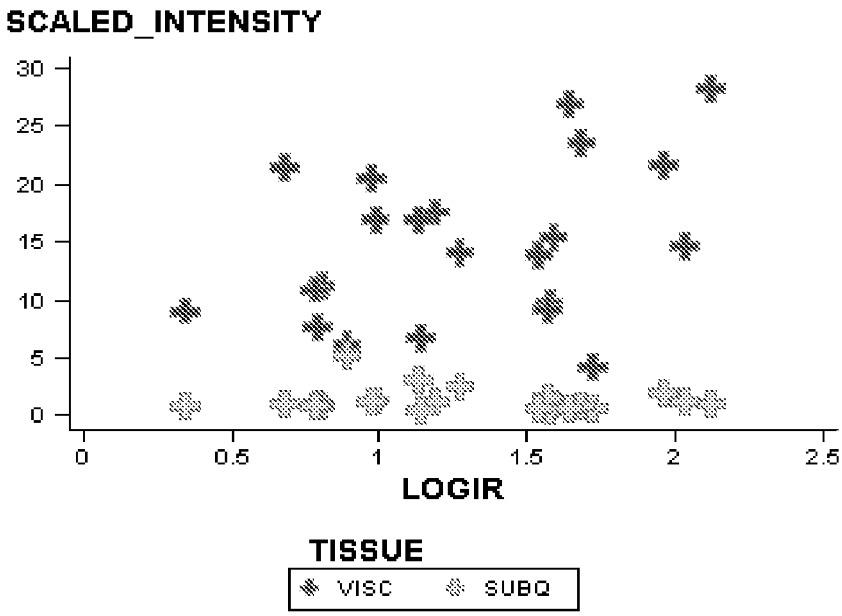

Figure 13

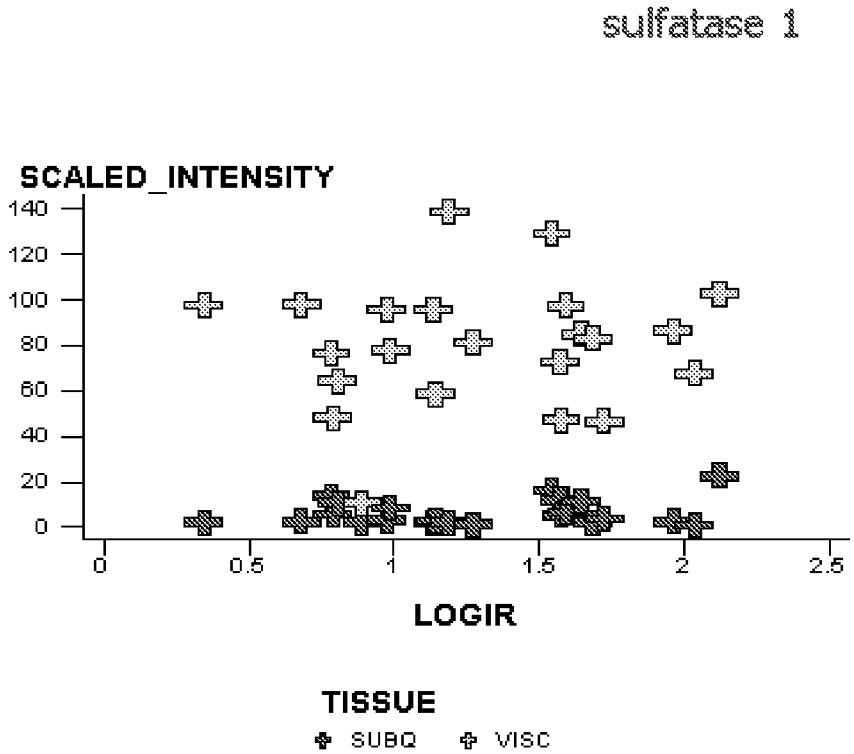
sulfatase 1
SCALED_INTENSITY
140
120
100
80
60
40
20
0
0
0.5
1
1.5
2
2.5
LOGIR
TISSUE
SUBQ
VISC

Figure 14

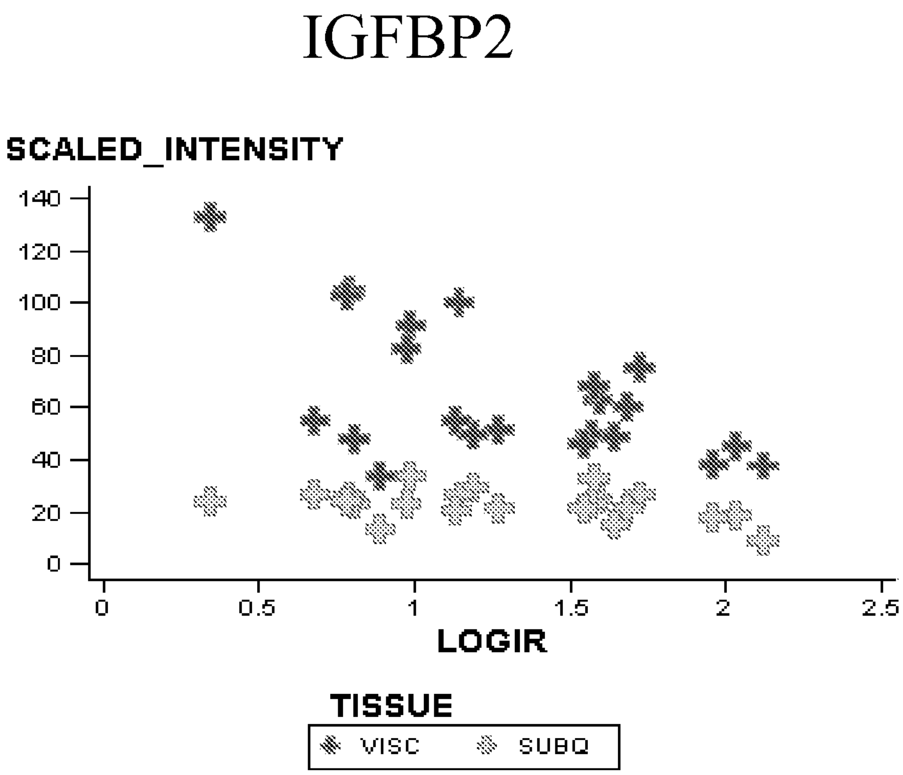
IGFBP2
SCALED_INTENSITY
140
120
100
80
60
40
20
0
0
0.5
1
1.5
2
2.5
LOGIR
TISSUE
VISC
SUBQ

Figure 15

cystatin E/M

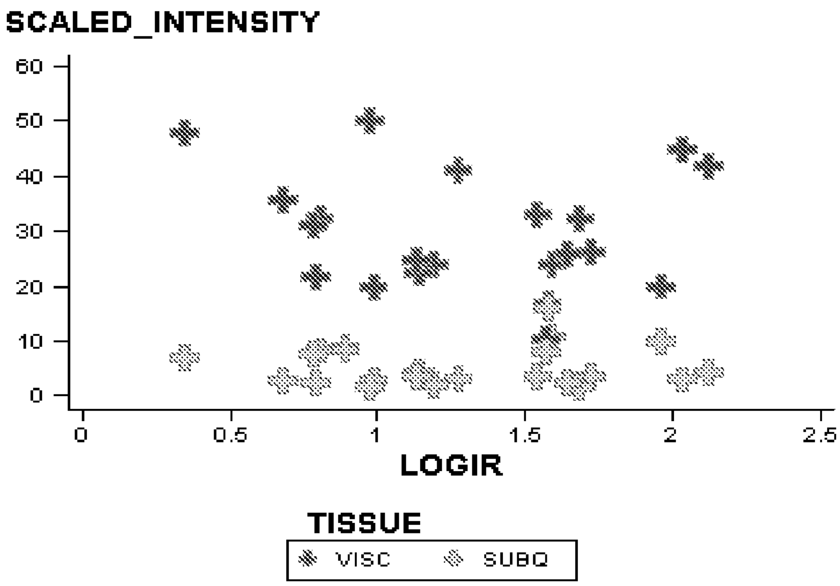

Figure 16

pregnancy-associated plasma protein A

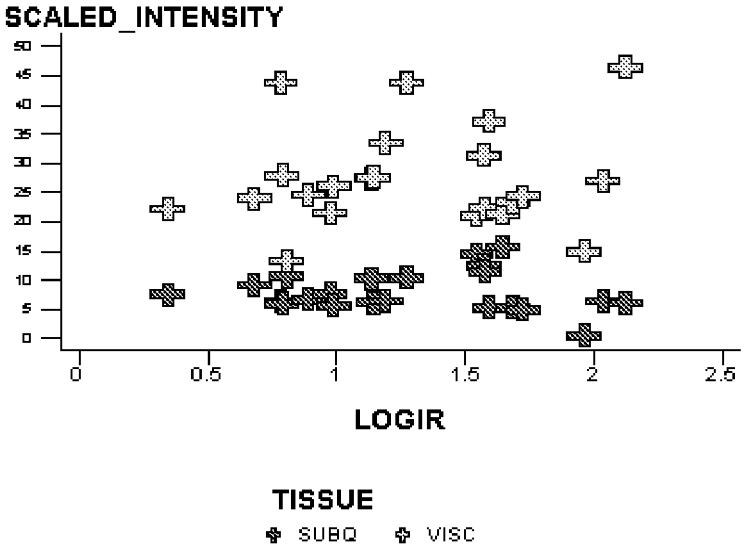

Figure 17

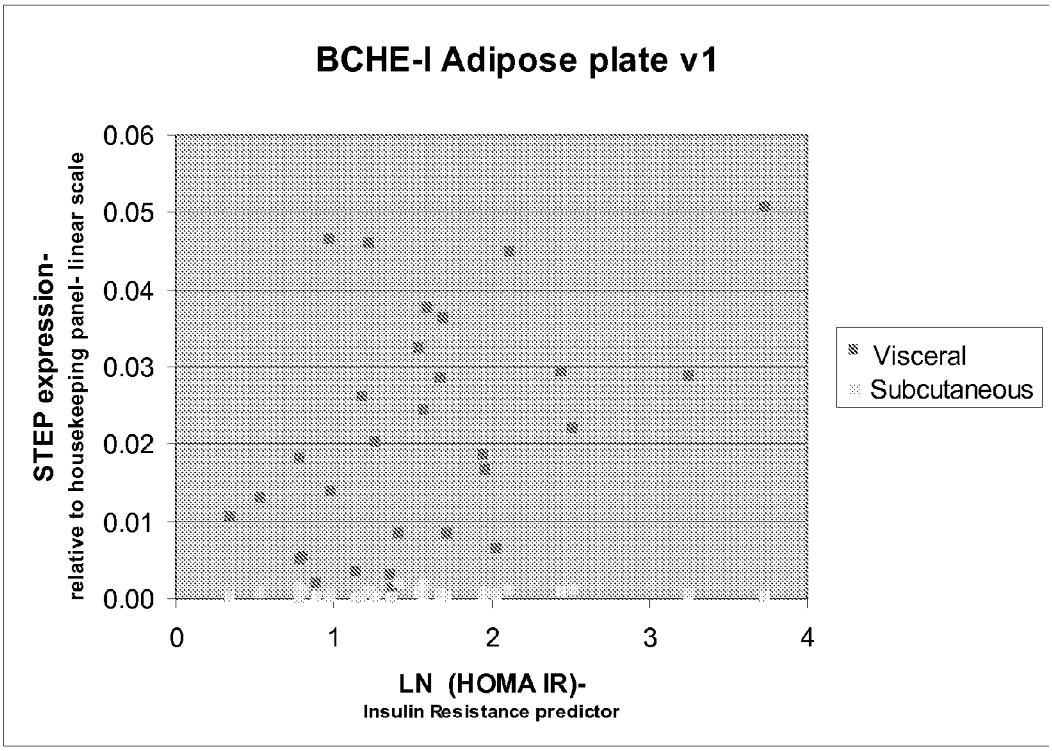
BCHE-I Adipose plate v1
STEP expression-
relative to housekeeping panel- linear scale
0.06
0.05
0.04
0.03
0.02
0.01
0.00
0
1
2
3
4
LN (HOMA IR)-
Insulin Resistance predictor
Visceral
Subcutaneous

Figure 18

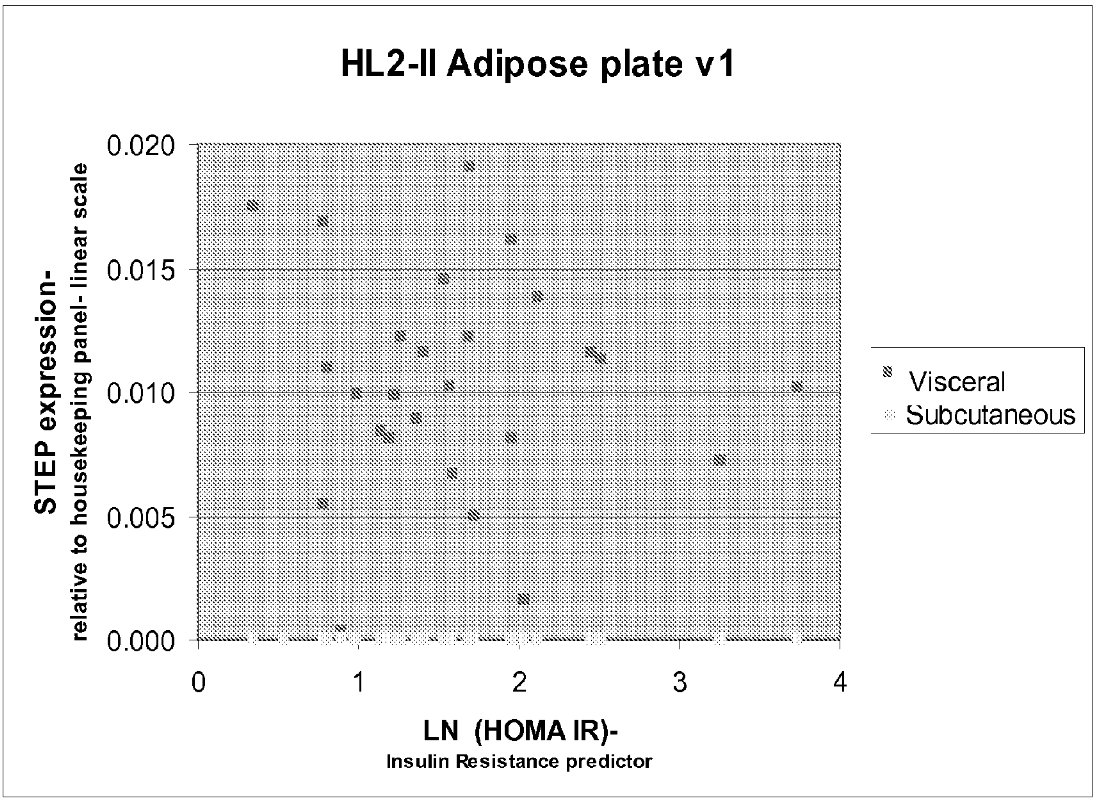
HL2-II Adipose plate v1
STEP expression-
relative to housekeeping panel- linear scale
0.020
0.015
0.010
0.005
0.000
0
1
2
3
4
LN (HOMA IR)-
Insulin Resistance predictor
Visceral
Subcutaneous

Europäisches Patentamt
European Patent Office
Office européen des brevets

# EUROPEAN SEARCH REPORT

Application Number
EP 09 15 1175

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KATZMARZYK PETER T ET AL: "Genetics of abdominal visceral fat levels"<br>AMERICAN JOURNAL OF HUMAN BIOLOGY,<br>vol. 11, no. 2, 1999, pages 225-235, XP008043512<br>ISSN: 1042-0533<br>* abstract *<br>----- | 1-8,15, 16 | INV.<br>G01N33/68<br>A61K31/16<br>A61K39/395 |
| X | US 2003/082533 A1 (YUE HENRY [US] ET AL)<br>1 May 2003 (2003-05-01)<br>* sequences 1-7 *<br>* page 2, paragraph 13 *<br>* page 3, paragraphs 18,20 *<br>* page 5, paragraph 53 *<br>* page 8, paragraph 91 - page 10, paragraph 115 *<br>* claims 1-21 *<br>----- | 9-11 | |
| A | WEBER-HAMANN BETTINA ET AL: "Hypercortisolemic depression is associated with increased intra-abdominal fat"<br>PSYCHOSOMATIC MEDICINE,<br>vol. 64, no. 2, March 2002 (2002-03), pages 274-277, XP002318886<br>ISSN: 0033-3174<br>* abstract *<br>* page 275, column 1, paragraph 5 *<br>-----<br>-/-- | 1-16 | TECHNICAL FIELDS SEARCHED (IPC)<br>G01N<br>A61K |

The present search report has been drawn up for all claims

| Place of search | Date of completion of the search | Examiner |
|---|---|---|
| The Hague | 19 March 2009 | van der Kooij, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

8

EPO FORM 1503 03.82 (P04C01)

Europäisches Patentamt
European Patent Office
Office européen des brevets

# EUROPEAN SEARCH REPORT

Application Number
EP 09 15 1175

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BROCHU MARTIN ET AL: "Visceral adipose tissue is an independent correlate of glucose disposal in older obese postmenopausal women" JOURNAL OF CLINICAL ENDOCRINOLOGY AND METABOLISM, vol. 85, no. 7, July 2000 (2000-07), pages 2378-2384, XP002318887 ISSN: 0021-972X * page 2379, column 1, paragraph 4 - paragraph 5 * ----- | 1-16 | |
| A | TSUJI SHOUTARO ET AL: "Human intelectin is a novel soluble lectin that recognizes galactofuranose in carbohydrate chains of bacterial cell wall" JOURNAL OF BIOLOGICAL CHEMISTRY, vol. 276, no. 26, 29 June 2001 (2001-06-29), pages 23456-23463, XP002519863 ISSN: 0021-9258 * the whole document * ----- | 1-16 | TECHNICAL FIELDS SEARCHED (IPC) |
| P,X | US 2004/220099 A1 (GONG DA-WEI [US] ET AL) 4 November 2004 (2004-11-04) * page 1, paragraph 4 - paragraph 5 * * page 3, paragraphs 21,32 * * page 5, paragraphs 56,59 * * figures 1,2,7,8 * * page 8, paragraph 78 - paragraph 80; claims 17-19 * ----- | 1-16 | |

The present search report has been drawn up for all claims

| Place of search | Date of completion of the search | Examiner |
|---|---|---|
| The Hague | 19 March 2009 | van der Kooij, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.....................................................................
& : member of the same patent family, corresponding document

8

EPO FORM 1503 03.82 (P04C01)

## ANNEX TO THE EUROPEAN SEARCH REPORT ON EUROPEAN PATENT APPLICATION NO. EP 09 15 1175

This annex lists the patent family members relating to the patent documents cited in the above-mentioned European search report.
The members are as contained in the European Patent Office EDP file on
The European Patent Office is in no way liable for these particulars which are merely given for the purpose of information.

19-03-2009

| Patent document cited in search report | Publication date | Patent family member(s) | Publication date |
|---|---|---|---|
| US 2003082533 A1 | 01-05-2003 | NONE | |
| US 2004220099 A1 | 04-11-2004 | US 2008085864 A1 | 10-04-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9604912 A
- EP 0963376 A
- WO 9825947 A
- WO 0002911 A
- US 6004746 A
- US 5541109 A
- US 5525490 A
- WO 9951741 A
- WO 0017221 A
- WO 0014271 A
- WO 0005410 A


### Non-patent literature cited in the description


- **MARIN, P.** Neuro-endocrine Syndrome. *Neuroendocrine News,* 1996, vol. 21 (3), 2
- **HARLOW, E. ; LANE, D.** Antibodies: A Laboratory Manual. Cold Spring Harbor Laboratory Press, 1988
- **NG.** *Science,* 1999, vol. 283, 2085-2089
- **UBARRETXENA-BELANDIA.** *Biochem.,* 1999, vol. 38, 7398-7405
- **FERNANDEZ.** *Curr. Opin. Chem. Biol.,* 1998, vol. 2, 547-603
- **KASUS-JACOBI.** *Oncogene,* 2000, vol. 19, 2052-2059
- **VIDAL ; LEGRAIN.** *Nucleic Acids Research,* 1999, vol. 27, 919-929
- **NIELSON.** *Science,* 1991, vol. 274, 1497-1500
- **BERRY.** *Biochem. Soc. Trans.,* 1994, vol. 22, 1033-1036
- **WODAK.** *Ann. N. Y. Acad. Sci.,* 1987, vol. 501, 1-13
- **PABO.** *Biochemistry,* 1986, vol. 25, 5987-5991
- **OSTRESH.** *Methods in Enzymology,* 1996, vol. 267, 220-234
- **DORNER.** *Bioorg. Med. Chem.,* 1996, vol. 4, 709-715
- **ROSE.** *Biochemistry,* 1996, vol. 35, 12933-12944
- **RUTENBER.** *Bioorg. Med. Chem.,* 1996, vol. 4, 1545-1558
- **WODICKA L ; DONG H ; MITTMANN M ; HO MH ; LOCKHART DJ.** Genome-wide expression monitoring in Saccharomyces cerevisiae. *Nat Biotechnol,* 1997, vol. 15, 1359-67
- **DRAPER N. ; SMITH H.** Applied Regression Analysis. John Wiley and Sons, 1966
- **SEARLE S. R.** Linear Models. John Wiley and Sons, 1971
- **SOKAL R. R. ; ROHLF F.** J. Biometry. W. H. Freeman and Company, 1969

SEQUENCE LISTING

<110> F. Hoffmann-La Roche AG

<120> SPECIFIC MARKERS FOR METABOLIC SYNDROME

<130> 21742 EP1

<150> US60/523845
<151> 2003-11-20

<150> US60/523845
<151> 2003-11-20

<150> EP04026956.5
<151> 2004-11-12

<160> 38

<170> PatentIn version 3.5

<210> 1
<211> 1940
<212> DNA
<213> Homo sapiens

<220>
<221> Annexin A8
<222> (1)..(1940)
<223> X16662

<400> 1
aggcctgctc actcctcagc tgcaggagcc agacgtgtgg agtcccagca gaggccaacc 60

tgtgtctctt catctccgtg agaaaggtgc ccccgaagtg aaagagatgg cctggtggaa 120

agcctggatt gaacaggagg gtgtcacagt gaagagcagc tcccacttca acccagaccc 180

tgatgcagag accctctaca aagccatgaa ggggatcggg accaacgagc aggctatcat 240

cgatgtgctc accaagagaa gcaacacgca gcggcagcag atcgccaagt ccttcaaggc 300

tcagttcggc aaggacctca ctgagacctt gaagtctgag ctcagtggca agtttgagag 360

gctcattgtg gcccttatgt atccgccata cagatacgaa gccaaggagc tgcatgacgc 420

catgaagggc ttaggaacca aggagggtgt catcattgag atcctggcct ctcggaccaa 480

gaaccagctg cgggagataa tgaaggcgta tgaggaagac tatgggtcca gcctggagga 540

ggacatccaa gcagacacaa gtggctacct ggagaggatc ctggtgtgcc tcctgcaggg 600

cagcagggat gatgtgagca gctttgtgga cccggcactg gccctccaag acgcacagga 660

tctgtatgcg gcaggcgaga agattcgtgg gactgatgag atgaaattca tcaccatcct 720

gtgcacgcgc agtgccactc acctgctgag agtgtttgaa gagtatgaga aaattgccaa 780

caagagcatt gaggacagca tcaagagtga gacccatggc tcactggagg aggccatgct 840

cactgtggtg aaatgcaccc aaaacctcca cagctacttt gcagagagac tctactatgc 900

catgaaggga gcagggacgc gtgatgggac cctgataaga aacatcgttt caaggagcga 960

gattgactta aatcttatca aatgtcactt caagaagatg tacggcaaga ccctcagcag 1020

catgatcatg gaagacacca gcggcgacta caagaacgcc ctgctgagcc tggtgggcag 1080

cgacccctga ggcacagaag aacaagagca aagaccatga agccagagtc tccaggactc 1140
ctcactcaac ctcggccatg gacgcaggtt gggtgtgagg ggggtcccag cctttcggtc 1200
ttctatttcc ctatttccag tgctttccag ccgggtttct gacccagagg tggaaccggc 1260
ctggactcct cttcccaact tcctccaggt catttcccag tgtgagcaca atgccaacct 1320
tagtgtttct ccagccagac agatgcctca gcatgaaggg cttggggact tgtggatcat 1380
tccttcctcc ctgcaggagc ttcccaagct ggtcacagag tctcctgggc acaggttata 1440
cagaccccag ccccattccc atctactgaa acagggtctc cacaagaggg gccagggaat 1500
atgggttttt aacaagcgtc ttacaaaaca cttctctatc atgcagccgg agagctggct 1560
gggagccctt ttgttttaga acacacatcc ttcagcagct gagaaatgaa cacgaatcca 1620
tcccaaccga gatgccatta acattcatct aaaaatgtta ggctctaaat ggacgaaaaa 1680
ttctctcgcc atcttaataa caaaataaac tacaaattcc tgacccaagg acactgtgtt 1740
ataagaggcg tgggctcccc tggtggctga ccaggtcagc tgccctggcc ttgcacccct 1800
ctgcatgcag cacagaaggg tgtgaccatg ccctcagcac cactcttgtc cccactgaac 1860
ggcaactgag actgggtacc tggagattct gaagtgcctt tgctgtggtt ttcaaaataa 1920
taaagatttg tattcaactc 1940

<210> 2
<211> 327
<212> PRT
<213> Homo sapiens

<220>
<221> Annexin A8
<222> (1)..(327)
<223> NP001621

<400> 2

Met Ala Trp Trp Lys Ala Trp Ile Glu Gln Glu Gly Val Thr Val Lys
1 5 10 15

Ser Ser Ser His Phe Asn Pro Asp Pro Asp Ala Glu Thr Leu Tyr Lys
20 25 30

Ala Met Lys Gly Ile Gly Thr Asn Glu Gln Ala Ile Ile Asp Val Leu
35 40 45

Thr Lys Arg Ser Asn Thr Gln Arg Gln Gln Ile Ala Lys Ser Phe Lys
50 55 60

Ala Gln Phe Gly Lys Asp Leu Thr Glu Thr Leu Lys Ser Glu Leu Ser
65 70 75 80

Gly Lys Phe Glu Arg Leu Ile Val Ala Leu Met Tyr Pro Pro Tyr Arg
85 90 95

```
Tyr Glu Ala Lys Glu Leu His Asp Ala Met Lys Gly Leu Gly Thr Lys
            100                 105                 110

Glu Gly Val Ile Ile Glu Ile Leu Ala Ser Arg Thr Lys Asn Gln Leu
        115                 120                 125

Arg Glu Ile Met Lys Ala Tyr Glu Glu Asp Tyr Gly Ser Ser Leu Glu
    130                 135                 140

Glu Asp Ile Gln Ala Asp Thr Ser Gly Tyr Leu Glu Arg Ile Leu Val
145                 150                 155                 160

Cys Leu Leu Gln Gly Ser Arg Asp Asp Val Ser Ser Phe Val Asp Pro
                165                 170                 175

Ala Leu Ala Leu Gln Asp Ala Gln Asp Leu Tyr Ala Ala Gly Glu Lys
            180                 185                 190

Ile Arg Gly Thr Asp Glu Met Lys Phe Ile Thr Ile Leu Cys Thr Arg
        195                 200                 205

Ser Ala Thr His Leu Leu Arg Val Phe Glu Glu Tyr Glu Lys Ile Ala
    210                 215                 220

Asn Lys Ser Ile Glu Asp Ser Ile Lys Ser Glu Thr His Gly Ser Leu
225                 230                 235                 240

Glu Glu Ala Met Leu Thr Val Val Lys Cys Thr Gln Asn Leu His Ser
                245                 250                 255

Tyr Phe Ala Glu Arg Leu Tyr Tyr Ala Met Lys Gly Ala Gly Thr Arg
            260                 265                 270

Asp Gly Thr Leu Ile Arg Asn Ile Val Ser Arg Ser Glu Ile Asp Leu
        275                 280                 285

Asn Leu Ile Lys Cys His Phe Lys Lys Met Tyr Gly Lys Thr Leu Ser
    290                 295                 300

Ser Met Ile Met Glu Asp Thr Ser Gly Asp Tyr Lys Asn Ala Leu Leu
305                 310                 315                 320

Ser Leu Val Gly Ser Asp Pro
                325

<210>  3
<211>  5417
<212>  DNA
<213>  Homo sapiens

<220>
```

<221> Complement component 4A
<222> (1)..(5417)
<223> NM007293

<400> 3
agaaggtagc agacagacag acggatctaa cctctcttgg atcctccagc catgaggctg 60
ctctgggggc tgatctgggc atccagcttc ttcaccttat ctctgcagaa gcccaggttg 120
ctcttgttct ctccttctgt ggttcatctg gggtccccc tatcggtggg ggtgcagctc 180
caggatgtgc cccgaggaca ggtagtgaaa ggatcagtgt tcctgagaaa cccatctcgt 240
aataatgtcc cctgctcccc aaaggtggac ttcaccctta gctcagaaag agacttcgca 300
ctcctcagtc tccaggtgcc cttgaaagat gcgaagagct gtggcctcca tcaactcctc 360
agaggccctg aggtccagct ggtggcccat tcgccatggc taaaggactc tctgtccaga 420
acgacaaaca tccagggtat caacctgctc ttctcctctc gccgggggca cctcttttg 480
cagacggacc agcccattta caaccctggc cagcgggttc ggtaccgggt ctttgctctg 540
gatcagaaga tgcgcccgag cactgacacc atcacagtca tggtggagaa ctctcacggc 600
ctccgcgtgc ggaagaagga ggtgtacatg ccctcgtcca tcttccagga tgactttgtg 660
atcccagaca tctcagagcc agggacctgg aagatctcag cccgattctc agatggcctg 720
gaatccaaca gcagcaccca gtttgaggtg aagaaatatg tccttcccaa ctttgaggtg 780
aagatcaccc ctggaaagcc ctacatcctg acggtgccag gccatcttga tgaaatgcag 840
ttagacatcc aggccaggta catctatggg aagccagtgc agggggtggc atatgtgcgc 900
tttgggctcc tagatgagga tggtaagaag actttctttc gggggctgga gagtcagacc 960
aagctggtga atggacagag ccacatttcc ctctcaaagg cagagttcca ggacgccctg 1020
gagaagctga atatgggcat tactgacctc caggggctgc gcctctacgt tgctgcagcc 1080
atcattgagt ctccaggtgg ggagatggag gaggcagagc tcacatcctg gtattttgtg 1140
tcatctccct tctccttgga tcttagcaag accaagcgac accttgtgcc tggggccccc 1200
ttcctgctgc aggccttggt ccgtgagatg tcaggctccc cagcttctgg cattcctgtc 1260
aaagtttctg ccacggtgtc ttctcctggg tctgttcctg aagcccagga cattcagcaa 1320
aacacagacg ggagcggcca agtcagcatt ccaataatta tccctcagac catctcagag 1380
ctgcagctct cagtatctgc aggctcccca catccagcga tagccaggct cactgtggca 1440
gccccacctt caggaggccc cgggtttctg tctattgagc ggccggattc tcgacctcct 1500
cgtgttgggg acactctgaa cctgaacttg cgagccgtgg gcagtggggc cacctttct 1560
cattactact acatgatcct atcccgaggg cagatcgtgt tcatgaatcg agagcccaag 1620
aggaccctga cctcggtctc ggtgtttgtg gaccatcacc tggcaccctc cttctacttt 1680
gtggccttct actaccatgg agaccaccca gtggccaact ccctgcgagt ggatgtccag 1740
gctggggcct gcgagggcaa gctggagctc agcgtggacg gtgccaagca gtaccggaac 1800
ggggagtccg tgaagctcca cttagaaacc gactccctag ccctggtggc gctgggagcc 1860
ttggacacag ctctgtatgc tgcaggcagc aagtcccaca agcccctcaa catgggcaag 1920

gtctttgaag ctatgaacag ctatgacctc ggctgtggtc ctgggggtgg ggacagtgcc 1980
cttcaggtgt tccaggcagc gggcctggcc ttttctgatg gagaccagtg gaccttatcc 2040
agaaagagac taagctgtcc caaggagaag acaacccgga aaaagagaaa cgtgaacttc 2100
caaaaggcga ttaatgagaa attgggtcag tatgcttccc cgacagccaa gcgctgctgc 2160
caggatgggg tgacacgtct gcccatgatg cgttcctgcg agcagcgggc agcccgcgtg 2220
cagcagccgg actgccggga gcccttcctg tcctgctgcc aatttgctga gagtctgcgc 2280
aagaagagca gggacaaggg ccaggcgggc ctccaacgag ccctggagat cctgcaggag 2340
gaggacctga ttgatgagga tgacattccc gtgcgcagct tcttcccaga gaactggctc 2400
tggagagtgg aaacagtgga ccgctttcaa atattgacac tgtggctccc cgactctctg 2460
accacgtggg agatccatgg cctgagcctg tccaaaacca aaggcctatg tgtggccacc 2520
ccagtccagc tccgggtgtt ccgcgagttc cacctgcacc tccgcctgcc catgtctgtc 2580
cgccgctttg agcagctgga gctgcggcct gtcctctata actacctgga taaaaacctg 2640
actgtgagcg tccacgtgtc cccagtggag ggctgtgcc tggctggggg cggagggctg 2700
gcccagcagg tgctggtgcc tgcgggctct gcccggcctg ttgccttctc tgtggtgccc 2760
acggcagccg ccgctgtgtc tctgaaggtg gtggctcgag ggtccttcga attccctgtg 2820
ggagatgcgg tgtccaaggt tctgcagatt gagaaggaag ggccatcca tagagaggag 2880
ctggtctatg aactcaaccc cttggaccac cgaggccgga ccttggaaat acctggcaac 2940
tctgatccca atatgatccc tgatggggac tttaacagct acgtcagggt tacagcctca 3000
gatccattgg acactttagg ctctgagggg gccttgtcac caggaggcgt ggcctccctc 3060
ttgaggcttc ctcgaggctg tggggagcaa accatgatct acttggctcc gacactggct 3120
gcttcccgct acctggacaa gacagagcag tggagcacac tgcctcccga gaccaaggac 3180
cacgccgtgg atctgatcca gaaaggctac atgcggatcc agcagtttcg gaaggcggat 3240
ggttcctatg cggcttggtt gtcacgggac agcagcacct ggctcacagc ctttgtgttg 3300
aaggtcctga gtttggccca ggagcaggta ggaggctcgc ctgagaaact gcaggagaca 3360
tctaactggc ttctgtccca gcagcaggct gacggctcgt tccaggaccc ctgtccagtg 3420
ttagacagga gcatgcaggg gggtttggtg ggcaatgatg agactgtggc actcacagcc 3480
tttgtgacca tcgcccttca tcatgggctg gccgtcttcc aggatgaggg tgcagagcca 3540
ttgaagcaga gagtggaagc ctccatctca aaggcaaact catttttggg ggagaaagca 3600
agtgctgggc tcctgggtgc ccacgcagct gccatcacgg cctatgccct gtcactgacc 3660
aaggcgcctg tggacctgct cggtgttgcc cacaacaacc tcatggcaat ggcccaggag 3720
actggagata acctgtactg gggctcagtc actggttctc agagcaatgc cgtgtcgccc 3780
accccggctc ctcgcaaccc atccgacccc atgccccagg ccccagccct gtggattgaa 3840
accacagcct acgccctgct gcacctcctg cttcacgagg gcaaagcaga gatggcagac 3900
caggcttcgg cctggctcac ccgtcagggc agcttccaag gggattccg cagtacccaa 3960

```
gacacggtga ttgccctgga tgccctgtct gcctactgga ttgcctccca caccactgag    4020
gagaggggtc tcaatgtgac tctcagctcc acaggccgga atgggttcaa gtcccacgcg    4080
ctgcagctga acaaccgcca gattcgcggc ctggaggagg agctgcagtt ttccttgggc    4140
agcaagatca atgtgaaggt gggaggaaac agcaaaggaa ccctgaaggt ccttcgtacc    4200
tacaatgtcc tggacatgaa gaacacgacc tgccaggacc tacagataga agtgacagtc    4260
aaaggccacg tcgagtacac gatggaagca aacgaggact atgagtacga tgagcttcca    4320
gccaaggatg acccagatgc ccctctgcag cccgtgacac ccctgcagct gtttgagggt    4380
cggaggaacc gccgcaggag ggaggcgccc aaggtggtgg aggagcagga gtccagggtg    4440
cactacaccg tgtgcatctg gcggaacggc aaggtggggc tgtctggcat ggccatcgcg    4500
gacgtcaccc tcctgagtgg attccacgcc ctgcgtgctg acctggagaa gctgacctcc    4560
ctctctgacc gttacgtgag tcactttgag accgaggggc cccacgtcct gctgtatttt    4620
gactcggtcc ccacctcccg ggagtgcgtg ggctttgagg ctgtgcagga agtgccggtg    4680
gggctggtgc agccggccag cgcaaccctg tacgactact acaaccccga gcgcagatgt    4740
tctgtgtttt acggggcacc aagtaagagc agactcttgg ccaccttgtg ttctgctgaa    4800
gtctgccagt gtgctgaggg gaagtgccct cgccagcgtc gcgccctgga gcggggtctg    4860
caggacgagg atggctacag gatgaagttt gcctgctact acccccgtgt ggagtacggc    4920
ttccaggtta aggttctccg agaagacagc agagctgctt tccgcctctt tgagaccaag    4980
atcacccaag tcctgcactt caccaaggat gtcaaggccg ctgctaatca gatgcgcaac    5040
ttcctggttc gagcctcctg ccgccttcgc ttggaacctg ggaaagaata tttgatcatg    5100
ggtctggatg gggccaccta tgacctcgag ggacaccccc agtacctgct ggactcgaat    5160
agctggatcg aggagatgcc ctctgaacgc ctgtgccgga gcacccgcca gcgggcagcc    5220
tgtgcccagc tcaacgactt cctccaggag tatggcactc aggggtgcca ggtgtgaggg    5280
ctgccctccc acctccgctg ggaggaacct gaacctggga accatgaagc tggaagcact    5340
gctgtgtccg ctttcatgaa cacagcctgg gaccagggca tattaaaggc ttttggcagc    5400
aaagtgtcag tgttggc                                                   5417
```

```
<210>  4
<211>  1741
<212>  PRT
<213>  Homo sapiens

<220>
<221>  Complement component 4A
<222>  (1)..(1741)
<223>  NP009224

<400>  4

Met Arg Leu Leu Trp Gly Leu Ile Trp Ala Ser Ser Phe Phe Thr Leu
1               5                   10                  15
```

```
Ser Leu Gln Lys Pro Arg Leu Leu Leu Phe Ser Pro Ser Val Val His
            20                  25                  30

Leu Gly Val Pro Leu Ser Val Gly Val Gln Leu Gln Asp Val Pro Arg
        35                  40                  45

Gly Gln Val Val Lys Gly Ser Val Phe Leu Arg Asn Pro Ser Arg Asn
    50                  55                  60

Asn Val Pro Cys Ser Pro Lys Val Asp Phe Thr Leu Ser Ser Glu Arg
65                  70                  75                  80

Asp Phe Ala Leu Leu Ser Leu Gln Val Pro Leu Lys Asp Ala Lys Ser
                85                  90                  95

Cys Gly Leu His Gln Leu Leu Arg Gly Pro Glu Val Gln Leu Val Ala
            100                 105                 110

His Ser Pro Trp Leu Lys Asp Ser Leu Ser Arg Thr Thr Asn Ile Gln
        115                 120                 125

Gly Ile Asn Leu Leu Phe Ser Ser Arg Arg Gly His Leu Phe Leu Gln
    130                 135                 140

Thr Asp Gln Pro Ile Tyr Asn Pro Gly Gln Arg Val Arg Tyr Arg Val
145                 150                 155                 160

Phe Ala Leu Asp Gln Lys Met Arg Pro Ser Thr Asp Thr Ile Thr Val
                165                 170                 175

Met Val Glu Asn Ser His Gly Leu Arg Val Arg Lys Lys Glu Val Tyr
            180                 185                 190

Met Pro Ser Ser Ile Phe Gln Asp Asp Phe Val Ile Pro Asp Ile Ser
        195                 200                 205

Glu Pro Gly Thr Trp Lys Ile Ser Ala Arg Phe Ser Asp Gly Leu Glu
    210                 215                 220

Ser Asn Ser Ser Thr Gln Phe Glu Val Lys Lys Tyr Val Leu Pro Asn
225                 230                 235                 240

Phe Glu Val Lys Ile Thr Pro Gly Lys Pro Tyr Ile Leu Thr Val Pro
                245                 250                 255

Gly His Leu Asp Glu Met Gln Leu Asp Ile Gln Ala Arg Tyr Ile Tyr
            260                 265                 270

Gly Lys Pro Val Gln Gly Val Ala Tyr Val Arg Phe Gly Leu Leu Asp
        275                 280                 285
```

Glu Asp Gly Lys Lys Thr Phe Phe Arg Gly Leu Glu Ser Gln Thr Lys
290 295 300

Leu Val Asn Gly Gln Ser His Ile Ser Leu Ser Lys Ala Glu Phe Gln
305 310 315 320

Asp Ala Leu Glu Lys Leu Asn Met Gly Ile Thr Asp Leu Gln Gly Leu
325 330 335

Arg Leu Tyr Val Ala Ala Ala Ile Ile Glu Ser Pro Gly Gly Glu Met
340 345 350

Glu Glu Ala Glu Leu Thr Ser Trp Tyr Phe Val Ser Ser Pro Phe Ser
355 360 365

Leu Asp Leu Ser Lys Thr Lys Arg His Leu Val Pro Gly Ala Pro Phe
370 375 380

Leu Leu Gln Ala Leu Val Arg Glu Met Ser Gly Ser Pro Ala Ser Gly
385 390 395 400

Ile Pro Val Lys Val Ser Ala Thr Val Ser Ser Pro Gly Ser Val Pro
405 410 415

Glu Ala Gln Asp Ile Gln Gln Asn Thr Asp Gly Ser Gly Gln Val Ser
420 425 430

Ile Pro Ile Ile Ile Pro Gln Thr Ile Ser Glu Leu Gln Leu Ser Val
435 440 445

Ser Ala Gly Ser Pro His Pro Ala Ile Ala Arg Leu Thr Val Ala Ala
450 455 460

Pro Pro Ser Gly Gly Pro Gly Phe Leu Ser Ile Glu Arg Pro Asp Ser
465 470 475 480

Arg Pro Pro Arg Val Gly Asp Thr Leu Asn Leu Asn Leu Arg Ala Val
485 490 495

Gly Ser Gly Ala Thr Phe Ser His Tyr Tyr Tyr Met Ile Leu Ser Arg
500 505 510

Gly Gln Ile Val Phe Met Asn Arg Glu Pro Lys Arg Thr Leu Thr Ser
515 520 525

Val Ser Val Phe Val Asp His His Leu Ala Pro Ser Phe Tyr Phe Val
530 535 540

Ala Phe Tyr Tyr His Gly Asp His Pro Val Ala Asn Ser Leu Arg Val
545 550 555 560

```
Asp Val Gln Ala Gly Ala Cys Glu Gly Lys Leu Glu Leu Ser Val Asp
                565                 570                 575

Gly Ala Lys Gln Tyr Arg Asn Gly Glu Ser Val Lys Leu His Leu Glu
            580                 585                 590

Thr Asp Ser Leu Ala Leu Val Ala Leu Gly Ala Leu Asp Thr Ala Leu
        595                 600                 605

Tyr Ala Ala Gly Ser Lys Ser His Lys Pro Leu Asn Met Gly Lys Val
    610                 615                 620

Phe Glu Ala Met Asn Ser Tyr Asp Leu Gly Cys Gly Pro Gly Gly Gly
625                 630                 635                 640

Asp Ser Ala Leu Gln Val Phe Gln Ala Ala Gly Leu Ala Phe Ser Asp
                645                 650                 655

Gly Asp Gln Trp Thr Leu Ser Arg Lys Arg Leu Ser Cys Pro Lys Glu
            660                 665                 670

Lys Thr Thr Arg Lys Lys Arg Asn Val Asn Phe Gln Lys Ala Ile Asn
        675                 680                 685

Glu Lys Leu Gly Gln Tyr Ala Ser Pro Thr Ala Lys Arg Cys Cys Gln
    690                 695                 700

Asp Gly Val Thr Arg Leu Pro Met Met Arg Ser Cys Glu Gln Arg Ala
705                 710                 715                 720

Ala Arg Val Gln Gln Pro Asp Cys Arg Glu Pro Phe Leu Ser Cys Cys
                725                 730                 735

Gln Phe Ala Glu Ser Leu Arg Lys Lys Ser Arg Asp Lys Gly Gln Ala
            740                 745                 750

Gly Leu Gln Arg Ala Leu Glu Ile Leu Gln Glu Glu Asp Leu Ile Asp
        755                 760                 765

Glu Asp Asp Ile Pro Val Arg Ser Phe Phe Pro Glu Asn Trp Leu Trp
    770                 775                 780

Arg Val Glu Thr Val Asp Arg Phe Gln Ile Leu Thr Leu Trp Leu Pro
785                 790                 795                 800

Asp Ser Leu Thr Thr Trp Glu Ile His Gly Leu Ser Leu Ser Lys Thr
                805                 810                 815

Lys Gly Leu Cys Val Ala Thr Pro Val Gln Leu Arg Val Phe Arg Glu
            820                 825                 830
```

```
Phe His Leu His Leu Arg Leu Pro Met Ser Val Arg Arg Phe Glu Gln
        835                 840                 845

Leu Glu Leu Arg Pro Val Leu Tyr Asn Tyr Leu Asp Lys Asn Leu Thr
    850                 855                 860

Val Ser Val His Val Ser Pro Val Glu Gly Leu Cys Leu Ala Gly Gly
865                 870                 875                 880

Gly Gly Leu Ala Gln Gln Val Leu Val Pro Ala Gly Ser Ala Arg Pro
                885                 890                 895

Val Ala Phe Ser Val Val Pro Thr Ala Ala Ala Ala Val Ser Leu Lys
            900                 905                 910

Val Val Ala Arg Gly Ser Phe Glu Phe Pro Val Gly Asp Ala Val Ser
        915                 920                 925

Lys Val Leu Gln Ile Glu Lys Glu Gly Ala Ile His Arg Glu Glu Leu
    930                 935                 940

Val Tyr Glu Leu Asn Pro Leu Asp His Arg Gly Arg Thr Leu Glu Ile
945                 950                 955                 960

Pro Gly Asn Ser Asp Pro Asn Met Ile Pro Asp Gly Asp Phe Asn Ser
                965                 970                 975

Tyr Val Arg Val Thr Ala Ser Asp Pro Leu Asp Thr Leu Gly Ser Glu
            980                 985                 990

Gly Ala Leu Ser Pro Gly Gly Val  Ala Ser Leu Leu Arg  Leu Pro Arg
        995                 1000                 1005

Gly Cys  Gly Glu Gln Thr Met  Ile Tyr Leu Ala Pro  Thr Leu Ala
    1010                 1015                 1020

Ala Ser  Arg Tyr Leu Asp Lys  Thr Glu Gln Trp Ser  Thr Leu Pro
    1025                 1030                 1035

Pro Glu  Thr Lys Asp His Ala  Val Asp Leu Ile Gln  Lys Gly Tyr
    1040                 1045                 1050

Met Arg  Ile Gln Gln Phe Arg  Lys Ala Asp Gly Ser  Tyr Ala Ala
    1055                 1060                 1065

Trp Leu  Ser Arg Asp Ser Ser  Thr Trp Leu Thr Ala  Phe Val Leu
    1070                 1075                 1080

Lys Val  Leu Ser Leu Ala Gln  Glu Gln Val Gly Gly  Ser Pro Glu
    1085                 1090                 1095
```

```
Lys Leu  Gln Glu Thr Ser Asn  Trp Leu Leu Ser Gln  Gln Gln Ala
    1100                 1105                 1110

Asp Gly  Ser Phe Gln Asp Pro  Cys Pro Val Leu Asp  Arg Ser Met
    1115                 1120                 1125

Gln Gly  Gly Leu Val Gly Asn  Asp Glu Thr Val Ala  Leu Thr Ala
    1130                 1135                 1140

Phe Val  Thr Ile Ala Leu His  His Gly Leu Ala Val  Phe Gln Asp
    1145                 1150                 1155

Glu Gly  Ala Glu Pro Leu Lys  Gln Arg Val Glu Ala  Ser Ile Ser
    1160                 1165                 1170

Lys Ala  Asn Ser Phe Leu Gly  Glu Lys Ala Ser Ala  Gly Leu Leu
    1175                 1180                 1185

Gly Ala  His Ala Ala Ala Ile  Thr Ala Tyr Ala Leu  Ser Leu Thr
    1190                 1195                 1200

Lys Ala  Pro Val Asp Leu Leu  Gly Val Ala His Asn  Asn Leu Met
    1205                 1210                 1215

Ala Met  Ala Gln Glu Thr Gly  Asp Asn Leu Tyr Trp  Gly Ser Val
    1220                 1225                 1230

Thr Gly  Ser Gln Ser Asn Ala  Val Ser Pro Thr Pro  Ala Pro Arg
    1235                 1240                 1245

Asn Pro  Ser Asp Pro Met Pro  Gln Ala Pro Ala Leu  Trp Ile Glu
    1250                 1255                 1260

Thr Thr  Ala Tyr Ala Leu Leu  His Leu Leu Leu His  Glu Gly Lys
    1265                 1270                 1275

Ala Glu  Met Ala Asp Gln Ala  Ser Ala Trp Leu Thr  Arg Gln Gly
    1280                 1285                 1290

Ser Phe  Gln Gly Gly Phe Arg  Ser Thr Gln Asp Thr  Val Ile Ala
    1295                 1300                 1305

Leu Asp  Ala Leu Ser Ala Tyr  Trp Ile Ala Ser His  Thr Thr Glu
    1310                 1315                 1320

Glu Arg  Gly Leu Asn Val Thr  Leu Ser Ser Thr Gly  Arg Asn Gly
    1325                 1330                 1335

Phe Lys  Ser His Ala Leu Gln  Leu Asn Asn Arg Gln  Ile Arg Gly
    1340                 1345                 1350
```

```
Leu Glu  Glu Glu Leu Gln Phe  Ser Leu Gly Ser Lys  Ile Asn Val
    1355                 1360                 1365

Lys Val  Gly Gly Asn Ser Lys  Gly Thr Leu Lys Val  Leu Arg Thr
    1370                 1375                 1380

Tyr Asn  Val Leu Asp Met Lys  Asn Thr Thr Cys Gln  Asp Leu Gln
    1385                 1390                 1395

Ile Glu  Val Thr Val Lys Gly  His Val Glu Tyr Thr  Met Glu Ala
    1400                 1405                 1410

Asn Glu  Asp Tyr Glu Tyr Asp  Glu Leu Pro Ala Lys  Asp Asp Pro
    1415                 1420                 1425

Asp Ala  Pro Leu Gln Pro Val  Thr Pro Leu Gln Leu  Phe Glu Gly
    1430                 1435                 1440

Arg Arg  Asn Arg Arg Arg Arg  Glu Ala Pro Lys Val  Val Glu Glu
    1445                 1450                 1455

Gln Glu  Ser Arg Val His Tyr  Thr Val Cys Ile Trp  Arg Asn Gly
    1460                 1465                 1470

Lys Val  Gly Leu Ser Gly Met  Ala Ile Ala Asp Val  Thr Leu Leu
    1475                 1480                 1485

Ser Gly  Phe His Ala Leu Arg  Ala Asp Leu Glu Lys  Leu Thr Ser
    1490                 1495                 1500

Leu Ser  Asp Arg Tyr Val Ser  His Phe Glu Thr Glu  Gly Pro His
    1505                 1510                 1515

Val Leu  Leu Tyr Phe Asp Ser  Val Pro Thr Ser Arg  Glu Cys Val
    1520                 1525                 1530

Gly Phe  Glu Ala Val Gln Glu  Val Pro Val Gly Leu  Val Gln Pro
    1535                 1540                 1545

Ala Ser  Ala Thr Leu Tyr Asp  Tyr Tyr Asn Pro Glu  Arg Arg Cys
    1550                 1555                 1560

Ser Val  Phe Tyr Gly Ala Pro  Ser Lys Ser Arg Leu  Leu Ala Thr
    1565                 1570                 1575

Leu Cys  Ser Ala Glu Val Cys  Gln Cys Ala Glu Gly  Lys Cys Pro
    1580                 1585                 1590

Arg Gln  Arg Arg Ala Leu Glu  Arg Gly Leu Gln Asp  Glu Asp Gly
    1595                 1600                 1605
```

Tyr Arg Met Lys Phe Ala Cys Tyr Tyr Pro Arg Val Glu Tyr Gly
1610 1615 1620

Phe Gln Val Lys Val Leu Arg Glu Asp Ser Arg Ala Ala Phe Arg
1625 1630 1635

Leu Phe Glu Thr Lys Ile Thr Gln Val Leu His Phe Thr Lys Asp
1640 1645 1650

Val Lys Ala Ala Ala Asn Gln Met Arg Asn Phe Leu Val Arg Ala
1655 1660 1665

Ser Cys Arg Leu Arg Leu Glu Pro Gly Lys Glu Tyr Leu Ile Met
1670 1675 1680

Gly Leu Asp Gly Ala Thr Tyr Asp Leu Glu Gly His Pro Gln Tyr
1685 1690 1695

Leu Leu Asp Ser Asn Ser Trp Ile Glu Glu Met Pro Ser Glu Arg
1700 1705 1710

Leu Cys Arg Ser Thr Arg Gln Arg Ala Ala Cys Ala Gln Leu Asn
1715 1720 1725

Asp Phe Leu Gln Glu Tyr Gly Thr Gln Gly Cys Gln Val
1730 1735 1740

<210> 5
<211> 3890
<212> DNA
<213> Homo sapiens

<220>
<221> Complement component 7
<222> (1)..(3890)
<223> J03507

<400> 5
atgaaggtga taagcttatt cattttggtg ggatttatag gagagttcca aagtttttca 60

agtgcctcct ctccagtcaa ctgccagtgg gacttctatg ccccttggtc agaatgcaat 120

ggctgtacca agactcagac tcgcaggcgg tcagttgctg tgtatgggca gtatggaggc 180

cagccttgtg ttggaaatgc ttttgaaaca cagtcctgtg aacctacaag aggatgtcca 240

acagaggagg gatgtggaga gcgtttcagg tgcttttcag gtcagtgcat cagcaaatca 300

ttggtttgca atggggattc tgactgtgat gaagacagtg ctgatgaaga cagatgtgag 360

gactcagaaa ggagaccttc ctgtgatatc gataaacctc ctcctaacat agaacttact 420

ggaaatggtt acaatgaact cactggccag tttaggaaca gagtcatcaa taccaaaagt 480

tttggtggtc aatgtagaaa ggtgtttagt ggggatggaa aagatttcta caggctgagt 540

ggaaatgtcc tgtcctatac attccaggtg aaaataaata atgattttaa ttatgaattt 600

```
tacaatagta cttggtctta tgtaaaacat acgtcgacag aacacacatc atctagtcgg       660
aagcgctcct tttttagatc ttcatcatct tcttcacgca gttatacttc acataccaat       720
gaaatccata aaggaaagag ttaccaactg ctggttgttg agaacactgt tgaagtggct       780
cagttcatta ataacaatcc agaattttta caacttgctg agccattctg gaaggagctt       840
tcccacctcc cctctctgta tgactacagt gcctaccgaa gattaatcga ccagtacggg       900
acacattatc tgcaatctgg gtcgttagga ggagaataca gagttctatt ttatgtggac       960
tcagaaaaat taaaacaaaa tgattttaat tcagtcgaag aaaagaaatg taaatcctca      1020
ggttggcatt ttgtcgttaa attttcaagt catggatgca aggaactgga aaacgcttta      1080
aaagctgctt caggaaccca gaacaatgta ttgcgaggag aaccgttcat cagaggggga      1140
ggtgcaggct tcatatctgg ccttagttac ctagagctgg acaatcctgc tggaaacaaa      1200
aggcgatatt ctgcctgggc agaatctgtg actaatcttc ctcaagtcat aaaacaaaag      1260
ctgacacctt tatatgagct ggtaaaggaa gtaccttgtg cctctgtgaa aaaactatac      1320
ctgaaatggg ctcttgaaga gtatctggat gaatttgacc cctgtcattg ccggccttgt      1380
caaaatggtg gtttggctac tgttgagggg acccattgtc tgtgccattg caaaccgtac      1440
acatttggtg cggcgtgtga gcaaggagtc ctcgtaggga atcaagcagg aggggttgat      1500
ggaggttgga gttgctggtc ctcttggagc ccctgtgtcc aagggaagaa aacaagaagc      1560
cgtgaatgca ataacccacc tcccagtggg ggtgggagat cctgcgttgg agaaacgaca      1620
gaaagcacac aatgcgaaga tgaggagctg gagcacttga ggttgcttga accacattgc      1680
tttcctttgt ctttggttcc aacagaattc tgtccatcac ctcctgcctt gaaagatgga      1740
tttgttcaag atgaaggtcc aatgtttcct gtggggaaaa atgtagtgta cacttgcaat      1800
gaaggatact ctcttattgg aaacccagtg gccagatgtg gagaagattt acggtggctt      1860
gttggggaaa tgcattgtca gaaaattgcc tgtgttctac ctgtactgat ggatggcata      1920
cagagtcacc cccaaaaacc tttctacaca gttggtgaga aggtgactgt ttcctgttca      1980
ggtggcatgt ccttagaagg tccttcagca tttctctgtg gctccagcct taagtggagt      2040
cctgagatga agaatgcccg ctgtgtacaa aaagaaaatc cgttaacaca ggcagtgcct      2100
aaatgtcagc gctgggagaa actgcagaat tcaagatgtg tttgtaaaat gccctacgaa      2160
tgtggacctt ccttggatgt atgtgctcaa gatgagagaa gcaaaaggat actgcctctg      2220
acagtttgca agatgcatgt tctccactgt cagggtagaa attacaccct tactggtagg      2280
gacagctgta ctctgcctgc ctcagctgag aaagcttgtg gtgcctgccc actgtgggga      2340
aaatgtgatg ctgagagcag caaatgtgtc tgccgagaag catcggagtg cgaggaagaa      2400
gggtttagca tttgtgtgga agtgaacggc aaggagcaga cgatgtctga gtgtgaggcg      2460
ggcgctctga gatgcagagg gcagagcatc tctgtcacca gcataaggcc ttgtgctgcg      2520
gaaacccagt aggctcctgg aggccatggt cagcttgctt ggaatccagc aggcagctgg      2580
ggctgagtga aaacatctgc acaactgggc actggacagc ttttccttct tctccagtgt      2640
```

ctaccttcct cctcaactcc cagccatctg tataaacaca atcctttgtt ctcccaaatc 2700
tgaatcgaat tactcttttg cctccttttt aatgtcagta aggatatgag cctttgcaca 2760
ggctggctgc gtgttcttga aataggtgtt accttctctg ggccttggtt ttttaaaatc 2820
tgtaaaatta gaggattgca ctagagaaac ttgaatgctc cattcaggcc tatcatttta 2880
ttaagtatga ttgacacagc ccatgggcca gaacacactc tacaaaatga ctaggataac 2940
agaaagaacg tgatctcctg attagagagg gtggttttcc tcaatggaac caaatataaa 3000
gaggacttga acaaaaatga cagatacaaa ctatttctat cctgagtagt aatctcacac 3060
ttcatcctat agagtcaacc accacagata ggaattcctt attctttttt taattttttt 3120
aagacagagt ctcactttgt tgcccaggct ggagcgcagt ggggtgatct catctccctg 3180
caacctccgc ctcctgggtt gaagcgattc ttgtgcctca gcttcccaag cagctgggat 3240
tacaggtgcc cgccaccacg cccagctaat ttttgcattt ttagtagaga tgggtttcac 3300
catgttggcc atgctcgtct ccaactcctg acctcaggta atccgtctgc cttggcctcc 3360
caaatgctgg gattacagac atgaaccacc acgcctggct ggaatactta ctcttgtcgg 3420
gagattgaac cactaaaatg ttagagcaga attcattatg ctgtggtcac aggggtgtct 3480
tgtctgagaa caaatacaat tcagtcttct ctttggggtt ttagtatgtg tcaaacatag 3540
gactggaagt ttgcccctgt tctttttctt tttgaaagaa catcagttca tgcctgaggc 3600
atgagtgact gtgcatttga gatagttttc cctattctgt ggatacagtc ccagagtttt 3660
cagggagtac acaggtagat tagtttgaag cattgacctt ttatttattc cttatttctc 3720
tttcatcaaa acaaaacagc agctgtggga ggagaaatga gagggcttaa atgaaattta 3780
aaataagcta tattatacaa atactatctc tgtattgttc tgaccctggt aaatatattt 3840
caaaacttca gatgacaagg attagaacac tcattaagat gctattcttc 3890

<210> 6
<211> 843
<212> PRT
<213> Homo sapiens

<220>
<221> Complement component 7
<222> (1)..(843)
<223> NP000578

<400> 6

Met Lys Val Ile Ser Leu Phe Ile Leu Val Gly Phe Ile Gly Glu Phe
1               5                   10                  15

Gln Ser Phe Ser Ser Ala Ser Ser Pro Val Asn Cys Gln Trp Asp Phe
            20                  25                  30

Tyr Ala Pro Trp Ser Glu Cys Asn Gly Cys Thr Lys Thr Gln Thr Arg
        35                  40                  45

```
Arg Arg Ser Val Ala Val Tyr Gly Gln Tyr Gly Gly Gln Pro Cys Val
    50                  55                  60

Gly Asn Ala Phe Glu Thr Gln Ser Cys Glu Pro Thr Arg Gly Cys Pro
65                  70                  75                  80

Thr Glu Glu Gly Cys Gly Glu Arg Phe Arg Cys Phe Ser Gly Gln Cys
                85                  90                  95

Ile Ser Lys Ser Leu Val Cys Asn Gly Asp Ser Asp Cys Asp Glu Asp
            100                 105                 110

Ser Ala Asp Glu Asp Arg Cys Glu Asp Ser Glu Arg Arg Pro Ser Cys
        115                 120                 125

Asp Ile Asp Lys Pro Pro Pro Asn Ile Glu Leu Thr Gly Asn Gly Tyr
    130                 135                 140

Asn Glu Leu Thr Gly Gln Phe Arg Asn Arg Val Ile Asn Thr Lys Ser
145                 150                 155                 160

Phe Gly Gly Gln Cys Arg Lys Val Phe Ser Gly Asp Gly Lys Asp Phe
                165                 170                 175

Tyr Arg Leu Ser Gly Asn Val Leu Ser Tyr Thr Phe Gln Val Lys Ile
            180                 185                 190

Asn Asn Asp Phe Asn Tyr Glu Phe Tyr Asn Ser Thr Trp Ser Tyr Val
        195                 200                 205

Lys His Thr Ser Thr Glu His Thr Ser Ser Ser Arg Lys Arg Ser Phe
    210                 215                 220

Phe Arg Ser Ser Ser Ser Ser Ser Arg Ser Tyr Thr Ser His Thr Asn
225                 230                 235                 240

Glu Ile His Lys Gly Lys Ser Tyr Gln Leu Leu Val Val Glu Asn Thr
                245                 250                 255

Val Glu Val Ala Gln Phe Ile Asn Asn Asn Pro Glu Phe Leu Gln Leu
            260                 265                 270

Ala Glu Pro Phe Trp Lys Glu Leu Ser His Leu Pro Ser Leu Tyr Asp
        275                 280                 285

Tyr Ser Ala Tyr Arg Arg Leu Ile Asp Gln Tyr Gly Thr His Tyr Leu
    290                 295                 300

Gln Ser Gly Ser Leu Gly Gly Glu Tyr Arg Val Leu Phe Tyr Val Asp
305                 310                 315                 320
```

```
Ser Glu Lys Leu Lys Gln Asn Asp Phe Asn Ser Val Glu Glu Lys Lys
                325                 330                 335

Cys Lys Ser Ser Gly Trp His Phe Val Val Lys Phe Ser Ser His Gly
            340                 345                 350

Cys Lys Glu Leu Glu Asn Ala Leu Lys Ala Ala Ser Gly Thr Gln Asn
        355                 360                 365

Asn Val Leu Arg Gly Glu Pro Phe Ile Arg Gly Gly Gly Ala Gly Phe
    370                 375                 380

Ile Ser Gly Leu Ser Tyr Leu Glu Leu Asp Asn Pro Ala Gly Asn Lys
385                 390                 395                 400

Arg Arg Tyr Ser Ala Trp Ala Glu Ser Val Thr Asn Leu Pro Gln Val
                405                 410                 415

Ile Lys Gln Lys Leu Thr Pro Leu Tyr Glu Leu Val Lys Glu Val Pro
            420                 425                 430

Cys Ala Ser Val Lys Lys Leu Tyr Leu Lys Trp Ala Leu Glu Glu Tyr
        435                 440                 445

Leu Asp Glu Phe Asp Pro Cys His Cys Arg Pro Cys Gln Asn Gly Gly
    450                 455                 460

Leu Ala Thr Val Glu Gly Thr His Cys Leu Cys His Cys Lys Pro Tyr
465                 470                 475                 480

Thr Phe Gly Ala Ala Cys Glu Gln Gly Val Leu Val Gly Asn Gln Ala
                485                 490                 495

Gly Gly Val Asp Gly Gly Trp Ser Cys Trp Ser Ser Trp Ser Pro Cys
            500                 505                 510

Val Gln Gly Lys Lys Thr Arg Ser Arg Glu Cys Asn Asn Pro Pro Pro
        515                 520                 525

Ser Gly Gly Gly Arg Ser Cys Val Gly Glu Thr Thr Glu Ser Thr Gln
    530                 535                 540

Cys Glu Asp Glu Glu Leu Glu His Leu Arg Leu Leu Glu Pro His Cys
545                 550                 555                 560

Phe Pro Leu Ser Leu Val Pro Thr Glu Phe Cys Pro Ser Pro Pro Ala
                565                 570                 575

Leu Lys Asp Gly Phe Val Gln Asp Glu Gly Thr Met Phe Pro Val Gly
            580                 585                 590
```

Lys Asn Val Val Tyr Thr Cys Asn Glu Gly Tyr Ser Leu Ile Gly Asn
595 600 605

Pro Val Ala Arg Cys Gly Glu Asp Leu Arg Trp Leu Val Gly Glu Met
610 615 620

His Cys Gln Lys Ile Ala Cys Val Leu Pro Val Leu Met Asp Gly Ile
625 630 635 640

Gln Ser His Pro Gln Lys Pro Phe Tyr Thr Val Gly Glu Lys Val Thr
645 650 655

Val Ser Cys Ser Gly Gly Met Ser Leu Glu Gly Pro Ser Ala Phe Leu
660 665 670

Cys Gly Ser Ser Leu Lys Trp Ser Pro Glu Met Lys Asn Ala Arg Cys
675 680 685

Val Gln Lys Glu Asn Pro Leu Thr Gln Ala Val Pro Lys Cys Gln Arg
690 695 700

Trp Glu Lys Leu Gln Asn Ser Arg Cys Val Cys Lys Met Pro Tyr Glu
705 710 715 720

Cys Gly Pro Ser Leu Asp Val Cys Ala Gln Asp Glu Arg Ser Lys Arg
725 730 735

Ile Leu Pro Leu Thr Val Cys Lys Met His Val Leu His Cys Gln Gly
740 745 750

Arg Asn Tyr Thr Leu Thr Gly Arg Asp Ser Cys Thr Leu Pro Ala Ser
755 760 765

Ala Glu Lys Ala Cys Gly Ala Cys Pro Leu Trp Gly Lys Cys Asp Ala
770 775 780

Glu Ser Ser Lys Cys Val Cys Arg Glu Ala Ser Glu Cys Glu Glu Glu
785 790 795 800

Gly Phe Ser Ile Cys Val Glu Val Asn Gly Lys Glu Gln Thr Met Ser
805 810 815

Glu Cys Glu Ala Gly Ala Leu Arg Cys Arg Gly Gln Ser Ile Ser Val
820 825 830

Thr Ser Ile Arg Pro Cys Ala Ala Glu Thr Gln
835 840

<210> 7
<211> 1420
<212> DNA

<213> Homo sapiens

<220>
<221> Fibroblast growth factor 9
<222> (1)..(1420)
<223> D14838

<400> 7
tgaaacagca gattactttt atttatgcat ttaatggatt gaagaaaaga accttttttt 60

ttctctctct ctctgcaact gcagtaaggg aggggagttg gatatacctc gcctaatatc 120

tcctgggttg acaccatcat tattgtttat tcttgtgctc caaaagccga gtcctctgat 180

ggctccctta ggtgaagttg ggaactattt cggtgtgcag gatgcggtac cgtttgggaa 240

tgtgcccgtg ttgccggtgg acagcccggt tttgttaagt gaccacctgg gtcagtccga 300

agcagggggg ctccccaggg gacccgcagt cacggacttg gatcatttaa aggggattct 360

caggcggagg cagctatact gcaggactgg atttcactta gaaatcttcc ccaatggtac 420

tatccaggga accaggaaag accacagccg atttggcatt ctggaattta tcagtatagc 480

agtgggcctg gtcagcattc gaggcgtgga cagtggactc tacctcggga tgaatgagaa 540

gggggagctg tatggatcag aaaaactaac ccaagagtgt gtattcagag aacagttcga 600

agaaaactgg tataatacgt actcgtcaaa cctatataag cacgtggaca ctggaaggcg 660

atactatgtt gcattaaata aagatgggac cccgagagaa gggactagga ctaaacggca 720

ccagaaattc acacattttt tacctagacc agtggacccc gacaaagtac ctgaactgta 780

taaggatatt ctaagccaaa gttgacaaag acaatttctt cacttgagcc cttaaaaaag 840

taaccactat aaaggtttca cgcggtgggt tcttattgat tcgctgtgtc atcacatcag 900

ctccactgtt gccaaacttt gtcgcatgca taatgtatga tggaggcttg gatgggaata 960

tgctgatttt gttctgcact taaaggcttc tcctcctgga gggctgccta gggccacttg 1020

cttgatttat catgagagaa gaggagagag agagagactg agcgctagga gtgtgtgtat 1080

gtgtgtgtgt gtgtgtgtgt gtgtgtgtgt atgtgtgtag cgggagatgt gggcggagcg 1140

agagcaaaag gactgcggcc tgatgcatgc tggaaaaaag acacgctttt catttctgat 1200

cagttgtact tcatcctata tcagcacagc tgccatactt cgacttatca ggattctggc 1260

tggtggcctg cgcgagggtg cagtcttact taaaagactt tcagttaatt ctcactggta 1320

tcatcgcagt gaacttaaag caaagacctc ttagtaaaaa ataaaaaaaa ataaaaaata 1380

aaaataaaaa aagttaaatt tatttataga aattccaaaa 1420

<210> 8
<211> 208
<212> PRT
<213> Homo sapiens

<220>
<221> Firbroblast growth factor 9
<222> (1)..(208)
<223> NP002001

<400> 8

Met Ala Pro Leu Gly Glu Val Gly Asn Tyr Phe Gly Val Gln Asp Ala
1               5                   10                  15

Val Pro Phe Gly Asn Val Pro Val Leu Pro Val Asp Ser Pro Val Leu
            20                  25                  30

Leu Ser Asp His Leu Gly Gln Ser Glu Ala Gly Gly Leu Pro Arg Gly
        35                  40                  45

Pro Ala Val Thr Asp Leu Asp His Leu Lys Gly Ile Leu Arg Arg Arg
    50                  55                  60

Gln Leu Tyr Cys Arg Thr Gly Phe His Leu Glu Ile Phe Pro Asn Gly
65                  70                  75                  80

Thr Ile Gln Gly Thr Arg Lys Asp His Ser Arg Phe Gly Ile Leu Glu
                85                  90                  95

Phe Ile Ser Ile Ala Val Gly Leu Val Ser Ile Arg Gly Val Asp Ser
            100                 105                 110

Gly Leu Tyr Leu Gly Met Asn Glu Lys Gly Glu Leu Tyr Gly Ser Glu
        115                 120                 125

Lys Leu Thr Gln Glu Cys Val Phe Arg Glu Gln Phe Glu Glu Asn Trp
    130                 135                 140

Tyr Asn Thr Tyr Ser Ser Asn Leu Tyr Lys His Val Asp Thr Gly Arg
145                 150                 155                 160

Arg Tyr Tyr Val Ala Leu Asn Lys Asp Gly Thr Pro Arg Glu Gly Thr
                165                 170                 175

Arg Thr Lys Arg His Gln Lys Phe Thr His Phe Leu Pro Arg Pro Val
            180                 185                 190

Asp Pro Asp Lys Val Pro Glu Leu Tyr Lys Asp Ile Leu Ser Gln Ser
        195                 200                 205

<210> 9
<211> 4049
<212> DNA
<213> Homo sapiens

<220>
<221> Gremlin
<222> (1)..(4049)
<223> AF110137

<400> 9
gcggccgcac tcagcgccac gcgtcgaaag cgcaggcccc gaggacccgc cgcactgaca 60

```
gtatgagccg cacagcctac acggtgggag ccctgcttct cctcttgggg accctgctgc    120
cggctgctga agggaaaaag aaagggtccc aaggtgccat ccccccgcca gacaaggccc    180
agcacaatga ctcagagcag actcagtcgc cccagcagcc tggctccagg aaccgggggc    240
ggggccaagg gcggggcact gccatgcccg gggaggaggt gctggagtcc agccaagagg    300
ccctgcatgt gacggagcgc aaatacctga agcgagactg gtgcaaaacc cagccgctta    360
agcagaccat ccacgaggaa ggctgcaaca gtcgcaccat catcaaccgc ttctgttacg    420
gccagtgcaa ctctttctac atccccaggc acatccggaa ggaggaaggt tcctttcagt    480
cctgctcctt ctgcaagccc aagaaattca ctaccatgat ggtcacactc aactgccctg    540
aactacagcc acctaccaag aagaagagag tcacacgtgt gaagcagtgt cgttgcatat    600
ccatcgattt ggattaagcc aaatccaggt gcacccagca tgtcctagga atgcagcccc    660
aggaagtccc agacctaaaa caaccagatt cttacttggc ttaaacctag aggccagaag    720
aacccccagc tgcctcctgg caggagcctg cttgtgcgta gttcgtgtgc atgagtgtgg    780
atgggtgcct gtgggtgttt ttagacacca gagaaaacac agtctctgct agagagcact    840
ccctattttg taaacatatc tgctttaatg gggatgtacc agaaacccac ctcaccccgg    900
ctcacatcta aggggcgggg gccgtggtct ggttctgact ttgtgttttt gtgccctcct    960
ggggaccaga atctcctttc ggaatgaatg ttcatggaag aggctcctct gagggcaaga   1020
gacctgtttt agtgctgcat tcgacatgga aaagtccttt taacctgtgc ttgcatcctc   1080
ctttcctcct cctcctcaca atccatctct tcttaagttg atagtgacta tgtcagtcta   1140
atctcttgtt tgccaaggtt cctaaattaa ttcacttaac catgatgcaa atgtttttca   1200
ttttgtgaag accctccaga ctctgggaga ggctggtgtg ggcaaggaca agcaggatag   1260
tggagtgaga aagggagggt ggagggtgag gccaaatcag gtccagcaaa agtcagtagg   1320
gacattgcag aagcttgaaa ggccaatacc agaacacagg ctgatgcttc tgagaaagtc   1380
ttttcctagt atttaacaga acccaagtga acagaggaga aatgagattg ccagaaagtg   1440
attaactttg gccgttgcaa tctgctcaaa cctaacacca aactgaaaac ataaatactg   1500
accactccta tgttcggacc caagcaagtt agctaaacca aaccaactcc tctgctttgt   1560
ccctcaggtg gaaaagagag gtagtttaga actctctgca taggggtggg aattaatcaa   1620
aaacckcaga ggctgaaatt cctaatacct ttcctttatc gtggttatag tcagctcatt   1680
tccattccac tatttcccat aatgcttctg agagccacta acttgattga taaagatcct   1740
gcctctgctg agtgtacctg acagtaagtc taaagatgar agagtttagg gactactctg   1800
ttttagcaag aratattktg gggtctttt tgttttaact attgtcagga gattgggcta    1860
ragagaagac gacgagagta aggaaataaa gggrattgcc tctggctaga gagtaagtta   1920
ggtgttaata cctggtagaa atgtaaggga tatgacctcc ctttctttat gtgctcactg   1980
aggatctgag gggaccctgt taggagagca tagcatcatg atgtattagc tgttcatctg   2040
ctactggttg gatggacata actattgtaa ctattcagta tttactggta ggcactgtcc   2100
```

```
tctgattaaa cttggcctac tggcaatggc tacttaggat tgatctaagg gccaaagtgc     2160
agggtgggtg aactttattg tactttggat ttggttaacc tgttttcttc aagcctgagg     2220
ttttatatac aaactccctg aatactcttt ttgccttgta tcttctcagc ctcctagcca     2280
agtcctatgt aatatggaaa acaaacactg cagacttgag attcagttgc cgatcaaggc     2340
tctggcattc agagaaccct tgcaactcga gaagctgttt ttatttcgtt tttgttttga     2400
tccagtgctc tcccatctaa caactaaaca ggagccattt caaggcggga gatattttaa     2460
acacccaaaa tgttgggtct gattttcaaa cttttaaact cactactgat gattctcacg     2520
ctaggcgaat ttgtccaaac acatagtgtg tgtgttttgt atacactgta tgaccccacc     2580
ccaaatcttt gtattgtcca cattctccaa caataaagca cagagtggat ttaattaagc     2640
acacaaatgc taaggcagaa ttttgagggt gggagagaag aaaagggaaa gaagctgaaa     2700
atgtaaaacc acaccaggga ggaaaaatga cattcagaac cagcaaacac tgaatttctc     2760
ttgttgtttt aactctgcca caagaatgca atttcgttaa tggagatgac ttaagttggc     2820
agcagtaatc ttcttttagg agcttgtacc acagtcttgc acataagtgc agatttggct     2880
caagtaaaga gaatttcctc aacactaact tcactgggat aatcagcagc gtaactaccc     2940
taaaagcata tcactagcca aagagggaaa tatctgttct tcttactgtg cctatattaa     3000
gactagtaca aatgtggtgt gtcttccaac tttcattgaa aatgccatat ctataccata     3060
ttttattcga gtcactgatg atgtaatgat atattttttc attattatag tagaatattt     3120
ttatggcaag atatttgtgg tcttgatcat acctattaaa ataatgccaa acaccaaata     3180
tgaattttat gatgtacact ttgtgcttgg cattaaaaga aaaaaacaca catcctggaa     3240
gtctgtaagt tgtttttttgt tactgtaggt cttcaaagtt aagagtgtaa gtgaaaaatc     3300
tggaggagag gataatttcc actgtgtgga atgtgaatag ttaaatgaaa agttatggtt     3360
atttaatgta attattactt caaatccttt ggtcactgtg atttcaagca tgttttcttt     3420
ttctccttta tatgactttc tctgagttgg gcaaagaaga agctgacaca ccgtatgttg     3480
ttagagtctt ttatctggtc aggggaaaca aaatcttgac ccagctgaac atgtcttcct     3540
gagtcagtgc ctgaatcttt attttttaaa ttgaatgttc cttaaaggtt aacatttcta     3600
aagcaatatt aagaaagact ttaaatgtta ttttggaaga cttacgatgc atgtatacaa     3660
acgaatagca gataatgatg actagttcac acataaagtc cttttaagga gaaaatctaa     3720
aatgaaaagt ggataaacag aacatttata agtgatcagt taatgcctaa gagtgaaagt     3780
agttctattg acattcctca agatatttaa tatcaactgc attatgtatt atgtctgctt     3840
aaatcattta aaaacggcaa agaattatat agactatgag gtaccttgct gtgtaggagg     3900
atgaaagggg agttgatagt ctcataaaac taatttggct tcaagtttca tgaatctgta     3960
actagaattt aattttcacc ccaataatgt tctatatagc ctttgctaaa gagcaactaa     4020
taaattaaac ctattctttc aaaaaaaaa                                       4049
```

<210> 10
<211> 184
<212> PRT
<213> Homo sapiens

<220>
<221> Gremlin
<222> (1)..(184)
<223> NP037504

<400> 10

```
Met Ser Arg Thr Ala Tyr Thr Val Gly Ala Leu Leu Leu Leu Leu Gly
1               5                   10                  15

Thr Leu Leu Pro Ala Ala Glu Gly Lys Lys Lys Gly Ser Gln Gly Ala
            20                  25                  30

Ile Pro Pro Pro Asp Lys Ala Gln His Asn Asp Ser Glu Gln Thr Gln
        35                  40                  45

Ser Pro Gln Gln Pro Gly Ser Arg Asn Arg Gly Arg Gly Gln Gly Arg
    50                  55                  60

Gly Thr Ala Met Pro Gly Glu Glu Val Leu Glu Ser Ser Gln Glu Ala
65                  70                  75                  80

Leu His Val Thr Glu Arg Lys Tyr Leu Lys Arg Asp Trp Cys Lys Thr
                85                  90                  95

Gln Pro Leu Lys Gln Thr Ile His Glu Glu Gly Cys Asn Ser Arg Thr
            100                 105                 110

Ile Ile Asn Arg Phe Cys Tyr Gly Gln Cys Asn Ser Phe Tyr Ile Pro
        115                 120                 125

Arg His Ile Arg Lys Glu Glu Gly Ser Phe Gln Ser Cys Ser Phe Cys
    130                 135                 140

Lys Pro Lys Lys Phe Thr Thr Met Met Val Thr Leu Asn Cys Pro Glu
145                 150                 155                 160

Leu Gln Pro Pro Thr Lys Lys Lys Arg Val Thr Arg Val Lys Gln Cys
                165                 170                 175

Arg Cys Ile Ser Ile Asp Leu Asp
            180
```

<210> 11
<211> 1146
<212> DNA
<213> Homo sapiens

<220>

<221> Intelectin
<222> (1)..(1146)
<223> AK000029

<400> 11
```
agggagggag tgaaggagct ctctgtaccc aaggaaagtg cagctgagac tcagacaaga      60
ttacaatgaa ccaactcagc ttcctgctgt ttctcatagc gaccaccaga ggatggagta     120
cagatgaggc taatacttac ttcaaggaat ggacctgttc ttcgtctcca tctctgccca     180
gaagctgcaa ggaaatcaaa gacgaatgtc ctagtgcatt tgatggcctg tattttctcc     240
gcactgagaa tggtgttatc taccagacct tctgtgacat gacctctggg ggtggcggct     300
ggaccctggt ggccagcgtg catgagaatg acatgcgtgg gaagtgcacg gtgggcgatc     360
gctggtccag tcagcagggc agcaaagcag actacccaga gggggacggc aactgggcca     420
actacaacac ctttggatct gcagaggcgg ccacgagcga tgactacaag aaccctggct     480
actacgacat ccaggccaag gacctgggca tctggcacgt gcccaataag tcccccatgc     540
agcactggag aaacagctcc ctgctgaggt accgcacgga cactggcttc ctccagacac     600
tgggacataa tctgtttggc atctaccaga aatatccagt gaaatatgga gaaggaaagt     660
gttggactga caacggcccg gtgatccctg tggtctatga ttttggcgac gcccagaaaa     720
cagcatctta ttactcaccc tatggccagc gggaattcac tgcgggattt gttcagttca     780
gggtatttaa taacgagaga gcagccaacg ccttgtgtgc tggaatgagg gtcaccggat     840
gtaacactga gcaccactgc attggtggag gaggatactt tccagaggcc agtccccagc     900
agtgtggaga tttttctggt tttgattgga gtggatatgg aactcatgtt ggttacagca     960
gcagccgtga gataactgag gcagctgtgc ttctattcta tcgttgagag ttttgtggga    1020
gggaacccag acctctcctc ccaaccatga gatcccaagg atggagaaca acttacccag    1080
tagctagaat gttaatggca gaagagaaaa caataaatca tattgactca aaaaaaaaaa    1140
aaaaaa                                                               1146
```

<210> 12
<211> 313
<212> PRT
<213> Homo sapiens

<220>
<221> Intelectin
<222> (1)..(313)
<223> NP060095

<400> 12

```
Met Asn Gln Leu Ser Phe Leu Leu Phe Leu Ile Ala Thr Thr Arg Gly
1               5                   10                  15

Trp Ser Thr Asp Glu Ala Asn Thr Tyr Phe Lys Glu Trp Thr Cys Ser
            20                  25                  30

Ser Ser Pro Ser Leu Pro Arg Ser Cys Lys Glu Ile Lys Asp Glu Cys
```

```
        35                  40                  45

Pro Ser Ala Phe Asp Gly Leu Tyr Phe Leu Arg Thr Glu Asn Gly Val
    50                  55                  60

Ile Tyr Gln Thr Phe Cys Asp Met Thr Ser Gly Gly Gly Gly Trp Thr
65                  70                  75                  80

Leu Val Ala Ser Val His Glu Asn Asp Met Arg Gly Lys Cys Thr Val
                85                  90                  95

Gly Asp Arg Trp Ser Ser Gln Gln Gly Ser Lys Ala Val Tyr Pro Glu
            100                 105                 110

Gly Asp Gly Asn Trp Ala Asn Tyr Asn Thr Phe Gly Ser Ala Glu Ala
        115                 120                 125

Ala Thr Ser Asp Asp Tyr Lys Asn Pro Gly Tyr Tyr Asp Ile Gln Ala
    130                 135                 140

Lys Asp Leu Gly Ile Trp His Val Pro Asn Lys Ser Pro Met Gln His
145                 150                 155                 160

Trp Arg Asn Ser Ser Leu Leu Arg Tyr Arg Thr Asp Thr Gly Phe Leu
                165                 170                 175

Gln Thr Leu Gly His Asn Leu Phe Gly Ile Tyr Gln Lys Tyr Pro Val
            180                 185                 190

Lys Tyr Gly Glu Gly Lys Cys Trp Thr Asp Asn Gly Pro Val Ile Pro
        195                 200                 205

Val Val Tyr Asp Phe Gly Asp Ala Gln Lys Thr Ala Ser Tyr Tyr Ser
    210                 215                 220

Pro Tyr Gly Gln Arg Glu Phe Thr Ala Gly Phe Val Gln Phe Arg Val
225                 230                 235                 240

Phe Asn Asn Glu Arg Ala Ala Asn Ala Leu Cys Ala Gly Met Arg Val
                245                 250                 255

Thr Gly Cys Asn Thr Glu His His Cys Ile Gly Gly Gly Gly Tyr Phe
            260                 265                 270

Pro Glu Ala Ser Pro Gln Gln Cys Gly Asp Phe Ser Gly Phe Asp Trp
        275                 280                 285

Ser Gly Tyr Gly Thr His Val Gly Tyr Ser Ser Ser Arg Glu Ile Thr
    290                 295                 300

Glu Ala Ala Val Leu Leu Phe Tyr Arg
```

305 310

<210> 13
<211> 1213
<212> DNA
<213> Homo sapiens

<220>
<221> Kallikrein 11
<222> (1)..(1213)
<223> BC022068

<400> 13
agcgtgggag tgcaggcttg gaaagcagga gagctcagcc tacgtcttta atcctcctgc 60

ccaccccttg gattctgtct ccactgggac tcaagagagg aacctggggc ccgctcctcc 120

cccctccagg ccatgaggat tctgcagtta atcctgcttg ctctggcaac agggcttgta 180

gggggagaga ccaggatcat caaggggttc gagtgcaagc ctcactccca gccctggcag 240

gcagccctgt tcgagaagac gcggctactc tgtggggcga cgctcatcgc ccccagatgg 300

ctcctgacag cagcccactg cctcaagccc cgctacatag ttcacctggg gcagcacaac 360

ctccagaagg aggagggctg tgagcagacc cggacagcca ctgagtcctt cccccacccc 420

ggcttcaaca acagcctccc caacaaagac caccgcaatg acatcatgct ggtgaagatg 480

gcatcgccag tctccatcac ctgggctgtg cgacccctca ccctctcctc acgctgtgtc 540

actgctggca ccagctgcct catttccggc tggggcagca cgtccagccc ccagttacgc 600

ctgcctcaca ccttgcgatg cgccaacatc accatcattg agcaccagaa gtgtgagaac 660

gcctaccccg gcaacatcac agacaccatg gtgtgtgcca gcgtgcagga agggggcaag 720

gactcctgcc agggtgactc cgggggccct ctggtctgta accagtctct tcaaggcatt 780

atctcctggg gccaggatcc gtgtgcgatc acccgaaagc ctggtgtcta cacgaaagtc 840

tgcaaatatg tggactggat ccaggagacg atgaagaaca attagactgg acccacccac 900

cacagcccat caccctccat ttccacttgg tgtttggttc ctgttcactc tgttaataag 960

aaaccctaag ccaagaccct ctacgaacat tctttgggcc tcctggacta caggagatgc 1020

tgtcacttaa taatcaacct ggggttcgaa atcagtgaga cctggattca aattctgcct 1080

tgaaatattg tgactctggg aatgacaaca cctggtttgt tctctgttgt atccccagcc 1140

ccaaagacag ctcctggcca tatatcaagg tttcaataaa tatttgctaa atgagtgaaa 1200

aaaaaaaaaa aaa 1213

<210> 14
<211> 250
<212> PRT
<213> Homo sapiens

<220>
<221> Kallikrein 11
<222> (1)..(250)
<223> NP006844

<400> 14

Met Arg Ile Leu Gln Leu Ile Leu Leu Ala Leu Ala Thr Gly Leu Val
1 5 10 15

Gly Gly Glu Thr Arg Ile Ile Lys Gly Phe Glu Cys Lys Pro His Ser
20 25 30

Gln Pro Trp Gln Ala Ala Leu Phe Glu Lys Thr Arg Leu Leu Cys Gly
35 40 45

Ala Thr Leu Ile Ala Pro Arg Trp Leu Leu Thr Ala Ala His Cys Leu
50 55 60

Lys Pro Arg Tyr Ile Val His Leu Gly Gln His Asn Leu Gln Lys Glu
65 70 75 80

Glu Gly Cys Glu Gln Thr Arg Thr Ala Thr Glu Ser Phe Pro His Pro
85 90 95

Gly Phe Asn Asn Ser Leu Pro Asn Lys Asp His Arg Asn Asp Ile Met
100 105 110

Leu Val Lys Met Ala Ser Pro Val Ser Ile Thr Trp Ala Val Arg Pro
115 120 125

Leu Thr Leu Ser Ser Arg Cys Val Thr Ala Gly Thr Ser Cys Leu Ile
130 135 140

Ser Gly Trp Gly Ser Thr Ser Ser Pro Gln Leu Arg Leu Pro His Thr
145 150 155 160

Leu Arg Cys Ala Asn Ile Thr Ile Ile Glu His Gln Lys Cys Glu Asn
165 170 175

Ala Tyr Pro Gly Asn Ile Thr Asp Thr Met Val Cys Ala Ser Val Gln
180 185 190

Glu Gly Gly Lys Asp Ser Cys Gln Gly Asp Ser Gly Gly Pro Leu Val
195 200 205

Cys Asn Gln Ser Leu Gln Gly Ile Ile Ser Trp Gly Gln Asp Pro Cys
210 215 220

Ala Ile Thr Arg Lys Pro Gly Val Tyr Thr Lys Val Cys Lys Tyr Val
225 230 235 240

Asp Trp Ile Gln Glu Thr Met Lys Asn Asn
245 250

<210> 15

<211> 2114
<212> DNA
<213> Homo sapiens

<220>
<221> Mesothelin (variant 2)
<222> (1)..(2114)
<223> U40434

<400> 15
aggaattccg gtggccggcc actcccgtct gctgtgacgc gcggacagag agctaccggt 60

ggacccacgg tgcctccctc cctgggatct acacagacca tggccttgca acggctcgac 120

ccctgttggt cctgtgggga ccgccctggc agcctcctgt tcctgctctt cagcctcgga 180

tgggtgcatc ccgcgaggac cctggctgga gagacaggga cggagtctgc cccccctgggg 240

ggagtcctga caaccccccca taacatttcc agcctctccc ctcgccaact ccttggcttc 300

ccgtgtgcgg aggtgtccgg cctgagcacg gagcgtgtcc gggagctggc tgtggccttg 360

gcacagaaga atgtcaagct ctcaacagag cagctgcgct gtctggctca ccggctctct 420

gagcccccccg aggacctgga cgccctccca ttggacctgc tgctattcct caacccagat 480

gcgttctcgg ggccccaggc ctgcacccgt ttcttctccc gcatcacgaa ggccaatgtg 540

gacctgctcc cgagggggggc tcccgagcga cagcggctgc tgcctgcggc tctggcctgc 600

tggggtgtgc gggggtctct gctgagcgag gctgatgtgc gggctctggg aggcctggct 660

tgcgacctgc ctgggcgctt tgtggccgag tcggccgaag tgctgctacc ccggctggtg 720

agctgcccgg gacccctgga ccaggaccag caggaggcag ccagggcggc tctgcagggc 780

gggggacccc cctacggccc cccgtcgaca tggtctgtct ccacgatgga cgctctgcgg 840

ggcctgctgc ccgtgctggg ccagcccatc atccgcagca tcccgcaggg catcgtggcc 900

gcgtggcggc aacgctcctc tcgggaccca tcctggcggc agcctgaacg gaccatcctc 960

cggccgcggt tccggcggga agtggagaag acagcctgtc cttcaggcaa gaaggcccgc 1020

gagatagacg agagcctcat cttctacaag aagtgggagc tggaagcctg cgtggatgcg 1080

gccctgctgg ccacccagat ggaccgcgtg aacgccatcc ccttcaccta cgagcagctg 1140

gacgtcctaa agcataaact ggatgagctc tacccacaag gttaccccga gtctgtgatc 1200

cagcacctgg gctacctctt cctcaagatg agccctgagg acattcgcaa gtggaatgtg 1260

acgtccctgg agaccctgaa ggctttgctt gaagtcgaca aagggcacga aatgagtcct 1320

caggctcctc ggcggcccct cccacaggtg gccaccctga tcgaccgctt tgtgaaggga 1380

aggggccagc tagacaaaga caccctagac accctgaccg ccttctaccc tgggtacctg 1440

tgctccctca gccccgagga gctgagctcc gtgcccccca gcagcatctg ggcggtcagg 1500

ccccaggacc tggacacgtg tgacccaagg cagctggacg tcctctatcc caaggcccgc 1560

cttgctttcc agaacatgaa cgggtccgaa tacttcgtga agatccagtc cttcctgggt 1620

ggggccccca cggaggattt gaaggcgctc agtcagcaga atgtgagcat ggacttggcc 1680

acgttcatga agctgcggac ggatgcggtg ctgccgttga ctgtggctga ggtgcagaaa 1740

cttctgggac cccacgtgga gggcctgaag gcggaggagc ggcaccgccc ggtgcgggac 1800

tggatcctac ggcagcggca ggacgacctg gacacgctgg ggctggggct acagggcggc 1860

atccccaacg gctacctggt cctagacctc agcgtgcaag agaccctctc ggggacgccc 1920

tgcctcctag gacctggacc tgttctcacc gtcctggcac tgctcctagc ctccaccctg 1980

gcctgagggc cccactccct tgctggcccc agccctgctg gggatccccg cctggccagg 2040

agcaggcacg ggtgatcccc gttccacccc aagagaactc gcgctcagta aacgggaaca 2100

tgccccctgc agac 2114

<210> 16
<211> 628
<212> PRT
<213> Homo sapiens

<220>
<221> Mesothelin (variant 2)
<222> (1)..(628)
<223> NP037536

<400> 16

Met Ala Leu Gln Arg Leu Asp Pro Cys Trp Ser Cys Gly Asp Arg Pro
1               5                   10                  15

Gly Ser Leu Leu Phe Leu Leu Phe Ser Leu Gly Trp Val His Pro Ala
            20                  25                  30

Arg Thr Leu Ala Gly Glu Thr Gly Thr Glu Ser Ala Pro Leu Gly Gly
        35                  40                  45

Val Leu Thr Thr Pro His Asn Ile Ser Ser Leu Ser Pro Arg Gln Leu
    50                  55                  60

Leu Gly Phe Pro Cys Ala Glu Val Ser Gly Leu Ser Thr Glu Arg Val
65                  70                  75                  80

Arg Glu Leu Ala Val Ala Leu Ala Gln Lys Asn Val Lys Leu Ser Thr
                85                  90                  95

Glu Gln Leu Arg Cys Leu Ala His Arg Leu Ser Glu Pro Pro Glu Asp
            100                 105                 110

Leu Asp Ala Leu Pro Leu Asp Leu Leu Leu Phe Leu Asn Pro Asp Ala
        115                 120                 125

Phe Ser Gly Pro Gln Ala Cys Thr Arg Phe Phe Ser Arg Ile Thr Lys
    130                 135                 140

Ala Asn Val Asp Leu Leu Pro Arg Gly Ala Pro Glu Arg Gln Arg Leu
145                 150                 155                 160

```
Leu Pro Ala Ala Leu Ala Cys Trp Gly Val Arg Gly Ser Leu Leu Ser
                165                 170                 175

Glu Ala Asp Val Arg Ala Leu Gly Gly Leu Ala Cys Asp Leu Pro Gly
            180                 185                 190

Arg Phe Val Ala Glu Ser Ala Glu Val Leu Leu Pro Arg Leu Val Ser
        195                 200                 205

Cys Pro Gly Pro Leu Asp Gln Asp Gln Gln Glu Ala Ala Arg Ala Ala
    210                 215                 220

Leu Gln Gly Gly Gly Pro Pro Tyr Gly Pro Pro Ser Thr Trp Ser Val
225                 230                 235                 240

Ser Thr Met Asp Ala Leu Arg Gly Leu Leu Pro Val Leu Gly Gln Pro
                245                 250                 255

Ile Ile Arg Ser Ile Pro Gln Gly Ile Val Ala Ala Trp Arg Gln Arg
            260                 265                 270

Ser Ser Arg Asp Pro Ser Trp Arg Gln Pro Glu Arg Thr Ile Leu Arg
        275                 280                 285

Pro Arg Phe Arg Arg Glu Val Glu Lys Thr Ala Cys Pro Ser Gly Lys
    290                 295                 300

Lys Ala Arg Glu Ile Asp Glu Ser Leu Ile Phe Tyr Lys Lys Trp Glu
305                 310                 315                 320

Leu Glu Ala Cys Val Asp Ala Ala Leu Leu Ala Thr Gln Met Asp Arg
                325                 330                 335

Val Asn Ala Ile Pro Phe Thr Tyr Glu Gln Leu Asp Val Leu Lys His
            340                 345                 350

Lys Leu Asp Glu Leu Tyr Pro Gln Gly Tyr Pro Glu Ser Val Ile Gln
        355                 360                 365

His Leu Gly Tyr Leu Phe Leu Lys Met Ser Pro Glu Asp Ile Arg Lys
    370                 375                 380

Trp Asn Val Thr Ser Leu Glu Thr Leu Lys Ala Leu Leu Glu Val Asp
385                 390                 395                 400

Lys Gly His Glu Met Ser Pro Gln Ala Pro Arg Arg Pro Leu Pro Gln
                405                 410                 415

Val Ala Thr Leu Ile Asp Arg Phe Val Lys Gly Arg Gly Gln Leu Asp
            420                 425                 430
```

```
Lys Asp Thr Leu Asp Thr Leu Thr Ala Phe Tyr Pro Gly Tyr Leu Cys
        435                 440                 445

Ser Leu Ser Pro Glu Glu Leu Ser Ser Val Pro Pro Ser Ser Ile Trp
    450                 455                 460

Ala Val Arg Pro Gln Asp Leu Asp Thr Cys Asp Pro Arg Gln Leu Asp
465                 470                 475                 480

Val Leu Tyr Pro Lys Ala Arg Leu Ala Phe Gln Asn Met Asn Gly Ser
                485                 490                 495

Glu Tyr Phe Val Lys Ile Gln Ser Phe Leu Gly Gly Ala Pro Thr Glu
            500                 505                 510

Asp Leu Lys Ala Leu Ser Gln Gln Asn Val Ser Met Asp Leu Ala Thr
        515                 520                 525

Phe Met Lys Leu Arg Thr Asp Ala Val Leu Pro Leu Thr Val Ala Glu
    530                 535                 540

Val Gln Lys Leu Leu Gly Pro His Val Glu Gly Leu Lys Ala Glu Glu
545                 550                 555                 560

Arg His Arg Pro Val Arg Asp Trp Ile Leu Arg Gln Arg Gln Asp Asp
                565                 570                 575

Leu Asp Thr Leu Gly Leu Gly Leu Gln Gly Gly Ile Pro Asn Gly Tyr
            580                 585                 590

Leu Val Leu Asp Leu Ser Val Gln Glu Thr Leu Ser Gly Thr Pro Cys
        595                 600                 605

Leu Leu Gly Pro Gly Pro Val Leu Thr Val Leu Ala Leu Leu Leu Ala
    610                 615                 620

Ser Thr Leu Ala
625


<210>  17
<211>  2650
<212>  DNA
<213>  Homo sapiens


<220>
<221>  Pleiotrophin
<222>  (1)..(2650)
<223>  AB004306

<400>  17
gaattcggca cgagctgcag ggtcaggagg agaatcgtgg ggccaggagg gcagaggcac      60

actccatctt cgtgctcctc acaggccctg cctccctgcc tgctaaggac acagggaagg     120
```

gggtccccac ctcagtgcct gcctcccttc cctgtgcctg tgtacctggc agtcacagcc 180
acctggcgtg tcccagaaac caaccggctg acctcatctc ctgcccggcc ccacctccat 240
tggctttggc ttttggcgtt tgtgctgccc gaccctttct cctgtccgga tgcgcagggc 300
agggcctgag ccgtcgagct gcacccacag caggctgcct ttggtgactc accgggtgaa 360
cgggggcatt gcgaggcatc ccctccctgg gtttggctcc tgcccacggg gctgacagta 420
gaaatcacag gctgtgagac agctggagcc cagctctgct tgaacctatt ttaggtctct 480
gatccccgct tcctctttag actcccctag agctcagcca gtgctcaacc tgaggctggg 540
ggtctctgag gaagagtgag ttggagctga ggggtctggg gctgtcccct gagagagggg 600
ccagaggcag tgtcaagagc cgggcagtct gattgtggct caccctccat cactcccagg 660
gcccctggcc cagcagccgc agctcccaac cacaatatcc tttggggttt ggcctacgga 720
gctggggcgg atgaccccca aatagccctg gcagattccc cctagacccg cccgcaccat 780
ggtcaggcat gcccctcctc atcgctggca cagcccagag ggtataaaca gtgctggagg 840
ctggcggggc aggccagctg agtcctgagc agcagcccag cggatcctga gaacttcagg 900
gtgagtttgg ggacccttga ttgttctttc tttttcgcta ttgtaaaatt catgttatat 960
ggagggggca aagttttcag ggtgttgttt agaatgggaa gatgtccctt gtatcaccat 1020
ggaccctcat gataattttg tttctttcac tttctactct gttgacaacc attgtctcct 1080
cttattttct tttcattttc tgtaactttt tcgttaaact ttagcttgca tttgtaacga 1140
atttttaaat tcacttttgt ttatttgtca gattgtaagt actttctcta atcacttttt 1200
tttcaaggca atcagggtat attatattgt acttcagcac agttttagag aacaattgtt 1260
ataattaaat gataaggtag aatatttctg catataaatt ctggctggcg tggaaatatt 1320
cttattggta gaaacaacta catcctggtc atcatcctgc ctttctcttt atggttacaa 1380
tgatatacac tgtttgagat gaggataaaa tactctgagt ccaaaccggg cccctctgct 1440
aaccatgttc atgccttctt ctttttccta cagctcctgg gcaacgtgct ggttgttgtg 1500
ctgtctcatc attttggcaa agaattaatt ccaactcaaa aatgcaggct caacagtacc 1560
agcagcagcg tcgaaaattt gcagctgcct tcttggcatt cattttcata ctggcagctg 1620
tggatactgc tgaagcaggg aagaaagaga aaccagaaaa aaaagtgaag aagtctgact 1680
gtggagaatg gcagtggagt gtgtgtgtgc ccaccagtgg agactgtggg ctgggcacac 1740
gggagggcac tcggactgga gctgagtgca agcaaaccat gaagacccag agatgtaaga 1800
tcccctgcaa ctggaagaag caatttggcg cggagtgcaa ataccagttc caggcctggg 1860
gagaatgtga cctgaacaca gccctgaaga ccagaactgg aagtctgaag cgagccctgc 1920
acaatgccga atgccagaag actgtcacca tctccaagcc ctgtggcaaa ctgaccaagc 1980
ccaaacctca agcagaatct aagaagaaga aaaaggaagg caagaaacag gagaagatgc 2040
tggattaaaa gatgtcacct gtggaacata aaaaggacat cagcaaacag gatcaattca 2100
ctcctcaggt gcaggctgcc tatcagaagg tggtggctgg tgtggccaat gccctggctc 2160

acaaatacca ctgagatctt tttccctctg ccaaaaatta tggggacatc atgaagcccc 2220

ttgagcatct gacttctggc taataaagga aatttatttt cattgcaata gtgtgttgga 2280

atttttttgtg tctctcactc ggaaggacat atgggagggc aaatcattta aaacatcaga 2340

atgagtattt ggtttagagt ttggcaacat atgccatatg ctggctgcca tgaacaaagg 2400

tggctataaa gaggtcatca gtatatgaaa cagcccctg ctgtccattc cttattccat 2460

agaaaagcct tgacttgagg ttagattttt tttatatttt gttttgtgtt attttttttct 2520

ttaacatccc taaaattttc cttacatgtt ttactagcca gatttttcct cctctcctga 2580

ctactcccag tcatagctgt ccctcttctc ttatggagat ccctcgacct gcaggcaggc 2640

atgcaagctt 2650

<210> 18
<211> 168
<212> PRT
<213> Homo sapiens

<220>
<221> Pleiotrophin
<222> (1)..(168)
<223> NP002816

<400> 18

```
Met Gln Ala Gln Gln Tyr Gln Gln Gln Arg Arg Lys Phe Ala Ala Ala
1               5                   10                  15

Phe Leu Ala Phe Ile Phe Ile Leu Ala Ala Val Asp Thr Ala Glu Ala
            20                  25                  30

Gly Lys Lys Glu Lys Pro Glu Lys Lys Val Lys Lys Ser Asp Cys Gly
        35                  40                  45

Glu Trp Gln Trp Ser Val Cys Val Pro Thr Ser Gly Asp Cys Gly Leu
    50                  55                  60

Gly Thr Arg Glu Gly Thr Arg Thr Gly Ala Glu Cys Lys Gln Thr Met
65                  70                  75                  80

Lys Thr Gln Arg Cys Lys Ile Pro Cys Asn Trp Lys Lys Gln Phe Gly
                85                  90                  95

Ala Glu Cys Lys Tyr Gln Phe Gln Ala Trp Gly Glu Cys Asp Leu Asn
            100                 105                 110

Thr Ala Leu Lys Thr Arg Thr Gly Ser Leu Lys Arg Ala Leu His Asn
        115                 120                 125

Ala Glu Cys Gln Lys Thr Val Thr Ile Ser Lys Pro Cys Gly Lys Leu
    130                 135                 140
```

Thr Lys Pro Lys Pro Gln Ala Glu Ser Lys Lys Lys Lys Lys Glu Gly
145                 150                 155                 160

Lys Lys Gln Glu Lys Met Leu Asp
                165

<210> 19
<211> 852
<212> DNA
<213> Homo sapiens

<220>
<221> Small inducible cytokine subfamily A member 21
<222> (1)..(852)
<223> AB002409

<400> 19
cttgcagctg cccacctcac cctcagctct ggcctcttac tcaccctcta ccacagacat 60

ggctcagtca ctggctctga gcctccttat cctggttctg gcctttggca tccccaggac 120

ccaaggcagt gatggagggg ctcaggactg ttgcctcaag tacagccaaa ggaagattcc 180

cgccaaggtt gtccgcagct accggaagca ggaaccaagc ttaggctgct ccatcccagc 240

tatcctgttc ttgccccgca agcgctctca ggcagagcta tgtgcagacc caaaggagct 300

ctgggtgcag cagctgatgc agcatctgga caagacacca tccccacaga aaccagccca 360

gggctgcagg aaggacaggg gggcctccaa gactggcaag aaaggaaagg gctccaaagg 420

ctgcaagagg actgagcggt cacagacccc taaagggcca tagcccagtg agcagcctgg 480

agccctggag accccaccag cctcaccaac gcttgaagcc tgaacccaag atgcaagaag 540

gaggctatgc tcaggggccc tggagcagcc accccatgct ggccttgcca cactctttct 600

cctgctttaa ccaccccatc tgcattccca gctctaccct gcatggctga gctgcccaca 660

gcaggccagg tccagagaga ccgaggaggg agagtctccc agggagcatg agaggaggca 720

gcaggactgt ccccttgaag gagaatcatc aggaccctgg acctgatacg gctccccagt 780

acaccccacc tcttccttgt aaatatgatt tatacctaac tgaataaaaa gctgttctgt 840

cttcccaccc gc 852

<210> 20
<211> 134
<212> PRT
<213> Homo sapiens

<220>
<221> Small inducible cytokine subfamily A member 21
<222> (1)..(134)
<223> NP002980

<400> 20

Met Ala Gln Ser Leu Ala Leu Ser Leu Leu Ile Leu Val Leu Ala Phe
1               5                   10                  15

Gly Ile Pro Arg Thr Gln Gly Ser Asp Gly Gly Ala Gln Asp Cys Cys
20 25 30

Leu Lys Tyr Ser Gln Arg Lys Ile Pro Ala Lys Val Val Arg Ser Tyr
35 40 45

Arg Lys Gln Glu Pro Ser Leu Gly Cys Ser Ile Pro Ala Ile Leu Phe
50 55 60

Leu Pro Arg Lys Arg Ser Gln Ala Glu Leu Cys Ala Asp Pro Lys Glu
65 70 75 80

Leu Trp Val Gln Gln Leu Met Gln His Leu Asp Lys Thr Pro Ser Pro
85 90 95

Gln Lys Pro Ala Gln Gly Cys Arg Lys Asp Arg Gly Ala Ser Lys Thr
100 105 110

Gly Lys Lys Gly Lys Gly Ser Lys Gly Cys Lys Arg Thr Glu Arg Ser
115 120 125

Gln Thr Pro Lys Gly Pro
130

<210> 21
<211> 685
<212> DNA
<213> Homo sapiens

<220>
<221> Trefoil Factor 3
<222> (1)..(685)
<223> NM003226

<400> 21
gccaaaacag tgggggctga actgacctct cccctttggg agagaaaaac tgtctgggag 60

cttgacaaag gcatgcagga gagaacagga gcagccacag ccaggaggga gagccttccc 120

caagcaaaca atccagagca gctgtgcaaa caacggtgca taaatgaggc ctcctggacc 180

atgaagcgag tcctgagctg cgtcccggag cccacggtgg tcatggctgc cagagcgctc 240

tgcatgctgg ggctggtcct ggccttgctg tcctccagct ctgctgagga gtacgtgggc 300

ctgtctgcaa accagtgtgc cgtgccagcc aaggacaggg tggactgcgg ctacccccat 360

gtcaccccca aggagtgcaa caaccggggc tgctgctttg actccaggat ccctggagtg 420

ccttggtgtt tcaagcccct gcaggaagca gaatgcacct tctgaggcac ctccagctgc 480

ccccggccgg gggatgcgag gctcggagca cccttgcccg gctgtgattg ctgccaggca 540

ctgttcatct cagcttttct gtccctttgc tcccggcaag cgcttctgct gaaagttcat 600

atctggagcc tgatgtctta acgaataaag gtcccatgct ccacccgagg acagttcttc 660

gtgcctgaaa aaaaaaaaaa aaaaa 685

<210> 22
<211> 130
<212> PRT
<213> Homo sapiens

<220>
<221> Trefoil Factor 3
<222> (1)..(130)
<223> NP003217

<400> 22

Met Gln Glu Arg Thr Gly Ala Ala Thr Ala Arg Arg Glu Ser Leu Pro
1 5 10 15

Gln Ala Asn Asn Pro Glu Gln Leu Cys Lys Gln Arg Cys Ile Asn Glu
20 25 30

Ala Ser Trp Thr Met Lys Arg Val Leu Ser Cys Val Pro Glu Pro Thr
35 40 45

Val Val Met Ala Ala Arg Ala Leu Cys Met Leu Gly Leu Val Leu Ala
50 55 60

Leu Leu Ser Ser Ser Ser Ala Glu Glu Tyr Val Gly Leu Ser Ala Asn
65 70 75 80

Gln Cys Ala Val Pro Ala Lys Asp Arg Val Asp Cys Gly Tyr Pro His
85 90 95

Val Thr Pro Lys Glu Cys Asn Asn Arg Gly Cys Cys Phe Asp Ser Arg
100 105 110

Ile Pro Gly Val Pro Trp Cys Phe Lys Pro Leu Gln Glu Ala Glu Cys
115 120 125

Thr Phe
130

<210> 23
<211> 1142
<212> DNA
<213> Homo sapiens

<220>
<221> Tissue factor pathway inhibitor 2
<222> (1)..(1142)
<223> D29992

<400> 23
gccgccagcg gctttctcgg acgccttgcc cagcgggccg cccgaccccc tgcaccatgg 60

accccgctcg ccccctgggg ctgtcgattc tgctgctttt cctgacggag gctgcactgg 120

gcgatgctgc tcaggagcca acaggaaata acgcggagat ctgtctcctg cccctagact 180

```
acggaccctg ccgggcccta cttctccgtt actactacga caggtacacg cagagctgcc      240
gccagttcct gtacgggggc tgcgagggca acgccaacaa tttctacacc tgggaggctt      300
gcgacgatgc ttgctggagg atagaaaaag ttcccaaagt ttgccggctg caagtgagtg      360
tggacgacca gtgtgagggg tccacagaaa agtatttctt taatctaagt tccatgacat      420
gtgaaaaatt cttttccggt gggtgtcacc ggaaccggat tgagaacagg tttccagatg      480
aagctacttg tatgggcttc tgcgcaccaa agaaaattcc atcattttgc tacagtccaa      540
aagatgaggg actgtgctct gccaatgtga ctcgctatta ttttaatcca agatacagaa      600
cctgtgatgc tttcacctat actggctgtg gagggaatga caataacttt gttagcaggg      660
aggattgcaa acgtgcatgt gcaaaagctt tgaaaaagaa aaagaagatg ccaaagcttc      720
gctttgccag tagaatccgg aaaattcgga agaagcaatt ttaaacattc ttaatatgtc      780
atcttgtttg tctttatggc ttatttgcct ttatggttgt atctgaagaa taatatgaca      840
gcatgaggaa acaaatcatt ggtgatttat tcaccagttt ttattaatac aagtcacttt      900
ttcaaaaatt tggatttttt tatatataac tagctgctat tcaaatgtga gtctaccatt      960
tttaatttat ggttcaactg tttgtgagac gaattcttgc aatgcataag atataaaagc     1020
aaatatgact cactcatttc ttggggtcgt attcctgatt tcagaagagg atcataactg     1080
aaacaacata agacaatata atcatgtgct tttaacatat ttgagaataa aaaggactag     1140
cc                                                                    1142
```

```
<210>  24
<211>  235
<212>  PRT
<213>  Homo sapiens

<220>
<221>  Tissue factor pathway inhibitor 2
<222>  (1)..(235)
<223>  NP006519

<400>  24

Met Asp Pro Ala Arg Pro Leu Gly Leu Ser Ile Leu Leu Leu Phe Leu
1               5                   10                  15

Thr Glu Ala Ala Leu Gly Asp Ala Ala Gln Glu Pro Thr Gly Asn Asn
            20                  25                  30

Ala Glu Ile Cys Leu Leu Pro Leu Asp Tyr Gly Pro Cys Arg Ala Leu
        35                  40                  45

Leu Leu Arg Tyr Tyr Tyr Asp Arg Tyr Thr Gln Ser Cys Arg Gln Phe
    50                  55                  60

Leu Tyr Gly Gly Cys Glu Gly Asn Ala Asn Asn Phe Tyr Thr Trp Glu
65                  70                  75                  80
```

```
Ala Cys Asp Asp Ala Cys Trp Arg Ile Glu Lys Val Pro Lys Val Cys
                85                  90                  95

Arg Leu Gln Val Ser Val Asp Asp Gln Cys Glu Gly Ser Thr Glu Lys
            100                 105                 110

Tyr Phe Phe Asn Leu Ser Ser Met Thr Cys Glu Lys Phe Phe Ser Gly
        115                 120                 125

Gly Cys His Arg Asn Arg Ile Glu Asn Arg Phe Pro Asp Glu Ala Thr
    130                 135                 140

Cys Met Gly Phe Cys Ala Pro Lys Lys Ile Pro Ser Phe Cys Tyr Ser
145                 150                 155                 160

Pro Lys Asp Glu Gly Leu Cys Ser Ala Asn Val Thr Arg Tyr Tyr Phe
                165                 170                 175

Asn Pro Arg Tyr Arg Thr Cys Asp Ala Phe Thr Tyr Thr Gly Cys Gly
            180                 185                 190

Gly Asn Asp Asn Asn Phe Val Ser Arg Glu Asp Cys Lys Arg Ala Cys
        195                 200                 205

Ala Lys Ala Leu Lys Lys Lys Lys Lys Met Pro Lys Leu Arg Phe Ala
    210                 215                 220

Ser Arg Ile Arg Lys Ile Arg Lys Lys Gln Phe
225                 230                 235
```

<210> 25
<211> 5699
<212> DNA
<213> Homo sapiens

<220>
<221> Sulfatase 1
<222> (1)..(5699)
<223> NM015170

<400> 25

```
ggagttctca gacctccagt ttcagccctg ccctcagcct ccaatccgta agagacaccc      60
agccccagca attggattgg gcagcccgtc ttgacacacc actgtgctga gtgcttgagg     120
acgtgtttca acagatggtt ggggttagtg tgtgtcatca cattcgagtg gggattaaga     180
gaaggaaggc tgccttgctg gagctgtgtg gtcttctcca agtgagagtc gcaggcaata     240
gaactacttt gcttttggag gaaaaggagg aattcatttt cagcagacac aagaaaagca     300
gttttttttt cagggattct tcacttctct tgaacaagga actcactcag agactaacac     360
aaaggaagta atttcttacc tggtcattat ttagtctaca ataagttcat ccttcttcag     420
tgtgaccagt aaattcttcc catactcttg aagagagcat aattggaatg gagaggtggt     480
```

```
gctgacggcc acccaccatc atctaaagaa gataaacttg gcaaatgaca tgcaggttct      540
tcaaggcaga ataattgcag aaaatcttca aaggacccta tctgcagatg ttctgaatac      600
ctctgagaat agagattgat tattcaacca ggatacctaa ttcaagaact ccagaaatca      660
ggagacggag acattttgtc agttttgcaa cattggacca aatacaatga agtattcttg      720
ctgtgctctg gttttggctg tcctgggcac agaattgctg ggaagcctct gttcgactgt      780
cagatccccg aggttcagag gacggataca gcaggaacga aaaaacatcc gacccaacat      840
tattcttgtg cctaccgatg atcaagatgt ggagctgggg tccctgcaag tcatgaacaa      900
aacgagaaag attatggaac atgggggggc caccttcatc aatgcctttg tgactacacc      960
catgtgctgc ccgtcacggt cctccatgct caccgggaag tatgtgcaca atcacaatgt     1020
ctacaccaac aacgagaact gctcttcccc ctcgtggcag gccatgcatg agcctcggac     1080
ttttgctgta tatcttaaca acactggcta cagaacagcc tttttttggaa aatacctcaa   1140
tgaatataat ggcagctaca tcccccctgg gtggcgagaa tggcttggat taatcaagaa     1200
ttctcgcttc tataattaca ctgtttgtcg caatggcatc aaagaaaagc atggatttga     1260
ttatgcaaag gactacttca cagacttaat cactaacgag agcattaatt acttcaaaat     1320
gtctaagaga atgtatcccc ataggcccgt tatgatggtg atcagccacg ctgcgcccca     1380
cggccccgag gactcagccc cacagttttc taaactgtac cccaatgctt cccaacacat     1440
aactcctagt tataactatg caccaaatat ggataaacac tggattatgc agtacacagg     1500
accaatgctg cccatccaca tggaatttac aaacattcta cagcgcaaaa ggctccagac     1560
tttgatgtca gtggatgatt ctgtggagag gctgtataac atgctcgtgg agacggggga     1620
gctggagaat acttacatca tttacaccgc cgaccatggt taccatattg ggcagtttgg     1680
actggtcaag ggaaatcca tgccatatga ctttgatatt cgtgtgcctt tttttattcg     1740
tggtccaagt gtagaaccag gatcaatagt cccacagatc gttctcaaca ttgacttggc     1800
ccccacgatc ctggatattg ctgggctcga cacacctcct gatgtggacg gcaagtctgt     1860
cctcaaactt ctggacccag aaaagccagg taacaggttt cgaacaaaca agaaggccaa     1920
aatttggcgt gatacattcc tagtggaaag aggcaaattt ctacgtaaga aggaagaatc     1980
cagcaagaat atccaacagt caaatcactt gcccaaatat gaacgggtca aagaactatg     2040
ccagcaggcc aggtaccaga cagcctgtga acaaccgggg cagaagtggc aatgcattga     2100
ggatacatct ggcaagcttc gaattcacaa gtgtaaagga cccagtgacc tgctcacagt     2160
ccggcagagc acgcggaacc tctacgctcg cggcttccat gacaaagaca aagagtgcag     2220
ttgtagggag tctggttacc gtgccagcag aagccaaaga aagagtcaac ggcaattctt     2280
gagaaaccag ggactccaa agtacaagcc cagatttgtc catactcggc agacacgttc     2340
cttgtccgtc gaatttgaag gtgaaatata tgacataaat ctggaagaag aagaagaatt     2400
gcaagtgttg caaccaagaa acattgctaa gcgtcatgat gaaggccaca aggggccaag     2460
agatctccag gcttccagtg gtggcaacag gggcaggatg ctggcagata gcagcaacgc     2520
```

cgtgggccca cctaccactg tccgagtgac acacaagtgt tttattcttc ccaatgactc 2580
tatccattgt gagagagaac tgtaccaatc ggccagagcg tggaaggacc ataaggcata 2640
cattgacaaa gagattgaag ctctgcaaga taaaattaag aatttaagag aagtgagagg 2700
acatctgaag agaaggaagc ctgaggaatg tagctgcagt aaacaaagct attacaataa 2760
agagaaaggt gtaaaaaagc aagagaaatt aaagagccat cttcacccat tcaaggaggc 2820
tgctcaggaa gtagatagca aactgcaact tttcaaggag aacaaccgta ggaggaagaa 2880
ggagaggaag gagaagagac ggcagaggaa gggggaagag tgcagcctgc ctggcctcac 2940
ttgcttcacg catgacaaca accactggca gacagccccg ttctggaacc tgggatcttt 3000
ctgtgcttgc acgagttcta acaataacac ctactggtgt ttgcgtacag ttaatgagac 3060
gcataatttt cttttctgtg agtttgctac tggctttttg gagtattttg atatgaatac 3120
agatccttat cagctcacaa atacagtgca cacggtagaa cgaggcattt tgaatcagct 3180
acacgtacaa ctaatggagc tcagaagctg tcaaggatat aagcagtgca acccaagacc 3240
taagaatctt gatgttggaa ataaagatgg aggaagctat gacctacaca gaggacagtt 3300
atgggatgga tgggaaggtt aatcagcccc gtctcactgc agacatcaac tggcaaggcc 3360
tagaggagct acacagtgtg aatgaaaaca tctatgagta cagacaaaac tacagactta 3420
gtctggtgga ctggactaat tacttgaagg atttagatag agtatttgca ctgctgaaga 3480
gtcactatga gcaaaataaa acaaataaga ctcaaactgc tcaaagtgac gggttcttgg 3540
ttgtctctgc tgagcacgct gtgtcaatgg agatggcctc tgctgactca gatgaagacc 3600
caaggcataa ggttgggaaa acacctcatt tgaccttgcc agctgacctt caaaccctgc 3660
atttgaaccg accaacatta agtccagaga gtaaacttga atggaataac gacattccag 3720
aagttaatca tttgaattct gaacactgga gaaaaaccga aaaatggacg gggcatgaag 3780
agactaatca tctggaaacc gatttcagtg gcgatggcat gacagagcta gagctcgggc 3840
ccagccccag gctgcagccc attcgcaggc acccgaaaga acttccccag tatggtggtc 3900
ctggaaagga catttttgaa gatcaactat atcttcctgt gcattccgat ggaatttcag 3960
ttcatcagat gttcaccatg gccaccgcag aacaccgaag taattccagc atagcgggga 4020
agatgttgac caaggtggag aagaatcacg aaaaggagaa gtcacagcac ctagaaggca 4080
gcgcctcctc ttcactctcc tctgattaga tgaaactgtt accttaccct aaacacagta 4140
tttcttttta actttttat ttgtaaacta ataaaggtaa tcacagccac caacattcca 4200
agctaccctg ggtacctttg tgcagtagaa gctagtgagc atgtgagcaa gcggtgtgca 4260
cacggagact catcgttata atttactatc tgccaagagt agaaagaaag gctggggata 4320
tttgggttgg cttggttttg attttttgct tgtttgtttg ttttgtacta aaacagtatt 4380
atcttttgaa tatcgtaggg acataagtat atacatgtta tccaatcaag atggctagaa 4440
tggtgccttt ctgagtgtct aaaacttgac acccctggta aatctttcaa cacacttcca 4500
ctgcctgcgt aatgaagttt tgattcattt ttaaccactg gaatttttca atgccgtcat 4560

tttcagttag atgattttgc actttgagat taaaatgcca tgtctatttg attagtctta 4620
ttttttatt tttacaggct tatcagtctc actgttggct gtcattgtga caaagtcaaa 4680
taaacccca aggacgacac acagtatgga tcacatattg tttgacatta agcttttgcc 4740
agaaaatgtt gcatgtgttt tacctcgact tgctaaaatc gattagcaga aaggcatggc 4800
taataatgtt ggtggtgaaa ataaataaat aagtaaacaa aatgaagatt gcctgctctc 4860
tctgtgccta gcctcaaagc gttcatcata catcatacct ttaagattgc tatattttgg 4920
gttattttct tgacaggaga aaaagatcta aagatctttt attttcatct tttttggttt 4980
tcttggcatg actaagaagc ttaaatgttg ataaaatatg actagttttg aatttacacc 5040
aagaacttct caataaaaga aaatcatgaa tgctccacaa tttcaacata ccacaagaga 5100
agttaatttc ttaacattgt gttctatgat tatttgtaag accttcacca agttctgata 5160
tcttttaaag acatagttca aaattgcttt tgaaaatctg tattcttgaa aatatccttg 5220
ttgtgtatta ggtttttaaa taccagctaa aggattacct cactgagtca tcagtaccct 5280
cctattcagc tccccaagat gatgtgtttt tgcttaccct aagagaggtt ttcttcttat 5340
ttttagataa ttcaagtgct tagataaatt atgttttctt taagtgttta tggtaaactc 5400
ttttaaagaa aatttaatat gttatagctg aatctttttg gtaactttaa atctttatca 5460
tagactctgt acatatgttc aaattagctg cttgcctgat gtgtgtatca tcggtgggat 5520
gacagaacaa acatatttat gatcatgaat aatgtgcttt gtaaaaagat ttcaagttat 5580
taggaagcat actctgtttt ttaatcatgt ataatattcc atgatacttt tatagaacaa 5640
ttctggcttc aggaaagtct agaagcaata tttcttcaaa taaaaggtgt ttaaactttt 5699

<210> 26
<211> 871
<212> PRT
<213> Homo sapiens

<220>
<221> Sulfatase 1
<222> (1)..(871)
<223> NP055985

<400> 26

Met Lys Tyr Ser Cys Cys Ala Leu Val Leu Ala Val Leu Gly Thr Glu
1               5                   10                  15

Leu Leu Gly Ser Leu Cys Ser Thr Val Arg Ser Pro Arg Phe Arg Gly
            20                  25                  30

Arg Ile Gln Gln Glu Arg Lys Asn Ile Arg Pro Asn Ile Ile Leu Val
        35                  40                  45

Pro Thr Asp Asp Gln Asp Val Glu Leu Gly Ser Leu Gln Val Met Asn
    50                  55                  60

```
Lys Thr Arg Lys Ile Met Glu His Gly Gly Ala Thr Phe Ile Asn Ala
65                  70                  75                  80

Phe Val Thr Thr Pro Met Cys Cys Pro Ser Arg Ser Ser Met Leu Thr
                85                  90                  95

Gly Lys Tyr Val His Asn His Asn Val Tyr Thr Asn Asn Glu Asn Cys
            100                 105                 110

Ser Ser Pro Ser Trp Gln Ala Met His Glu Pro Arg Thr Phe Ala Val
        115                 120                 125

Tyr Leu Asn Asn Thr Gly Tyr Arg Thr Ala Phe Phe Gly Lys Tyr Leu
    130                 135                 140

Asn Glu Tyr Asn Gly Ser Tyr Ile Pro Pro Gly Trp Arg Glu Trp Leu
145                 150                 155                 160

Gly Leu Ile Lys Asn Ser Arg Phe Tyr Asn Tyr Thr Val Cys Arg Asn
                165                 170                 175

Gly Ile Lys Glu Lys His Gly Phe Asp Tyr Ala Lys Asp Tyr Phe Thr
            180                 185                 190

Asp Leu Ile Thr Asn Glu Ser Ile Asn Tyr Phe Lys Met Ser Lys Arg
        195                 200                 205

Met Tyr Pro His Arg Pro Val Met Met Val Ile Ser His Ala Ala Pro
    210                 215                 220

His Gly Pro Glu Asp Ser Ala Pro Gln Phe Ser Lys Leu Tyr Pro Asn
225                 230                 235                 240

Ala Ser Gln His Ile Thr Pro Ser Tyr Asn Tyr Ala Pro Asn Met Asp
                245                 250                 255

Lys His Trp Ile Met Gln Tyr Thr Gly Pro Met Leu Pro Ile His Met
            260                 265                 270

Glu Phe Thr Asn Ile Leu Gln Arg Lys Arg Leu Gln Thr Leu Met Ser
        275                 280                 285

Val Asp Asp Ser Val Glu Arg Leu Tyr Asn Met Leu Val Glu Thr Gly
    290                 295                 300

Glu Leu Glu Asn Thr Tyr Ile Ile Tyr Thr Ala Asp His Gly Tyr His
305                 310                 315                 320

Ile Gly Gln Phe Gly Leu Val Lys Gly Lys Ser Met Pro Tyr Asp Phe
                325                 330                 335
```

Asp Ile Arg Val Pro Phe Phe Ile Arg Gly Pro Ser Val Glu Pro Gly
340 345 350

Ser Ile Val Pro Gln Ile Val Leu Asn Ile Asp Leu Ala Pro Thr Ile
355 360 365

Leu Asp Ile Ala Gly Leu Asp Thr Pro Pro Asp Val Asp Gly Lys Ser
370 375 380

Val Leu Lys Leu Leu Asp Pro Glu Lys Pro Gly Asn Arg Phe Arg Thr
385 390 395 400

Asn Lys Lys Ala Lys Ile Trp Arg Asp Thr Phe Leu Val Glu Arg Gly
405 410 415

Lys Phe Leu Arg Lys Lys Glu Glu Ser Ser Lys Asn Ile Gln Gln Ser
420 425 430

Asn His Leu Pro Lys Tyr Glu Arg Val Lys Glu Leu Cys Gln Gln Ala
435 440 445

Arg Tyr Gln Thr Ala Cys Glu Gln Pro Gly Gln Lys Trp Gln Cys Ile
450 455 460

Glu Asp Thr Ser Gly Lys Leu Arg Ile His Lys Cys Lys Gly Pro Ser
465 470 475 480

Asp Leu Leu Thr Val Arg Gln Ser Thr Arg Asn Leu Tyr Ala Arg Gly
485 490 495

Phe His Asp Lys Asp Lys Glu Cys Ser Cys Arg Glu Ser Gly Tyr Arg
500 505 510

Ala Ser Arg Ser Gln Arg Lys Ser Gln Arg Gln Phe Leu Arg Asn Gln
515 520 525

Gly Thr Pro Lys Tyr Lys Pro Arg Phe Val His Thr Arg Gln Thr Arg
530 535 540

Ser Leu Ser Val Glu Phe Glu Gly Glu Ile Tyr Asp Ile Asn Leu Glu
545 550 555 560

Glu Glu Glu Glu Leu Gln Val Leu Gln Pro Arg Asn Ile Ala Lys Arg
565 570 575

His Asp Glu Gly His Lys Gly Pro Arg Asp Leu Gln Ala Ser Ser Gly
580 585 590

Gly Asn Arg Gly Arg Met Leu Ala Asp Ser Ser Asn Ala Val Gly Pro
595 600 605

```
Pro Thr Thr Val Arg Val Thr His Lys Cys Phe Ile Leu Pro Asn Asp
    610                 615                 620

Ser Ile His Cys Glu Arg Glu Leu Tyr Gln Ser Ala Arg Ala Trp Lys
625                 630                 635                 640

Asp His Lys Ala Tyr Ile Asp Lys Glu Ile Glu Ala Leu Gln Asp Lys
                645                 650                 655

Ile Lys Asn Leu Arg Glu Val Arg Gly His Leu Lys Arg Arg Lys Pro
            660                 665                 670

Glu Glu Cys Ser Cys Ser Lys Gln Ser Tyr Tyr Asn Lys Glu Lys Gly
        675                 680                 685

Val Lys Lys Gln Glu Lys Leu Lys Ser His Leu His Pro Phe Lys Glu
    690                 695                 700

Ala Ala Gln Glu Val Asp Ser Lys Leu Gln Leu Phe Lys Glu Asn Asn
705                 710                 715                 720

Arg Arg Arg Lys Lys Glu Arg Lys Glu Lys Arg Arg Gln Arg Lys Gly
                725                 730                 735

Glu Glu Cys Ser Leu Pro Gly Leu Thr Cys Phe Thr His Asp Asn Asn
            740                 745                 750

His Trp Gln Thr Ala Pro Phe Trp Asn Leu Gly Ser Phe Cys Ala Cys
        755                 760                 765

Thr Ser Ser Asn Asn Asn Thr Tyr Trp Cys Leu Arg Thr Val Asn Glu
    770                 775                 780

Thr His Asn Phe Leu Phe Cys Glu Phe Ala Thr Gly Phe Leu Glu Tyr
785                 790                 795                 800

Phe Asp Met Asn Thr Asp Pro Tyr Gln Leu Thr Asn Thr Val His Thr
                805                 810                 815

Val Glu Arg Gly Ile Leu Asn Gln Leu His Val Gln Leu Met Glu Leu
            820                 825                 830

Arg Ser Cys Gln Gly Tyr Lys Gln Cys Asn Pro Arg Pro Lys Asn Leu
        835                 840                 845

Asp Val Gly Asn Lys Asp Gly Gly Ser Tyr Asp Leu His Arg Gly Gln
    850                 855                 860

Leu Trp Asp Gly Trp Glu Gly
865                 870
```

<210> 27
<211> 1433
<212> DNA
<213> Homo sapiens

<220>
<221> IGFBP2
<222> (1)..(1433)
<223> NM000597

<400> 27
attcggggcg agggaggagg aagaagcgga ggaggcggct cccgctcgca gggccgtgca 60

cctgcccgcc cgcccgctcg ctcgctcgcc cgccgcgccg cgctgccgac cgccagcatg 120

ctgccgagag tgggctgccc cgcgctgccg ctgccgccgc cgccgctgct gccgctgctg 180

ccgctgctgc tgctgctact gggcgcgagt ggcggcggcg gcggggcgcg cgcggaggtg 240

ctgttccgct gcccgccctg cacacccgag cgcctggccg cctgcgggcc cccgccggtt 300

gcgccgcccg ccgcggtggc cgcagtggcc ggaggcgccc gcatgccatg cgcggagctc 360

gtccgggagc cgggctgcgg ctgctgctcg gtgtgcgccc ggctggaggg cgaggcgtgc 420

ggcgtctaca ccccgcgctg cggccagggg ctgcgctgct atccccaccc gggctccgag 480

ctgcccctgc aggcgctggt catgggcgag ggcacttgtg agaagcgccg ggacgccgag 540

tatggcgcca gcccggagca ggttgcagac aatggcgatg accactcaga aggaggcctg 600

gtggagaacc acgtggacag caccatgaac atgttgggcg ggggaggcag tgctggccgg 660

aagcccctca agtcgggtat gaaggagctg gccgtgttcc gggagaaggt cactgagcag 720

caccggcaga tgggcaaggg tggcaagcat caccttggcc tggaggagcc caagaagctg 780

cgaccacccc ctgccaggac tccctgccaa caggaactgg accaggtcct ggagcggatc 840

tccaccatgc gccttccgga tgagcggggc cctctggagc acctctactc cctgcacatc 900

cccaactgtg acaagcatgg cctgtacaac ctcaaacagt gcaagatgtc tctgaacggg 960

cagcgtgggg agtgctggtg tgtgaacccc aacaccggga agctgatcca gggagccccc 1020

accatccggg gggaccccga gtgtcatctc ttctacaatg agcagcagga ggcttgcggg 1080

gtgcacaccc agcggatgca gtagaccgca gccagccggt gcctggcgcc cctgcccccc 1140

gcccctctcc aaacaccggc agaaaacgga gagtgcttgg gtggtgggtg ctggaggatt 1200

ttccagttct gacacacgta tttatatttg gaaagagacc agcaccgagc tcggcacctc 1260

cccggcctct ctcttcccag ctgcagatgc cacacctgct ccttcttgct ttccccgggg 1320

gaggaagggg gttgtggtcg ggagctgggg gtacaggttt ggggaggggg aagagaaatt 1380

tttatttttg aacccctgtg tcccttttgc ataagattaa aggaaggaaa agt 1433

<210> 28
<211> 328
<212> PRT
<213> Homo sapiens

<220>
<221> IGFBP2
<222> (1)..(328)
<223> NP000588

<400> 28

```
Met Leu Pro Arg Val Gly Cys Pro Ala Leu Pro Leu Pro Pro Pro Pro
1               5                   10                  15

Leu Leu Pro Leu Leu Pro Leu Leu Leu Leu Leu Leu Gly Ala Ser Gly
            20                  25                  30

Gly Gly Gly Gly Ala Arg Ala Glu Val Leu Phe Arg Cys Pro Pro Cys
        35                  40                  45

Thr Pro Glu Arg Leu Ala Ala Cys Gly Pro Pro Pro Val Ala Pro Pro
    50                  55                  60

Ala Ala Val Ala Ala Val Ala Gly Gly Ala Arg Met Pro Cys Ala Glu
65                  70                  75                  80

Leu Val Arg Glu Pro Gly Cys Gly Cys Cys Ser Val Cys Ala Arg Leu
                85                  90                  95

Glu Gly Glu Ala Cys Gly Val Tyr Thr Pro Arg Cys Gly Gln Gly Leu
            100                 105                 110

Arg Cys Tyr Pro His Pro Gly Ser Glu Leu Pro Leu Gln Ala Leu Val
        115                 120                 125

Met Gly Glu Gly Thr Cys Glu Lys Arg Arg Asp Ala Glu Tyr Gly Ala
    130                 135                 140

Ser Pro Glu Gln Val Ala Asp Asn Gly Asp Asp His Ser Glu Gly Gly
145                 150                 155                 160

Leu Val Glu Asn His Val Asp Ser Thr Met Asn Met Leu Gly Gly Gly
                165                 170                 175

Gly Ser Ala Gly Arg Lys Pro Leu Lys Ser Gly Met Lys Glu Leu Ala
            180                 185                 190

Val Phe Arg Glu Lys Val Thr Glu Gln His Arg Gln Met Gly Lys Gly
        195                 200                 205

Gly Lys His His Leu Gly Leu Glu Glu Pro Lys Lys Leu Arg Pro Pro
    210                 215                 220

Pro Ala Arg Thr Pro Cys Gln Gln Glu Leu Asp Gln Val Leu Glu Arg
225                 230                 235                 240

Ile Ser Thr Met Arg Leu Pro Asp Glu Arg Gly Pro Leu Glu His Leu
```

```
                245                 250                 255

Tyr Ser Leu His Ile Pro Asn Cys Asp Lys His Gly Leu Tyr Asn Leu
            260                 265                 270

Lys Gln Cys Lys Met Ser Leu Asn Gly Gln Arg Gly Glu Cys Trp Cys
        275                 280                 285

Val Asn Pro Asn Thr Gly Lys Leu Ile Gln Gly Ala Pro Thr Ile Arg
    290                 295                 300

Gly Asp Pro Glu Cys His Leu Phe Tyr Asn Glu Gln Gln Glu Ala Cys
305                 310                 315                 320

Gly Val His Thr Gln Arg Met Gln
                325


<210>  29
<211>  627
<212>  DNA
<213>  Homo sapiens


<220>
<221>  Cystatin E/M
<222>  (1)..(627)
<223>  NM001323

<400>  29
gcggccgcaa gctcggcact cacggctctg agggctccga cggcactgac ggccatggcg      60

cgttcgaacc tcccgctggc gctgggcctg gccctggtcg cattctgcct cctggcgctg     120

ccacgcgacg cccgggcccg gccgcaggag cgcatggtcg gagaactccg ggacctgtcg     180

cccgacgacc cgcaggtgca gaaggcggcg caggcggccg tggccagcta caacatgggc     240

agcaacagca tctactactt ccgagacacg cacatcatca aggcgcagag ccagctggtg     300

gccggcatca agtacttcct gacgatggag atggggagca cagactgccg caagaccagg     360

gtcactggag accacgtcga cctcaccact tgcccctgg cagcaggggc gcagcaggag      420

aagctgcgct gtgactttga ggtccttgtg gttccctggc agaactcctc tcagctccta     480

aagcacaact gtgtgcagat gtgataagtc cccgagggcg aaggccattg ggtttggggc     540

catggtggag ggcacttcag gtccgtgggc cgtatctgtc acaataaatg gccagtgctg     600

cttcttgcaa aaaaaaaaaa aaaaaaa                                         627


<210>  30
<211>  149
<212>  PRT
<213>  Homo sapiens


<220>
<221>  Cystatin E/M
<222>  (1)..(149)
<223>  NP001314
```

<400> 30

Met Ala Arg Ser Asn Leu Pro Leu Ala Leu Gly Leu Ala Leu Val Ala
1               5                   10                  15

Phe Cys Leu Leu Ala Leu Pro Arg Asp Ala Arg Ala Arg Pro Gln Glu
            20                  25                  30

Arg Met Val Gly Glu Leu Arg Asp Leu Ser Pro Asp Asp Pro Gln Val
        35                  40                  45

Gln Lys Ala Ala Gln Ala Ala Val Ala Ser Tyr Asn Met Gly Ser Asn
    50                  55                  60

Ser Ile Tyr Tyr Phe Arg Asp Thr His Ile Ile Lys Ala Gln Ser Gln
65                  70                  75                  80

Leu Val Ala Gly Ile Lys Tyr Phe Leu Thr Met Glu Met Gly Ser Thr
                85                  90                  95

Asp Cys Arg Lys Thr Arg Val Thr Gly Asp His Val Asp Leu Thr Thr
            100                 105                 110

Cys Pro Leu Ala Ala Gly Ala Gln Gln Glu Lys Leu Arg Cys Asp Phe
        115                 120                 125

Glu Val Leu Val Val Pro Trp Gln Asn Ser Ser Gln Leu Leu Lys His
    130                 135                 140

Asn Cys Val Gln Met
145

<210> 31
<211> 11025
<212> DNA
<213> Homo sapiens

<220>
<221> Pregnancy-assoc plasma protein A
<222> (1)..(11025)
<223> NM002581

<400> 31
gagcatcttt tggggggagg gaattcagcg gatcagtctt aagaggagct ttttttgga 60

gcgagaaatc atataaaata aaatgaaata aaacaaggag gaaggcaacc agctgttagg 120

ggaaaaataa ggcagataaa ggagcgggga gagaaattaa ttgccaacca ggaggagttg 180

ggctgtattt ttcaaaggtg gggagagtgg agcacacacc ttgaggagga aagcgagaaa 240

gaaaagaaaa aagcaagtgg aaagggggc tcgcccaaga agggtgaaga agcgaagaaa 300

gtcgaggcgc cgaggctccc aaagctggca gctccgggtg gcggtgcagg ggcgaagggg 360

gggcgggggg aaccgtcgga catgcggctc tggagttggg tgctgcacct ggggctgctg 420

```
agcgccgcgc tgggctgcgg gctggccgag cgtccccgcc gggcccggag agacccgcgg     480
gccggccgac ccccgcgccc cgccgccggc ccggccacct gcgccacccg ggcggcccgc     540
ggccgccgcg cctcgccgcc gccgccgccg ccgccgggcg gtgcctggga agccgtgcgc     600
gtcccccggc ggcggcagca gcgggaggcg aggggcgcca ccgaggagcc gagcccgccg     660
agccgggcgc tctatttcag cgggcgaggc gagcagctgc gcctccgggc cgacctcgag     720
ctgccccggg acgcgttcac gctgcaagtg tggctgcgag cggagggggg ccagaggtct     780
ccggcagtga tcacagggct gtatgacaaa tgttcttata tctcacgtga ccgaggatgg     840
gtcgtgggca ttcacaccat cagtgaccaa gacaacaaag acccacgcta ctttttctcc     900
ttgaagacag accgagcccg gcaagtgacc accatcaatg cccaccgcag ctacctccca     960
ggccagtggg tatacctagc tgccacctat gatgggcagt tcatgaagct ctatgtgaat    1020
ggtgcccagg tggccacctc tggggaacaa gtgggtggca tattcagccc actgacccag    1080
aagtgcaaag tgctcatgtt agggggcagt gccctgaatc acaactaccg gggctacatc    1140
gagcacttca gtctgtggaa ggtggccagg actcagcggg agatactgtc tgacatggaa    1200
acccatggcg cccacactgc tctacctcag ctcctcctcc aggagaactg ggacaatgtg    1260
aagcatgcct ggtcccccat gaaggatggc agcagcccca aagtggaatt cagcaatgcc    1320
cacggctttc tgctggacac gagtctggag cctcctctgt gcggacagac attgtgtgac    1380
aacacagagg tcattgccag ctacaatcag ctctcaagtt tccgccagcc caaggtggtg    1440
cgctaccgcg tggtcaacct ctatgaagat gatcataaga acccgacggt gacgcgcgag    1500
caggtggact tccagcacca tcagctggct gaggccttca agcaatacaa catctcctgg    1560
gagctggacg tgctggaggt gagcaactcc tcccttcgcc gccgcctcat cctggccaac    1620
tgtgacatca gcaagattgg ggatgagaac tgtgaccccg agtgcaacca cacgctgacg    1680
ggccacgacg gcggggattg ccgccacctg cgccaccctg ccttcgtgaa gaagcagcac    1740
aacggggtgt gtgacatgga ctgcaactat gaacggttca actttgatgg tggagagtgc    1800
tgtgaccctg aaatcaccaa tgtcactcag acttgctttg accccgactc tccacacaga    1860
gcctacttgg atgttaatga gctgaagaac attcttaaat tggatggatc aacacatctc    1920
aatattttct ttgcaaaatc ctcagaggag gagttggcag gagtagcaac ttggccatgg    1980
gacaaggagg ccctgatgca cttaggtggc attgtcttga acccatcttt ctatggcatg    2040
cctgggcaca cccacaccat gatccatgag attggtcaca gcctgggcct ctatcacgtc    2100
ttccgaggca tctcagaaat ccagtcctgc agtgacccct gcatggagac agagccctcc    2160
ttcgagactg gagacctctg caatgatacc aacccagccc ctaaacacaa gtcctgtggt    2220
gacccagggc caggaaatga cacctgtggc tttcatagct tcttcaacac tccttacaac    2280
aacttcatga gctatgcaga tgacgactgt acggactcct tcacgcccaa tcaagtcgcc    2340
agaatgcact gttacctgga cctggtctac cagggctggc agccctccag gaaaccagcg    2400
cctgttgccc tcgcccccca agttctgggc cacacaacgg actctgtgac actggagtgg    2460
```

ttcccaccta tagatggcca tttctttgaa agagaattgg gatcagcatg tcatctttgc 2520
ctggaaggga gaatcctggt gcagtatgct tccaacgctt cctccccaat gccctgcagc 2580
ccatcaggac actggagccc tcgtgaagca gaaggtcatc ctgatgttga acagccctgt 2640
aagtccagtg tccgcacctg gagcccaaat tcagctgtca acccacacac ggttcctcca 2700
gcctgccctg agcctcaagg ctgctacctc gagctggagt tcctctaccc cttggtccct 2760
gagtctctga ccatttgggt gacctttgtc tccactgact gggactctag tggagctgtc 2820
aatgacatca aactgttggc tgtcagtggg aagaacatct ccctgggtcc tcagaatgtc 2880
ttctgtgatg tcccactgac catcagactc tgggacgtgg gcgaggaggt gtatggcatc 2940
caaatctaca cgctggatga gcacctggag atcgatgctg ccatgttgac ctccactgca 3000
gacaccccac tctgtctaca gtgtaagccc ctgaagtata aggtggtccg ggaccctcct 3060
ctccagatgg atgtggcctc catcctacat ctcaatagga aattcgtaga catggatcta 3120
aatcttggca gtgtgtacca gtattgggtc ataactattt caggaactga agagagtgag 3180
ccatcacctg ctgtcacata catccatgga agtgggtact gtggcgatgg cattatacaa 3240
aaagaccaag gtgaacaatg cgacgacatg aataagatca atggtgatgg ctgctccctt 3300
ttctgccgac aagaagtctc cttcaattgt attgatgaac ccagccggtg ctatttccat 3360
gatggtgatg gggtatgtga ggagtttgaa caaaaaacca gcattaagga ctgtggtgtc 3420
tacacgcccc agggattcct ggatcagtgg gcatccaatg cttcagtatc tcatcaagac 3480
cagcaatgcc caggctgggt catcatcgga cagccagcag catcccaggt gtgtcgaacc 3540
aaggtgatag atctcagtga aggcatttcc cagcatgcct ggtacccttg caccatcagc 3600
tacccatatt cccagctggc tcagaccact ttttggctcc gggcgtattt ttctcaacca 3660
atggttgccg cagctgtcat tgtccacctg gtgacggatg ggacatatta tggggaccaa 3720
aagcaggaga ccatcagcgt gcagctgctt gataccaaag atcagagcca cgatctaggc 3780
ctccatgtcc tgagctgcag gaacaatccc ctgattatcc ctgtggtcca tgacctcagc 3840
cagcccttct accacagcca ggcggtacgt gtgagcttca gttcgcccct ggtcgccatc 3900
tcgggggtgg ccctccgttc cttcgacaac tttgaccccg tcaccctgag cagctgccag 3960
agaggggaga cctacagccc tgccgagcag agctgcgtgc acttcgcatg tgagaaaact 4020
gactgtccag agctggctgt ggagaatgct tctctcaatt gctccagcag cgaccgctac 4080
cacggtgccc agtgtactgt gagctgccgg acaggctacg tgctccagat acggcgggat 4140
gatgagctga tcaagagcca gacgggaccc agcgtcacag tgacctgtac agagggcaag 4200
tggaataagc aggtggcctg tgagccagtc gactgcagca tcccagatca ccatcaagtc 4260
tatgctgcct ccttctcctg ccctgagggc accacctttg gcagtcaatg ttccttccag 4320
tgccgtcacc ctgcacaatt gaaaggcaac aacagcctcc tgacctgcat ggaggatggg 4380
ctgtggtcct tcccagaggc cctgtgtgag ctcatgtgcc tcgctccacc ccctgtgccc 4440
aatgcagacc tccagaccgc ccggtgccga gagaataagc acaaggtggg ctccttctgc 4500

aaatacaaat gcaagcctgg ataccatgtg cctggatcct ctcggaagtc aaagaaacgg 4560
gccttcaaga ctcagtgtac ccaggatggc agctggcagg agggagcttg tgttcctgtg 4620
acctgtgacc cacctccacc aaaattccat gggctctacc agtgtactaa tggcttccag 4680
ttcaacagtg agtgtaggat caagtgtgaa gacagtgatg cctcccaggg acttgggagc 4740
aatgtcattc attgccggaa agatggcacc tggaacggct ccttccatgt ctgccaggag 4800
atgcaaggcc agtgctcggt tccaaacgag ctcaacagca acctcaaact gcagtgccct 4860
gatggctatg ccatagggtc ggagtgtgcc acctcgtgcc tggaccacaa cagcgagtcc 4920
atcatcctgc caatgaacgt gaccgtgcgt gacatccccc actggctgaa ccccacacgg 4980
gtagagagag ttgtctgcac tgctggtctc aagtggtatc ctcaccctgc tctgattcac 5040
tgtgtcaaag gctgtgagcc cttcatggga gacaattatt gtgatgccat caacaaccga 5100
gccttttgca actatgacgg tggggattgc tgcacctcca cagtgaagac caaaaaggtc 5160
accccattcc ctatgtcctg tgatctacaa ggtgactgtg cttgtcggga cccccaggcc 5220
caagaacaca gccggaaaga cctccgggga tacagccatg gctaaggaag gacaagaagt 5280
tgtcaaagaa ttcccaacgc caggacccac atccctttgg tattgatttc acagtcagct 5340
gctcaacgga atggcctctc cacaccaggg atccttagca cccaaccggt ctgcctttaa 5400
ttttacccag gaaggactca cattggggcg aatgaaccaa gtttcgccat gctggatgat 5460
gaaatggatt cccatcccaa agtctgagat ggattgcata tacagtgtgc agtcccagag 5520
cctcctaaaa ttctagccat ttgtcacaca accacagcaa gaaacgtgtt ctatatctag 5580
agtgtgccca tctgtgttta gtacacatgc atgcatacac acccatacaa acatctgtgt 5640
gagggcagtt ctggagatga gcagagagag accggaataa actcaatctt ttctttccca 5700
agctcctagc caacactatc cttgggagaa agaaatttgc agaaactgct aagaccaagt 5760
gtggagatgt caagctagtt cacactctga ggctcagaat atgtaggaca tgcacaattg 5820
tgcagtcctt tgggattgga agtgaaacag tctgtgatcc cctaccttct agggaactag 5880
gacctaggaa gaggtaaaga ttatcaggta tgcaaagcgc cccaattctt ctgctgccat 5940
gggggatttt accccaactc cagggttcga ggccaatctg agaatggctt aggattgcaa 6000
tgtcaaggta ttatatcagc cccttgcttg aggcttgagg tcataatatc cctctaggac 6060
ttacctgttc cccagatct tgccttggga ccacatttgc tgctactttt cctgctgctc 6120
tatcctatac attgaataat ccaagatggt agaactaggt taggaaaaat tccacacaac 6180
caaacagtct gccttaaaag tgacccacat ttttccatag ctcctcactt tttagccctt 6240
ctgcaagaga aaaaccctca tgggtccaca tggtgagaag ttaagtttcc tgtaagtggg 6300
cctctcaccc tggaaaggag ttgagggaca tcagatgctg gaaccctcac tgaaagtcca 6360
gaatgtctaa gccagtgtta gattttgtaa acaagtggaa cagtgttaaa tttctatgat 6420
gttggagcca tccagagact actggaattg tcgagacttt tggattatta tccttatcct 6480
tatcctaatc ttcctagccc ttcaggctag agtaggcttc gatcctgaga accttgctgt 6540

tgctctgagg agatataatt ctgggagaaa gaatctttta taagaacagt acagattgtt 6600
ctcaagaggg ccatcagaag gaagccaaag agttcacagc ctcagcacca acaactcaac 6660
atggtcatca tgttttctat atggtttttc cagctagcag tactcccttc catacctgtg 6720
actgggcagt gcttttctct ctcccatgtc tagcctccaa aagttaagtg aaaattagtc 6780
aactgcacgt ggaagccccc accactttgg ggatctcttt atttcttttc agccagggac 6840
ctgtccactc cctttgaatt aatatgggaa gaaattaata caggatgaac tggagagaag 6900
ggttgagtgt ggcatacttt ctgaaacctg gagctgggaa ttgcggagaa gggaaggtct 6960
agactagtta catcacatag ggattactgt aaatcaagtc atctcaagtc tagtgaagac 7020
agccaacaga aacaaaacct agcataggga tagaaaatac catgcacgtg tgcagcccca 7080
cctaattcct gcatccaagg caggtgttgt taatctatca tagcacttaa aaaaaaaaaa 7140
aaaaagagac caaaaataac tttaggaacc accatattat atcactccca atagcactga 7200
cctggtgatc aaaaacactt gagaagacat ctattggcca tctctggcca attacactaa 7260
gaaacatatc aaggtgcttt tggcacaggt gcccacaaat acggatgcag tgctgagata 7320
gtttatgaga cttgtaccat ttcacaaact ctgaaattgg gttccatatt ggcaaggctg 7380
ccacagttgt taagaataat cctctatgtt tcttcctcac aaaaccatat ctcatttata 7440
tccagaccat tacttcacta taattacaag gacaaattat tagcaagaaa taagaatagt 7500
attagaagaa ttgatcctat tttgaacccc tctccagtat cttcacactc ttgtcaactc 7560
tccaggcctc tctcttgccc tgagttatca gcctgtgtgg tgttaactac cttagaaggt 7620
acaagctaag aaatgtaaca gtatcaaccc tcccagttgc ttaattatac ccataggtaa 7680
tacaaaaagc tctgaagacc caaagatgac attactaatg atgtgatttc aggagccaca 7740
gaagaacctt accagcttcc ctcaaatcag tccttatcct ctttctatct tcactcccat 7800
catcatctat tttcacacta tccagctaag caaagattcc tggaggctga cttgtatctt 7860
cagactcaca gagtgaattc agctcttctg aatcaagacc cacccagtct ctttcattca 7920
gacctgttgc taacaaattt atatttgcca aggatattag gcaaaagagg ctacttgatt 7980
ggtggccaac ctcgtgccca catggaaggt atctttaata gggtcttttc aaaccttagt 8040
ggaggagggt cagctcaatt tgggcaatgc atttgttccc agtttcattt tcttcctggg 8100
aattaactcg tcatttcatt ccttcagtca tcttctgtgt aggtgaccgg agcactgaga 8160
ggcagctctg atgcactatt gtgtgtcagc agctcaaagg ccctaaaaca ctgaaggttc 8220
tgcatctgaa gtattagatt gttagcagca aaatatgaaa gatgaggtgg acagtcctct 8280
aagccctatt tagggaagct tttccaagcc acaatcttaa ctacctaccc aaaggatttg 8340
cattaccccc agattctgtg ccaacaacct tttaaggaaa tacagtcctt gggaaatgag 8400
ttttgatggt gaattggggt gttaaggaag ggaaagattg tcatagatgg tagggctttg 8460
aaaatgcagg gtatcagctg ccactcctgg cttcaacaca ttgagtcact gcctagacgg 8520
ttctcttggt cttattccca tcctggccaa tgcttaaata ctatttgttg aaaataattc 8580

tttgagacag atttcagcta cctcccttcc aggttcgatt taacttggtt gtaattgtca 8640
atttgttgtt ataggtctta cctgtgtgaa agaaagaaaa agaaagaaag aaagaaagag 8700
aaaggaaatt ataaggtcaa gttaacagtt ttgaggtttt gtgttttttt ctggaactac 8760
ttcaagtgag aaaataaaaa aaaatggtga caaagctgta cagatagaga taatagaaga 8820
caaagagatt aaaaggaaat aaaaatgcat gattaaaaac taagaataaa aaacctattt 8880
ttatgtttcc taaaggaaat tgtttattct acagcctcag taggtagaca caaacataaa 8940
gatttcccta gaagacatag agtgggattt gataacactg tctgttattt tctgtacatt 9000
gtggtaggtc caggaaatat gacattttcc cccttgatgt gttattgttg ttgttgggtg 9060
gggtgggcat tttgtttatt tgtttggtgg caatcagtgg tagtagggag tgggagggct 9120
tatattggtt tttccagcta ttaaggggac atattgtgtc gttgtgcttt tcacgttata 9180
aaatgtttat atttaccagt acagcactgg gctttataaa gactgcactc agaaccacac 9240
tgcacagtcc agttttttaa aaagctgcta catgacagac aggtaatccc actgagtgag 9300
ttttgagaaa caaatcaaac gaagtaaaca agaaacataa aaaccaaata gcaaatgaat 9360
aaaagcctgt tcttgtaact tattcaactt ttgccaaatt cctaccaatc acttgctttt 9420
taaaagaaat gtataatagc caaaagagaa attatgtccc tgttgtacag aagttagaat 9480
ttttgactcc aggcagcagt ttgctcagtg atcttgaaca agttatccaa ttgcctctac 9540
atttgcatca gtttctctag ctgcaaaatg gggataatac tatataccta cctcacagtg 9600
ggagggcagg agattttgag gccctgaggt tttaggtggg ctgtgagggc caacgcttga 9660
cacaaagtcc atgggttatt attcaagaat gcacaggccc atcggccttt tagaaagaca 9720
agacagggag tgcttgtttg atatttcaag gaataaagcc ggagctcctg aattgtagtc 9780
caccttaaaa gagagacctg tattggagaa tattttattt ttttggcaaa tttgatctta 9840
ccctttacca gttctataat ttggttaaaa gctgattatg tcctacaatg tcaaagtcag 9900
ctaactgtcg tctacttaag acttctggtc atttccaact tatagaggaa gggagtctct 9960
aaaatctctt cttcagaagg cacctcactt ctcagactta aaattccaca tcaagtgttc 10020
cattaaaaga agataaggca ttctgagtgc aaacaaatgg gggcttctta aactacacac 10080
cagcagtcag tgaggaaaac tttgaacaat tattgagttg ctttcttggg tctctataat 10140
caataacctg tctgcagata tctatctata taaagatatt atatataaat ataaatttac 10200
atatatatgc acatgtatat atagttgtac atatatgtgt gtatatatat acttaaatgt 10260
aatatttaca aaataaaact gtgatctcgt ctagagaaaa tgtattcata ttacaaactg 10320
ctcttccata tttatgtacc atattatacc tttttattat tgttataatt attatgggta 10380
tttctaatta atatgatgtt gaaacctgtt tggcaccttc tggaagctac caaaaaaatg 10440
acactccatt gaagtgctta aaagctgttc tcataagaat tctactggcc tattgtaaaa 10500
aagaaaaaaa aaaagaaaaa gaagaaagac acaaagaaaa taatctaaac accaaaaact 10560
aaacacaatt ccaatccttt ttctgtacct cacgcgcata aatttgctgc tcctattttt 10620

```
ttttctgttt atgtgttttt atggatctaa gttaaatctt ttggcaatat ataaaaatgt    10680
aaatagtaaa ctttatttat taagaatgtc atcttttttta atttatattt acacaattgt    10740
tcatctaatt tattttttct atacagtttt aaatactcag acatattttg ctgttcatga    10800
tatttttatc ctgttctcat ggatttgttt tcccatactg ttttctctga tctcaattac    10860
aggttggatc tcacaaataa taatgtcaga gacagaaata ttttgccact gttgattact    10920
atactttaaa gttctatatt atgaaaatat ataatagctt gtacgcttca aaaaaaaaaa    10980
aaaaaaaaaa aaaaaaaaaa aaaaaaaaaa aaaaaaaaaa aaaaa                    11025
```

<210> 32
<211> 1627
<212> PRT
<213> Homo sapiens

<220>
<221> Pregnancy-assoc plasma protein A
<222> (1)..(1627)
<223> NP002572

<400> 32

```
Met Arg Leu Trp Ser Trp Val Leu His Leu Gly Leu Leu Ser Ala Ala
1               5                   10                  15

Leu Gly Cys Gly Leu Ala Glu Arg Pro Arg Arg Ala Arg Arg Asp Pro
            20                  25                  30

Arg Ala Gly Arg Pro Pro Arg Pro Ala Ala Gly Pro Ala Thr Cys Ala
        35                  40                  45

Thr Arg Ala Ala Arg Gly Arg Arg Ala Ser Pro Pro Pro Pro Pro Pro
    50                  55                  60

Pro Gly Gly Ala Trp Glu Ala Val Arg Val Pro Arg Arg Arg Gln Gln
65                  70                  75                  80

Arg Glu Ala Arg Gly Ala Thr Glu Glu Pro Ser Pro Pro Ser Arg Ala
                85                  90                  95

Leu Tyr Phe Ser Gly Arg Gly Glu Gln Leu Arg Leu Arg Ala Asp Leu
            100                 105                 110

Glu Leu Pro Arg Asp Ala Phe Thr Leu Gln Val Trp Leu Arg Ala Glu
        115                 120                 125

Gly Gly Gln Arg Ser Pro Ala Val Ile Thr Gly Leu Tyr Asp Lys Cys
    130                 135                 140

Ser Tyr Ile Ser Arg Asp Arg Gly Trp Val Val Gly Ile His Thr Ile
145                 150                 155                 160
```

```
Ser Asp Gln Asp Asn Lys Asp Pro Arg Tyr Phe Phe Ser Leu Lys Thr
                165                 170                 175

Asp Arg Ala Arg Gln Val Thr Thr Ile Asn Ala His Arg Ser Tyr Leu
            180                 185                 190

Pro Gly Gln Trp Val Tyr Leu Ala Ala Thr Tyr Asp Gly Gln Phe Met
        195                 200                 205

Lys Leu Tyr Val Asn Gly Ala Gln Val Ala Thr Ser Gly Glu Gln Val
    210                 215                 220

Gly Gly Ile Phe Ser Pro Leu Thr Gln Lys Cys Lys Val Leu Met Leu
225                 230                 235                 240

Gly Gly Ser Ala Leu Asn His Asn Tyr Arg Gly Tyr Ile Glu His Phe
                245                 250                 255

Ser Leu Trp Lys Val Ala Arg Thr Gln Arg Glu Ile Leu Ser Asp Met
            260                 265                 270

Glu Thr His Gly Ala His Thr Ala Leu Pro Gln Leu Leu Leu Gln Glu
        275                 280                 285

Asn Trp Asp Asn Val Lys His Ala Trp Ser Pro Met Lys Asp Gly Ser
    290                 295                 300

Ser Pro Lys Val Glu Phe Ser Asn Ala His Gly Phe Leu Leu Asp Thr
305                 310                 315                 320

Ser Leu Glu Pro Pro Leu Cys Gly Gln Thr Leu Cys Asp Asn Thr Glu
                325                 330                 335

Val Ile Ala Ser Tyr Asn Gln Leu Ser Ser Phe Arg Gln Pro Lys Val
            340                 345                 350

Val Arg Tyr Arg Val Val Asn Leu Tyr Glu Asp Asp His Lys Asn Pro
        355                 360                 365

Thr Val Thr Arg Glu Gln Val Asp Phe Gln His His Gln Leu Ala Glu
    370                 375                 380

Ala Phe Lys Gln Tyr Asn Ile Ser Trp Glu Leu Asp Val Leu Glu Val
385                 390                 395                 400

Ser Asn Ser Ser Leu Arg Arg Arg Leu Ile Leu Ala Asn Cys Asp Ile
                405                 410                 415

Ser Lys Ile Gly Asp Glu Asn Cys Asp Pro Glu Cys Asn His Thr Leu
            420                 425                 430
```

```
Thr Gly His Asp Gly Gly Asp Cys Arg His Leu Arg His Pro Ala Phe
        435                 440                 445

Val Lys Lys Gln His Asn Gly Val Cys Asp Met Asp Cys Asn Tyr Glu
    450                 455                 460

Arg Phe Asn Phe Asp Gly Gly Glu Cys Cys Asp Pro Glu Ile Thr Asn
465                 470                 475                 480

Val Thr Gln Thr Cys Phe Asp Pro Asp Ser Pro His Arg Ala Tyr Leu
                485                 490                 495

Asp Val Asn Glu Leu Lys Asn Ile Leu Lys Leu Asp Gly Ser Thr His
            500                 505                 510

Leu Asn Ile Phe Phe Ala Lys Ser Ser Glu Glu Glu Leu Ala Gly Val
        515                 520                 525

Ala Thr Trp Pro Trp Asp Lys Glu Ala Leu Met His Leu Gly Gly Ile
    530                 535                 540

Val Leu Asn Pro Ser Phe Tyr Gly Met Pro Gly His Thr His Thr Met
545                 550                 555                 560

Ile His Glu Ile Gly His Ser Leu Gly Leu Tyr His Val Phe Arg Gly
                565                 570                 575

Ile Ser Glu Ile Gln Ser Cys Ser Asp Pro Cys Met Glu Thr Glu Pro
            580                 585                 590

Ser Phe Glu Thr Gly Asp Leu Cys Asn Asp Thr Asn Pro Ala Pro Lys
        595                 600                 605

His Lys Ser Cys Gly Asp Pro Gly Pro Gly Asn Asp Thr Cys Gly Phe
    610                 615                 620

His Ser Phe Phe Asn Thr Pro Tyr Asn Asn Phe Met Ser Tyr Ala Asp
625                 630                 635                 640

Asp Asp Cys Thr Asp Ser Phe Thr Pro Asn Gln Val Ala Arg Met His
                645                 650                 655

Cys Tyr Leu Asp Leu Val Tyr Gln Gly Trp Gln Pro Ser Arg Lys Pro
            660                 665                 670

Ala Pro Val Ala Leu Ala Pro Gln Val Leu Gly His Thr Thr Asp Ser
        675                 680                 685

Val Thr Leu Glu Trp Phe Pro Pro Ile Asp Gly His Phe Phe Glu Arg
    690                 695                 700
```

Glu Leu Gly Ser Ala Cys His Leu Cys Leu Glu Gly Arg Ile Leu Val
705 710 715 720

Gln Tyr Ala Ser Asn Ala Ser Ser Pro Met Pro Cys Ser Pro Ser Gly
725 730 735

His Trp Ser Pro Arg Glu Ala Glu Gly His Pro Asp Val Glu Gln Pro
740 745 750

Cys Lys Ser Ser Val Arg Thr Trp Ser Pro Asn Ser Ala Val Asn Pro
755 760 765

His Thr Val Pro Pro Ala Cys Pro Glu Pro Gln Gly Cys Tyr Leu Glu
770 775 780

Leu Glu Phe Leu Tyr Pro Leu Val Pro Glu Ser Leu Thr Ile Trp Val
785 790 795 800

Thr Phe Val Ser Thr Asp Trp Asp Ser Ser Gly Ala Val Asn Asp Ile
805 810 815

Lys Leu Leu Ala Val Ser Gly Lys Asn Ile Ser Leu Gly Pro Gln Asn
820 825 830

Val Phe Cys Asp Val Pro Leu Thr Ile Arg Leu Trp Asp Val Gly Glu
835 840 845

Glu Val Tyr Gly Ile Gln Ile Tyr Thr Leu Asp Glu His Leu Glu Ile
850 855 860

Asp Ala Ala Met Leu Thr Ser Thr Ala Asp Thr Pro Leu Cys Leu Gln
865 870 875 880

Cys Lys Pro Leu Lys Tyr Lys Val Val Arg Asp Pro Pro Leu Gln Met
885 890 895

Asp Val Ala Ser Ile Leu His Leu Asn Arg Lys Phe Val Asp Met Asp
900 905 910

Leu Asn Leu Gly Ser Val Tyr Gln Tyr Trp Val Ile Thr Ile Ser Gly
915 920 925

Thr Glu Glu Ser Glu Pro Ser Pro Ala Val Thr Tyr Ile His Gly Ser
930 935 940

Gly Tyr Cys Gly Asp Gly Ile Ile Gln Lys Asp Gln Gly Glu Gln Cys
945 950 955 960

Asp Asp Met Asn Lys Ile Asn Gly Asp Gly Cys Ser Leu Phe Cys Arg
965 970 975

```
Gln Glu Val Ser Phe Asn Cys Ile Asp Glu Pro Ser Arg Cys Tyr Phe
            980                 985                 990

His Asp Gly Asp Gly Val Cys Glu  Glu Phe Glu Gln Lys  Thr Ser Ile
        995                 1000                 1005

Lys Asp  Cys Gly Val Tyr Thr  Pro Gln Gly Phe Leu  Asp Gln Trp
    1010                 1015                 1020

Ala Ser  Asn Ala Ser Val Ser  His Gln Asp Gln Gln  Cys Pro Gly
    1025                 1030                 1035

Trp Val  Ile Ile Gly Gln Pro  Ala Ala Ser Gln Val  Cys Arg Thr
    1040                 1045                 1050

Lys Val  Ile Asp Leu Ser Glu  Gly Ile Ser Gln His  Ala Trp Tyr
    1055                 1060                 1065

Pro Cys  Thr Ile Ser Tyr Pro  Tyr Ser Gln Leu Ala  Gln Thr Thr
    1070                 1075                 1080

Phe Trp  Leu Arg Ala Tyr Phe  Ser Gln Pro Met Val  Ala Ala Ala
    1085                 1090                 1095

Val Ile  Val His Leu Val Thr  Asp Gly Thr Tyr Tyr  Gly Asp Gln
    1100                 1105                 1110

Lys Gln  Glu Thr Ile Ser Val  Gln Leu Leu Asp Thr  Lys Asp Gln
    1115                 1120                 1125

Ser His  Asp Leu Gly Leu His  Val Leu Ser Cys Arg  Asn Asn Pro
    1130                 1135                 1140

Leu Ile  Ile Pro Val Val His  Asp Leu Ser Gln Pro  Phe Tyr His
    1145                 1150                 1155

Ser Gln  Ala Val Arg Val Ser  Phe Ser Ser Pro Leu  Val Ala Ile
    1160                 1165                 1170

Ser Gly  Val Ala Leu Arg Ser  Phe Asp Asn Phe Asp  Pro Val Thr
    1175                 1180                 1185

Leu Ser  Ser Cys Gln Arg Gly  Glu Thr Tyr Ser Pro  Ala Glu Gln
    1190                 1195                 1200

Ser Cys  Val His Phe Ala Cys  Glu Lys Thr Asp Cys  Pro Glu Leu
    1205                 1210                 1215

Ala Val  Glu Asn Ala Ser Leu  Asn Cys Ser Ser Ser  Asp Arg Tyr
    1220                 1225                 1230
```

```
His Gly  Ala Gln Cys Thr Val  Ser Cys Arg Thr Gly  Tyr Val Leu
    1235                 1240                 1245

Gln Ile  Arg Arg Asp Asp Glu  Leu Ile Lys Ser Gln  Thr Gly Pro
    1250                 1255                 1260

Ser Val  Thr Val Thr Cys Thr  Glu Gly Lys Trp Asn  Lys Gln Val
    1265                 1270                 1275

Ala Cys  Glu Pro Val Asp Cys  Ser Ile Pro Asp His  His Gln Val
    1280                 1285                 1290

Tyr Ala  Ala Ser Phe Ser Cys  Pro Glu Gly Thr Thr  Phe Gly Ser
    1295                 1300                 1305

Gln Cys  Ser Phe Gln Cys Arg  His Pro Ala Gln Leu  Lys Gly Asn
    1310                 1315                 1320

Asn Ser  Leu Leu Thr Cys Met  Glu Asp Gly Leu Trp  Ser Phe Pro
    1325                 1330                 1335

Glu Ala  Leu Cys Glu Leu Met  Cys Leu Ala Pro Pro  Pro Val Pro
    1340                 1345                 1350

Asn Ala  Asp Leu Gln Thr Ala  Arg Cys Arg Glu Asn  Lys His Lys
    1355                 1360                 1365

Val Gly  Ser Phe Cys Lys Tyr  Lys Cys Lys Pro Gly  Tyr His Val
    1370                 1375                 1380

Pro Gly  Ser Ser Arg Lys Ser  Lys Lys Arg Ala Phe  Lys Thr Gln
    1385                 1390                 1395

Cys Thr  Gln Asp Gly Ser Trp  Gln Glu Gly Ala Cys  Val Pro Val
    1400                 1405                 1410

Thr Cys  Asp Pro Pro Pro Pro  Lys Phe His Gly Leu  Tyr Gln Cys
    1415                 1420                 1425

Thr Asn  Gly Phe Gln Phe Asn  Ser Glu Cys Arg Ile  Lys Cys Glu
    1430                 1435                 1440

Asp Ser  Asp Ala Ser Gln Gly  Leu Gly Ser Asn Val  Ile His Cys
    1445                 1450                 1455

Arg Lys  Asp Gly Thr Trp Asn  Gly Ser Phe His Val  Cys Gln Glu
    1460                 1465                 1470

Met Gln  Gly Gln Cys Ser Val  Pro Asn Glu Leu Asn  Ser Asn Leu
    1475                 1480                 1485
```

```
Lys Leu  Gln Cys Pro Asp Gly  Tyr Ala Ile Gly Ser  Glu Cys Ala
    1490                 1495                 1500

Thr Ser  Cys Leu Asp His Asn  Ser Glu Ser Ile Ile  Leu Pro Met
    1505                 1510                 1515

Asn Val  Thr Val Arg Asp Ile  Pro His Trp Leu Asn  Pro Thr Arg
    1520                 1525                 1530

Val Glu  Arg Val Val Cys Thr  Ala Gly Leu Lys Trp  Tyr Pro His
    1535                 1540                 1545

Pro Ala  Leu Ile His Cys Val  Lys Gly Cys Glu Pro  Phe Met Gly
    1550                 1555                 1560

Asp Asn  Tyr Cys Asp Ala Ile  Asn Asn Arg Ala Phe  Cys Asn Tyr
    1565                 1570                 1575

Asp Gly  Gly Asp Cys Cys Thr  Ser Thr Val Lys Thr  Lys Lys Val
    1580                 1585                 1590

Thr Pro  Phe Pro Met Ser Cys  Asp Leu Gln Gly Asp  Cys Ala Cys
    1595                 1600                 1605

Arg Asp  Pro Gln Ala Gln Glu  His Ser Arg Lys Asp  Leu Arg Gly
    1610                 1615                 1620

Tyr Ser  His Gly
    1625

<210>  33
<211>  2444
<212>  DNA
<213>  Homo sapiens

<220>
<221>  Butyrlcholinesterase
<222>  (1)..(2444)
<223>  NM000055

<400>  33
agtaacagtt gattgttaca ttcagtaaca ctgaatgtca gtgcagtcca atttacaggc      60

tggagcagca gctgcatcct gcatttcccc gaagtattac atgattttca ctccttgcaa     120

actttaccat ctttgttgca gagaatcgga aatcaatatg catagcaaag tcacaatcat     180

atgcatcaga tttctctttt ggtttctttt gctctgcatg cttattggga agtcacatac     240

tgaagatgac atcataattg caacaaagaa tggaaaagtc agagggatga acttgacagt     300

tttggtggc acggtaacag cctttcttgg aattccctat gcacagccac ctcttggtag      360

acttcgattc aaaaagccac agtctctgac caagtggtct gatatttgga atgccacaaa     420

atatgcaaat tcttgctgtc agaacataga tcaaagtttt ccaggcttcc atggatcaga     480
```

```
gatgtggaac ccaaacactg acctcagtga agactgttta tatctaaatg tatggattcc     540
agcacctaaa ccaaaaaatg ccactgtatt gatatggatt tatggtggtg gttttcaaac     600
tggaacatca tctttacatg tttatgatgg caagtttctg gctcgggttg aaagagttat     660
tgtagtgtca atgaactata gggtgggtgc cctaggattc ttagctttgc caggaaatcc     720
tgaggctcca gggaacatgg gtttatttga tcaacagttg gctcttcagt gggttcaaaa     780
aaatatagca gcctttggtg gaaatcctaa aagtgtaact ctctttggag aaagtgcagg     840
agcagcttca gttagcctgc atttgctttc tcctggaagc cattcattgt tcaccagagc     900
cattctgcaa agtggatcct ttaatgctcc ttgggcggta acatctcttt atgaagctag     960
gaacagaacg ttgaacttag ctaaattgac tggttgctct agagagaatg agactgaaat    1020
aatcaagtgt cttagaaata aagatcccca agaaattctt ctgaatgaag catttgttgt    1080
cccctatggg actcctttgt cagtaaactt tggtccgacc gtggatggtg attttctcac    1140
tgacatgcca gacatattac ttgaacttgg acaatttaaa aaaacccaga ttttggtggg    1200
tgttaataaa gatgaaggga cagctttttt agtctatggt gctcctggct tcagcaaaga    1260
taacaatagt atcataacta gaaaagaatt tcaggaaggt ttaaaaatat tttttccagg    1320
agtgagtgag tttggaaagg aatccatcct tttcattac acagactggg tagatgatca    1380
gagacctgaa aactaccgtg aggccttggg tgatgttgtt ggggattata atttcatatg    1440
ccctgccttg gagttcacca agaagttctc agaatgggga aataatgcct ttttctacta    1500
ttttgaacac cgatcctcca aacttccgtg gccagaatgg atgggagtga tgcatggcta    1560
tgaaattgaa tttgtctttg gtttacctct ggaaagaaga gataattaca caaaagccga    1620
ggaaattttg agtagatcca tagtgaaacg gtgggcaaat tttgcaaaat atgggaatcc    1680
aaatgagact cagaacaata gcacaagctg gcctgtcttc aaaagcactg aacaaaaata    1740
tctaaccttg aatacagagt caacaagaat aatgacgaaa ctacgtgctc aacaatgtcg    1800
attctggaca tcattttttc caaaagtctt ggaaatgaca ggaaatattg atgaagcaga    1860
atgggagtgg aaagcaggat tccatcgctg gaacaattac atgatggact ggaaaaatca    1920
atttaacgat tacactagca agaaagaaag ttgtgtgggt ctctaattaa tagatttacc    1980
ctttatagaa catattttcc tttagatcaa ggcaaaaata tcaggagctt ttttacacac    2040
ctactaaaaa agttattatg tagctgaaac aaaaatgcca gaaggataat attgattcct    2100
cacatcttta acttagtatt ttacctagca tttcaaaacc caaatggcta gaacatgttt    2160
aattaaattt cacaatataa agttctacag ttaattatgt gcatattaaa acaatggcct    2220
ggttcaattt ctttctttcc ttaataaatt taagtttttt ccccccaaaa ttatcagtgc    2280
tctgctttta gtcacgtgta ttttcattac cactcgtaaa aaggtatctt ttttaaatga    2340
attaaatatt gaaacactgt acaccatagt ttacaatatt atgtttccta attaaaataa    2400
gaattgaatg tcaatatgag atattaaaat aagcacagaa aatc                      2444
```

<210> 34
<211> 602
<212> PRT
<213> Homo sapiens

<220>
<221> Butyrlcholinesterase
<222> (1)..(602)
<223> NP000046

<400> 34

```
Met His Ser Lys Val Thr Ile Ile Cys Ile Arg Phe Leu Phe Trp Phe
1               5                   10                  15

Leu Leu Leu Cys Met Leu Ile Gly Lys Ser His Thr Glu Asp Asp Ile
            20                  25                  30

Ile Ile Ala Thr Lys Asn Gly Lys Val Arg Gly Met Asn Leu Thr Val
        35                  40                  45

Phe Gly Gly Thr Val Thr Ala Phe Leu Gly Ile Pro Tyr Ala Gln Pro
    50                  55                  60

Pro Leu Gly Arg Leu Arg Phe Lys Lys Pro Gln Ser Leu Thr Lys Trp
65                  70                  75                  80

Ser Asp Ile Trp Asn Ala Thr Lys Tyr Ala Asn Ser Cys Cys Gln Asn
                85                  90                  95

Ile Asp Gln Ser Phe Pro Gly Phe His Gly Ser Glu Met Trp Asn Pro
            100                 105                 110

Asn Thr Asp Leu Ser Glu Asp Cys Leu Tyr Leu Asn Val Trp Ile Pro
        115                 120                 125

Ala Pro Lys Pro Lys Asn Ala Thr Val Leu Ile Trp Ile Tyr Gly Gly
    130                 135                 140

Gly Phe Gln Thr Gly Thr Ser Ser Leu His Val Tyr Asp Gly Lys Phe
145                 150                 155                 160

Leu Ala Arg Val Glu Arg Val Ile Val Val Ser Met Asn Tyr Arg Val
                165                 170                 175

Gly Ala Leu Gly Phe Leu Ala Leu Pro Gly Asn Pro Glu Ala Pro Gly
            180                 185                 190

Asn Met Gly Leu Phe Asp Gln Gln Leu Ala Leu Gln Trp Val Gln Lys
        195                 200                 205

Asn Ile Ala Ala Phe Gly Gly Asn Pro Lys Ser Val Thr Leu Phe Gly
    210                 215                 220
```

Glu Ser Ala Gly Ala Ala Ser Val Ser Leu His Leu Leu Ser Pro Gly
225 230 235 240

Ser His Ser Leu Phe Thr Arg Ala Ile Leu Gln Ser Gly Ser Phe Asn
245 250 255

Ala Pro Trp Ala Val Thr Ser Leu Tyr Glu Ala Arg Asn Arg Thr Leu
260 265 270

Asn Leu Ala Lys Leu Thr Gly Cys Ser Arg Glu Asn Glu Thr Glu Ile
275 280 285

Ile Lys Cys Leu Arg Asn Lys Asp Pro Gln Glu Ile Leu Leu Asn Glu
290 295 300

Ala Phe Val Val Pro Tyr Gly Thr Pro Leu Ser Val Asn Phe Gly Pro
305 310 315 320

Thr Val Asp Gly Asp Phe Leu Thr Asp Met Pro Asp Ile Leu Leu Glu
325 330 335

Leu Gly Gln Phe Lys Lys Thr Gln Ile Leu Val Gly Val Asn Lys Asp
340 345 350

Glu Gly Thr Ala Phe Leu Val Tyr Gly Ala Pro Gly Phe Ser Lys Asp
355 360 365

Asn Asn Ser Ile Ile Thr Arg Lys Glu Phe Gln Glu Gly Leu Lys Ile
370 375 380

Phe Phe Pro Gly Val Ser Glu Phe Gly Lys Glu Ser Ile Leu Phe His
385 390 395 400

Tyr Thr Asp Trp Val Asp Asp Gln Arg Pro Glu Asn Tyr Arg Glu Ala
405 410 415

Leu Gly Asp Val Val Gly Asp Tyr Asn Phe Ile Cys Pro Ala Leu Glu
420 425 430

Phe Thr Lys Lys Phe Ser Glu Trp Gly Asn Asn Ala Phe Phe Tyr Tyr
435 440 445

Phe Glu His Arg Ser Ser Lys Leu Pro Trp Pro Glu Trp Met Gly Val
450 455 460

Met His Gly Tyr Glu Ile Glu Phe Val Phe Gly Leu Pro Leu Glu Arg
465 470 475 480

Arg Asp Asn Tyr Thr Lys Ala Glu Glu Ile Leu Ser Arg Ser Ile Val
485 490 495

```
Lys Arg Trp Ala Asn Phe Ala Lys Tyr Gly Asn Pro Asn Glu Thr Gln
            500                 505                 510

Asn Asn Ser Thr Ser Trp Pro Val Phe Lys Ser Thr Glu Gln Lys Tyr
        515                 520                 525

Leu Thr Leu Asn Thr Glu Ser Thr Arg Ile Met Thr Lys Leu Arg Ala
    530                 535                 540

Gln Gln Cys Arg Phe Trp Thr Ser Phe Phe Pro Lys Val Leu Glu Met
545                 550                 555                 560

Thr Gly Asn Ile Asp Glu Ala Glu Trp Glu Trp Lys Ala Gly Phe His
                565                 570                 575

Arg Trp Asn Asn Tyr Met Met Asp Trp Lys Asn Gln Phe Asn Asp Tyr
            580                 585                 590

Thr Ser Lys Lys Glu Ser Cys Val Gly Leu
        595                 600
```

<210> 35
<211> 1150
<212> DNA
<213> Homo sapiens

<220>
<221> Endothelial lectin HL-2
<222> (1)..(1150)
<223> NM080878

<400> 35

```
gcaggggagc tccgagtgtc cacaggaagg gaactatcag ctcctggcat ctgtaaggat      60
gctgtccatg ctgaggacaa tgaccagact ctgcttcctg ttattcttct ctgtggccac     120
cagtgggtgc agtgcagcag cagcctcttc tcttgagatg ctctcgaggg aattcgaaac     180
ctgtgccttc tccttttctt ccctgcctag aagctgcaaa gaaatcaagg aacgctgcca     240
tagtgcaggt gatggcctgt attttctccg caccaagaat ggtgttgtct accagacctt     300
ctgtgacatg acttctgggg gtggcggctg gaccctggtg gccagcgtgc acgagaatga     360
catgcgtggg aagtgcacgg tgggtgatcg ctggtccagt cagcagggca acaaagcaga     420
ctacccagag gggggatggca actgggccaa ctacaacacc tttggatctg cagaggcggc     480
cacgagcgat gactacaaga accctggcta ctacgacatc caggccaagg acctgggcat     540
ctggcatgtg cccaacaagt cccccatgca gcattggaga aacagcgccc tgctgaggta     600
ccgcaccaac actggcttcc tccagagact gggacataat ctgtttggca tctaccagaa     660
atacccagtg aaatacagat cagggaaatg ttggaatgac aatggcccag ccatacctgt     720
ggtctatgac tttggtgatg ctaagaagac tgcatcttat tactcaccgt atggtcaacg     780
ggaatttgtt gcaggattcg ttcagttccg ggtgtttaat aacgagagag cagccaacgc     840
```

```
cctttgtgct gggataaaag ttactggctg taacactgag catcactgca tcggtggagg      900
agggttcttc ccacagggca aaccccgtca gtgtggggac ttctccgcct ttgactggga      960
tggatatgga actcacgtta agagcagctg cagtcgggag ataacggagg cggctgtact     1020
cttgttctat agatgagaca gagctctgcg gtgtcagggc gagaacccat cttccaaccc     1080
cggctatttg gagacggaaa aactggaatt ctaacaagga ggagaggaga ctaaatcaca     1140
tcaatttgca                                                            1150

<210>  36
<211>  325
<212>  PRT
<213>  Homo sapiens

<220>
<221>  Endothelial lectin HL-2
<222>  (1)..(325)
<223>  NP543154

<400>  36

Met Leu Ser Met Leu Arg Thr Met Thr Arg Leu Cys Phe Leu Leu Phe
1               5                   10                  15

Phe Ser Val Ala Thr Ser Gly Cys Ser Ala Ala Ala Ala Ser Ser Leu
            20                  25                  30

Glu Met Leu Ser Arg Glu Phe Glu Thr Cys Ala Phe Ser Phe Ser Ser
        35                  40                  45

Leu Pro Arg Ser Cys Lys Glu Ile Lys Glu Arg Cys His Ser Ala Gly
    50                  55                  60

Asp Gly Leu Tyr Phe Leu Arg Thr Lys Asn Gly Val Val Tyr Gln Thr
65                  70                  75                  80

Phe Cys Asp Met Thr Ser Gly Gly Gly Gly Trp Thr Leu Val Ala Ser
                85                  90                  95

Val His Glu Asn Asp Met Arg Gly Lys Cys Thr Val Gly Asp Arg Trp
            100                 105                 110

Ser Ser Gln Gln Gly Asn Lys Ala Asp Tyr Pro Glu Gly Asp Gly Asn
        115                 120                 125

Trp Ala Asn Tyr Asn Thr Phe Gly Ser Ala Glu Ala Ala Thr Ser Asp
    130                 135                 140

Asp Tyr Lys Asn Pro Gly Tyr Tyr Asp Ile Gln Ala Lys Asp Leu Gly
145                 150                 155                 160

Ile Trp His Val Pro Asn Lys Ser Pro Met Gln His Trp Arg Asn Ser
```

165 170 175

Ala Leu Leu Arg Tyr Arg Thr Asn Thr Gly Phe Leu Gln Arg Leu Gly
180 185 190

His Asn Leu Phe Gly Ile Tyr Gln Lys Tyr Pro Val Lys Tyr Arg Ser
195 200 205

Gly Lys Cys Trp Asn Asp Asn Gly Pro Ala Ile Pro Val Val Tyr Asp
210 215 220

Phe Gly Asp Ala Lys Lys Thr Ala Ser Tyr Tyr Ser Pro Tyr Gly Gln
225 230 235 240

Arg Glu Phe Val Ala Gly Phe Val Gln Phe Arg Val Phe Asn Asn Glu
245 250 255

Arg Ala Ala Asn Ala Leu Cys Ala Gly Ile Lys Val Thr Gly Cys Asn
260 265 270

Thr Glu His His Cys Ile Gly Gly Gly Gly Phe Phe Pro Gln Gly Lys
275 280 285

Pro Arg Gln Cys Gly Asp Phe Ser Ala Phe Asp Trp Asp Gly Tyr Gly
290 295 300

Thr His Val Lys Ser Ser Cys Ser Arg Glu Ile Thr Glu Ala Ala Val
305 310 315 320

Leu Leu Phe Tyr Arg
325

<210> 37
<211> 2085
<212> DNA
<213> Homo sapiens

<220>
<221> Megakaryocyte potentiation factor precursor
<222> (1)..(2085)
<223> NM005823

<400> 37
tggccactcc cgtctgctgt gacgcgcgga cagagagcta ccggtggacc cacggtgcct 60

ccctccctgg gatctacaca gaccatggcc ttgccaacgg ctcgacccct gttggggtcc 120

tgtgggaccc ccgccctcgg cagcctcctg ttcctgctct tcagcctcgg atgggtgcag 180

ccctcgagga ccctggctgg agagacaggg caggaggctg cacccctgga cggagtcctg 240

gccaacccac ctaacatttc cagcctctcc cctcgccaac tccttggctt cccgtgtgcg 300

gaggtgtccg gcctgagcac ggagcgtgtc cgggagctgg ctgtggcctt ggcacagaag 360

aatgtcaagc tctcaacaga gcagctgcgc tgtctggctc accggctctc tgagcccccc 420

```
gaggacctgg acgccctccc attggacctg ctgctattcc tcaacccaga tgcgttctcg      480
gggccccagg cctgcacccg tttcttctcc cgcatcacga aggccaatgt ggacctgctc      540
ccgagggggg ctcccgagcg acagcggctg ctgcctgcgg ctctggcctg ctggggtgtg      600
cgggggtctc tgctgagcga ggctgatgtg cgggctctgg gaggcctggc ttgcgacctg      660
cctgggcgct ttgtggccga gtcggccgaa gtgctgctac ccggctggt gagctgcccg       720
ggaccccctgg accaggacca gcaggaggca gccagggcgg ctctgcaggg cgggggaccc     780
ccctacggcc ccccgtcgac atggtctgtc tccacgatgg acgctctgcg gggcctgctg      840
cccgtgctgg gccagcccat catccgcagc atcccgcagg gcatcgtggc cgcgtggcgg      900
caacgctcct ctcgggaccc atcctggcgg cagcctgaac ggaccatcct ccggccgcgg      960
ttccggcggg aagtggagaa gacagcctgt ccttcaggca agaaggcccg cgagatagac     1020
gagagcctca tcttctacaa gaagtgggag ctggaagcct gcgtggatgc ggccctgctg     1080
gccacccaga tggaccgcgt gaacgccatc cccttcacct acgagcagct ggacgtccta     1140
aagcataaac tggatgagct ctacccacaa ggttaccccg agtctgtgat ccagcacctg     1200
ggctacctct tcctcaagat gagccctgag gacattcgca agtggaatgt gacgtccctg     1260
gagaccctga aggctttgct tgaagtcaac aaagggcacg aaatgagtcc tcaggtggcc     1320
accctgatcg accgctttgt gaagggaagg ggccagctag acaaagacac cctagacacc     1380
ctgaccgcct tctaccctgg gtacctgtgc tccctcagcc ccgaggagct gagctccgtg     1440
ccccccagca gcatctgggc ggtcaggccc caggacctgg acacgtgtga cccaaggcag     1500
ctggacgtcc tctatcccaa ggcccgcctt gctttccaga acatgaacgg gtccgaatac     1560
ttcgtgaaga tccagtcctt cctgggtggg gcccccacgg aggatttgaa ggcgctcagt     1620
cagcagaatg tgagcatgga cttggccacg ttcatgaagc tgcggacgga tgcggtgctg     1680
ccgttgactg tggctgaggt gcagaaactt ctgggacccc acgtggaggg cctgaaggcg     1740
gaggagcggc accgcccggt gcgggactgg atcctacggc agcggcagga cgacctggac     1800
acgctggggc tggggctaca gggcggcatc cccaacggct acctggtcct agacctcagc     1860
gtgcaagagg ccctctcggg gacgccctgc ctcctaggac ctggacctgt tctcaccgtc     1920
ctggcactgc tcctagcctc caccctggcc tgagggcccc actcccttgc tggccccagc     1980
cctgctgggg atccccgcct ggccaggagc aggcacgggt gatccccgtt ccaccccaag     2040
agaactcgcg ctcagtaaac gggaacatgc cccctgcaga cacgt                    2085
```

<210> 38
<211> 622
<212> PRT
<213> Homo sapiens

<220>
<221> Megakaryocyte potentiation factor precursor
<222> (1)..(622)
<223> NP005814

<400> 38

Met Ala Leu Pro Thr Ala Arg Pro Leu Leu Gly Ser Cys Gly Thr Pro
1 5 10 15

Ala Leu Gly Ser Leu Leu Phe Leu Leu Phe Ser Leu Gly Trp Val Gln
20 25 30

Pro Ser Arg Thr Leu Ala Gly Glu Thr Gly Gln Glu Ala Ala Pro Leu
35 40 45

Asp Gly Val Leu Ala Asn Pro Pro Asn Ile Ser Ser Leu Ser Pro Arg
50 55 60

Gln Leu Leu Gly Phe Pro Cys Ala Glu Val Ser Gly Leu Ser Thr Glu
65 70 75 80

Arg Val Arg Glu Leu Ala Val Ala Leu Ala Gln Lys Asn Val Lys Leu
85 90 95

Ser Thr Glu Gln Leu Arg Cys Leu Ala His Arg Leu Ser Glu Pro Pro
100 105 110

Glu Asp Leu Asp Ala Leu Pro Leu Asp Leu Leu Leu Phe Leu Asn Pro
115 120 125

Asp Ala Phe Ser Gly Pro Gln Ala Cys Thr Arg Phe Phe Ser Arg Ile
130 135 140

Thr Lys Ala Asn Val Asp Leu Leu Pro Arg Gly Ala Pro Glu Arg Gln
145 150 155 160

Arg Leu Leu Pro Ala Ala Leu Ala Cys Trp Gly Val Arg Gly Ser Leu
165 170 175

Leu Ser Glu Ala Asp Val Arg Ala Leu Gly Gly Leu Ala Cys Asp Leu
180 185 190

Pro Gly Arg Phe Val Ala Glu Ser Ala Glu Val Leu Leu Pro Arg Leu
195 200 205

Val Ser Cys Pro Gly Pro Leu Asp Gln Asp Gln Gln Glu Ala Ala Arg
210 215 220

Ala Ala Leu Gln Gly Gly Gly Pro Pro Tyr Gly Pro Pro Ser Thr Trp
225 230 235 240

Ser Val Ser Thr Met Asp Ala Leu Arg Gly Leu Leu Pro Val Leu Gly
245 250 255

Gln Pro Ile Ile Arg Ser Ile Pro Gln Gly Ile Val Ala Ala Trp Arg

260 265 270

Gln Arg Ser Ser Arg Asp Pro Ser Trp Arg Gln Pro Glu Arg Thr Ile
275 280 285

Leu Arg Pro Arg Phe Arg Arg Glu Val Glu Lys Thr Ala Cys Pro Ser
290 295 300

Gly Lys Lys Ala Arg Glu Ile Asp Glu Ser Leu Ile Phe Tyr Lys Lys
305 310 315 320

Trp Glu Leu Glu Ala Cys Val Asp Ala Ala Leu Leu Ala Thr Gln Met
325 330 335

Asp Arg Val Asn Ala Ile Pro Phe Thr Tyr Glu Gln Leu Asp Val Leu
340 345 350

Lys His Lys Leu Asp Glu Leu Tyr Pro Gln Gly Tyr Pro Glu Ser Val
355 360 365

Ile Gln His Leu Gly Tyr Leu Phe Leu Lys Met Ser Pro Glu Asp Ile
370 375 380

Arg Lys Trp Asn Val Thr Ser Leu Glu Thr Leu Lys Ala Leu Leu Glu
385 390 395 400

Val Asn Lys Gly His Glu Met Ser Pro Gln Val Ala Thr Leu Ile Asp
405 410 415

Arg Phe Val Lys Gly Arg Gly Gln Leu Asp Lys Asp Thr Leu Asp Thr
420 425 430

Leu Thr Ala Phe Tyr Pro Gly Tyr Leu Cys Ser Leu Ser Pro Glu Glu
435 440 445

Leu Ser Ser Val Pro Pro Ser Ser Ile Trp Ala Val Arg Pro Gln Asp
450 455 460

Leu Asp Thr Cys Asp Pro Arg Gln Leu Asp Val Leu Tyr Pro Lys Ala
465 470 475 480

Arg Leu Ala Phe Gln Asn Met Asn Gly Ser Glu Tyr Phe Val Lys Ile
485 490 495

Gln Ser Phe Leu Gly Gly Ala Pro Thr Glu Asp Leu Lys Ala Leu Ser
500 505 510

Gln Gln Asn Val Ser Met Asp Leu Ala Thr Phe Met Lys Leu Arg Thr
515 520 525

Asp Ala Val Leu Pro Leu Thr Val Ala Glu Val Gln Lys Leu Leu Gly

```
        530                 535                 540

Pro His Val Glu Gly Leu Lys Ala Glu Glu Arg His Arg Pro Val Arg
545                 550                 555                 560

Asp Trp Ile Leu Arg Gln Arg Gln Asp Asp Leu Asp Thr Leu Gly Leu
                565                 570                 575

Gly Leu Gln Gly Gly Ile Pro Asn Gly Tyr Leu Val Leu Asp Leu Ser
            580                 585                 590

Val Gln Glu Ala Leu Ser Gly Thr Pro Cys Leu Leu Gly Pro Gly Pro
        595                 600                 605

Val Leu Thr Val Leu Ala Leu Leu Leu Ala Ser Thr Leu Ala
    610                 615                 620
```